# EUROPEAN PATENT SPECIFICATION

(11) **EP 3 341 477 B1**
(45) Date of publication and mention of the grant of the patent: **23.03.2022**
(21) Application number: 16842722.7
(22) Date of filing: 26.08.2016
(51) Int. Cl.: C12N 15/63, C12N 15/113, C12N 9/22

(54) **ENGINEERED CRISPR-CAS9 NUCLEASES**
MANIPULIERTE CRISPR-CAS9-NUKLEASEN
NUCLÉASES CRISPR-CAS9 MODIFIÉES

(30) Priority: 28.08.2015 US 201562211553 P; 09.09.2015 US 201562216033 P; 20.11.2015 US 201562258280 P; 28.12.2015 US 201562271938 P; 04.02.2016 US 201615015947
(43) Date of publication of application: 04.07.2018
(60) Divisional application: 22156340.6
(73) Proprietor: The General Hospital Corporation, Boston, MA 02114 (US)
(72) Inventor: JOUNG, J. Keith, Winchester, Massachusetts 01890 (US); KLEINSTIVER, Benjamin, Medford, Massachusetts 02155 (US); PATTANAYAK, Vikram, Wellesley, Massachusetts 02482 (US)
(74) Representative: Forresters IP LLP
(86) International application number: PCT/US2016/049147
(87) International publication number: WO 2017/040348

(56) References cited:
- WO-A1-2015/089364
- WO-A1-2016/115355
- US-A1- 2014 068 797
- KLEINSTIVER ET AL.: 'Engineered CRISPR-Cas9 nucleases with altered PAM specificities.' NATURE vol. 523, no. 7561, 23 July 2015, pages 481 - 485, XP055293257
- KLEINSTIVER ET AL.: 'High-fidelity CRISPR-Cas9 nucleases with no detectable genome-wide off- target effects.' NATURE vol. 529, no. 7587, 28 January 2016, pages 490 - 495, XP055303393

## Description

### CLAIM OF PRIORITY

This application claims priority under 35 USC §119(e) to U.S. Patent Application Serial Nos. 62/211,553, filed on August 28, 2015; 62/216,033, filed on September 9, 2015; 62/258,280, filed on November 20, 2015; 62/271,938, filed on December 28, 2015; and 15/015,947, filed on February 4, 2016.

### SEQUENCE LISTING

The instant application contains a Sequence Listing which has been submitted electronically in ASCII format. Said ASCII copy, created on August 26, 2016, is named SEQUENCE LISTING.txt and is 129,955 bytes in size.

### FEDERALLY SPONSORED RESEARCH OR DEVELOPMENT

This invention was made with Government support under Grant Nos. DPI GM105378 and R01 GM088040 awarded by the National Institutes of Health. The Government has certain rights in the invention.

### TECHNICAL FIELD

The invention relates, at least in part, to engineered Clustered Regularly Interspaced Short Palindromic Repeats (CRISPRs)/CRISPR-associated protein 9 (Cas9) nucleases with altered and improved target specificity and their use in genomic engineering, epigenomic engineering, genome targeting, genome editing, and in vitro diagnostics.

### BACKGROUND

CRISPR-Cas9 nucleases enable efficient genome editing in a wide variety of organisms and cell types (Sander & Joung, Nat Biotechnol 32, 347-355 (2014); Hsu et al., Cell 157, 1262-1278 (2014); Doudna & Charpentier, Science 346, 1258096 (2014); Barrangou & May, Expert Opin Biol Ther 15, 311-314 (2015)). Target site recognition by Cas9 is programmed by a chimeric single guide RNA (**sgRNA**) that encodes a sequence complementary to a target protospacer (Jinek et al., Science 337, 816-821 (2012)), but also requires recognition of a short neighboring PAM (Mojica et al., Microbiology 155, 733-740 (2009); Shah et al., RNA Biol 10, 891-899 (2013); Jiang et al., Nat Biotechnol 31, 233-239 (2013); Jinek et al., Science 337, 816-821 (2012); Sternberg et al., Nature 507, 62-67 (2014)).

### SUMMARY

As described herein, Cas9 Proteins can be engineered to show increased specificity, theoretically by reducing the binding affinity of Cas9 for DNA. Thus, described herein are a number of Cas9 variants that have increased specificity (i.e., induce substantially fewer off target effects at imperfectly matched or mismatched DNA sites) as compared to the wild type protein, as well as methods of using them.

The invention provides isolated *Streptococcus pyogenes* Cas9 (**SpCas9**) proteins with mutations Q695A and Q926A in SEQ ID NO:1 wherein the SpCas9 protein is at least 95% identical to the amino acid sequence of SEQ ID NO: 1. In some embodiments, the isolated protein comprises mutations Q695A and Q926A and optionally one, two, three, four or five of the following positions: L169A, Y450, N497, R661and/or D1135E i.e., comprising a sequence that is at least 95% identical to the amino acid sequence of SEQ ID NO:1 with mutations Q695A and Q926A and optionally one, two, three, four or five, of the following positions: L169, Y450, N497, R661, D1135E, and optionally one or more of a nuclear localization sequence, cell penetrating peptide sequence, and/or affinity tag. A mutation alters the amino acid to an amino acid other than the native amino acid (e.g., 497 is anything but N). In preferred embodiments the mutation, other than positions Q965A and Q926A, changes the amino acid to any amino acid other than the native one, arginine or lysine; in some embodiments, the amino acid is alanine.

In some embodiments, the variant SpCas9 proteins comprise mutations Q695A and Q926A and optionally one or two of the following: N497, R661 e.g., one or two of the following mutations: N497A, R661A.

In some embodiments, the variant SpCas9 proteins comprise mutations Q695A and Q926A, and optionally one, two, three, four or all five of L169, Y450, N497, R661 and D1135E, e.g., including but not limited to Q695A/Q926A, L169A/Q695A/Q926A, Y450A/Q695A/Q926A, R661A/Q695A/Q926A, N497A/Q695A/Q926A, Q695A/Q926A/D1135E, L169A/Y450A/Q695A/Q926A, L169A/R661A/Q695A/Q926A,Y450A/R661A/Q695A/Q926A, N497A/Q695A/Q926A/D1135E, R661A/Q695A/Q926A/D1135E, and Y450A/Q695A/Q926A/D1 135E.

In some embodiments, the variant SpCas9 proteins comprise mutations at Q695A and Q926A and further comprises mutations at N14; S15; S55; R63; R78; H160; K163; R165; L169; R403; N407; Y450; M495; N497; K510; Y515; W659; R661; M694; H698; A728; S730; K775; S777; R778; R780; K782; R783; K789; K797; Q805; N808; K810; R832; Q844; S845; K848; S851; K855; R859; K862; K890; Q920; K961; S964; K968; K974; R976; N980; H982; K1003; K1014; S1040; N1041; N1044; K1047; K1059; R1060; K1107; E1108; S1109; K1113; R1114; S1116; K1118; D1135; S1136; K1153; K1155 ; K1158; K1200; Q1221; H1241; Q1254; Q1256; K1289; K1296; K1297; R1298; K1300; H1311; K1325; K1334; T1337 and/or S1216.

In some embodiments, the variant SpCas9 proteins comprises mutations Q695A and Q926A and further comprises one or more of the following mutations: N14A; S15A; S55A; R63A; R78A; R165A; R403A; N407A; N497A; Y450A; K510A; Y515A; R661A;; S730A; K775A; S777A; R778A; R780A; K782A; R783A; K789A; K797A; Q805A; N808A; K810A; R832A; Q844A; S845A; K848A; S851A; K855A; R859A; K862A; K890A; Q920A; K961A; S964A; K968A; K974A; R976A; N980A; H982A; K1003A; K1014A; S1040A; N1041A; N1044A; K1047A; K1059A; R1060A; K1107A; E1108A; S1109A; K1113A; R1114A; S1116A; K1118A; D1135A; S1136A; K1153A; K1155A; K1158A; K1200A; Q1221A; H1241A; Q1254A; Q1256A; K1289A; K1296A; K1297A; R1298A; K1300A; H1311A; K1325A; K1334A; T1337A and/or S1216A. In some embodiments, the variant proteins include HF1(N497A/R661A/Q695A/Q926A)+K810A.. In some embodiments, the variant proteins include Q695A/Q926A/R780A, Q695A/Q926A/R976A, Q695A/Q926A/H982A, Q695A/Q926A/K855A, Q695A/Q926A/K848A/K1003A, Q695A/Q926A/K848A/K855A, Q695A/Q926A/K848A/H982A, Q695A/Q926A/K1003A/R1060A, Q695A/Q926A/K848A/R1060A, Q695A/Q926A/K855A/H982A, Q695A/Q926A/K855A/K1003A, Q695A/Q926A/K855A/R1060A, Q695A/Q926A/H982A/K1003A, Q695A/Q926A/H982A/R1060A, Q695A/Q926A/K1003A/R1060A, Q695A/Q926A/K810A/K1003A/R1060A, Q695A/Q926A/K848A/K1003A/R1060A. In some embodiments, the variants include N497A/R661A/Q695A/Q926A/K810A, N497A/R661A/Q695A/Q926A/K848A, N497A/R661A/Q695A/Q926A/K855A, N497A/R661A/Q695A/Q926A/R780A, N497A/R661A/Q695A/Q926A/K968A, N497A/R661A/Q695A/Q926A/H982A, N497A/R661A/Q695A/Q926A/K1003A, N497A/R661A/Q695A/Q926A/K1014A, N497A/R661A/Q695A/Q926A/K1047A, N497A/R661A/Q695A/Q926A/R1060A, N497A/R661A/Q695A/Q926A/K810A/K968A, N497A/R661A/Q695A/Q926A/K810A/K848A, N497A/R661A/Q695A/Q926A/K810A/K1003A, N497A/R661A/Q695A/Q926A/K810A/R1060A, N497A/R661A/Q695A/Q926A/K848A/K1003A, N497A/R661A/Q695A/Q926A/K848A/R1060A, N497A/R661A/Q695A/Q926A/K855A/K1003A, N497A/R661A/Q695A/Q926A/K855A/R1060A, N497A/R661A/Q695A/Q926A/K968A/K1003A, N497A/R661A/Q695A/Q926A/H982A/K1003A, N497A/R661A/Q695A/Q926A/H982A/R1060A, N497A/R661A/Q695A/Q926A/K1003A/R1060A, N497A/R661A/Q695A/Q926A/K810A/K1003A/R1060A, N497A/R661A/Q695A/Q926A/K848A/K1003A/R1060A, Q695A/Q926A/R780A, Q695A/Q926A/K810A, Q695A/Q926A/R832A, Q695A/Q926A/K848A, Q695A/Q926A/K855A, Q695A/Q926A/K968A, Q695A/Q926A/R976A, Q695A/Q926A/H982A, Q695A/Q926A/K1003A, Q695A/Q926A/K1014A, Q695A/Q926A/K1047A, Q695A/Q926A/R1060A, Q695A/Q926A/K848A/K968A, Q695A/Q926A/R976A, Q695A/Q926A/H982A, Q695A/Q926A/K855A, Q695A/Q926A/K848A/K1003A, Q695A/Q926A/K848A/K855A, Q695A/Q926A/K848A/H982A, Q695A/Q926A/K1003A/R1060A, Q695A/Q926A/R832A/R1060A, Q695A/Q926A/K968A/K1003A, Q695A/Q926A/K968A/R1060A, Q695A/Q926A/K848A/R1060A, Q695A/Q926A/K855A/H982A, Q695A/Q926A/K855A/K1003A, Q695A/Q926A/K855A/R1060A, Q695A/Q926A/H982A/K1003A, Q695A/Q926A/H982A/R1060A, Q695A/Q926A/K1003A/R1060A, Q695A/Q926A/K810A/K1003A/R1060A, Q695A/Q926A/K1003A/K1047A/R1060A, Q695A/Q926A/K968A/K1003A/R1060A, Q695A/Q926A/R832A/K1003A/R1060A, or Q695A/Q926A/K848A/K1003A/R1060A

Mutations to amino acids other than alanine at positions other than Q695 and Q926 are also included, and can be made and used in the present methods and compositions.

In some embodiments, the variant SpCas9 proteins comprise multiple substitution mutations: N497/R661/Q695/Q926 (quadruple variant mutants); Q695/Q926 (double mutant); R661/Q695/Q926 and N497/Q695/Q926 (triple mutants), wherein the mutation at Q695 is Q695A and the mutation at Q926 is Q926A. In some embodiments, additional substitution mutations at L169, Y450 and/or D1135 might be added to these double-, triple, and quadruple mutants or added to single mutants bearing substitutions at Q695A and Q926A. In some embodiments, the mutants have alanine in place of the wild type amino acid. In some embodiments, the mutants, other than position Q695A and Q926A, have any amino acid other than arginine or lysine (or the native amino acid).

In some embodiments, the variant SpCas9 proteins also comprise one or more mutations that decrease nuclease activity selected from the group consisting of mutations at D10, E762, D839, H983, or D986; and at H840 or N863. In some embodiments, the mutations are: (i) D10A or D10N, and (ii) H840A, H840N, or H840Y.

In some embodiments, the SpCas9 variants can also include one of the following sets of mutations: D1135V/R1335Q/T1337R (VQR variant); D1135E/R1335Q/T1337R (EQR variant); D1135V/G1218R/R1335Q/T1337R (VRQR variant); or D1135V/G1218R/R1335E/T1337R (VRER variant).

Also provided herein, which does not form part of the claimed invention, are isolated *Staphylococcus aureus* Cas9 (**SaCas9**) protein, with mutations at one, two, three, four, five, six, or more of the following positions: Y211, Y212, W229, Y230, R245, T392, N419, Y651, or R654, e.g., comprising a sequence that is at least 80% identical to the amino acid sequence of SEQ ID NO: 1 with mutations at one, two, three, four, or five, or six of the following positions: Y211, Y212, W229, Y230, R245, T392, N419, Y651, or R654, and optionally one or more of a nuclear localization sequence, cell penetrating peptide sequence, and/or affinity tag. In some embodiments which do not form part of the claimed invention, the SaCas9 variants described herein include the amino acid sequence of SEQ ID NO:2, with mutations at one, two, three, four, five, six, or more of the following positions: Y211, Y212, W229, Y230, R245, T392, N419, Y651and/or R654. In some embodiments which do not form part of the claimed invention, the variants include one or more of the following mutations: Y211A, Y212A, W229, Y230A, R245A, T392A, N419A, Y651, and/or R654A.

In some embodiments which do not form part of the claimed invention, the variant SaCas9 proteins comprise mutations at N419 and/or R654, and optionally one, two, three, four or more of the additional mutations Y211, Y212, W229, Y230, R245 and T392, preferably N419A/R654A, Y211A/R654A, Y211A/Y212A, Y211A/Y230A, Y211A/R245A, Y212A/Y230A, Y212A/R245A, Y230A/R245A, W229A/R654A, Y211A/Y212A/Y230A, Y211A/Y212A/R245A, Y211A/Y212A/Y651A, Y211A/Y230A/R245A, Y211A/Y230A/Y651A, Y211A/R245A/Y651A, Y211A/R245A/R654A, Y211A/R245A/N419A, Y211A/N419A/R654A, Y212A/Y230A/R245A, Y212A/Y230A/Y651A, Y212A/R245A/Y651A, Y230A/R245A/Y651A, R245A/N419A/R654A, T392A/N419A/R654A, R245A/T392A/N419A/R654A, Y211A/R245A/N419A/R654A, W229A/R245A/N419A/R654A, Y211A/R245A/T392A/N419A/R654A, or Y211A/W229A/R245A/N419A/R654A.

In some embodiments which do not form part of the claimed invention, the variant SaCas9 proteins comprise mutations at Y211; Y212; W229; Y230; R245; T392; N419; L446; Q488; N492; Q495; R497; N498; R499; Q500; K518; K523; K525; H557; R561; K572; R634; Y651; R654; G655; N658; S662; N667; R686; K692; R694; H700; K751; D786; T787; Y789; T882; K886; N888; 889; L909; N985; N986; R991; R1015; N44; R45; R51; R55; R59; R60; R116; R165; N169; R208; R209; Y211; T238; Y239; K248; Y256; R314; N394; Q414; K57; R61; H111; K114; V164; R165; L788; S790; R792; N804; Y868; K870; K878; K879; K881; Y897; R901; and/or K906.

In some embodiments which do not form part of the claimed invention, the variant SaCas9 proteins comprise one or more of the following mutations: Y211A; Y212A; W229A; Y230A; R245A; T392A; N419A; L446A; Q488A; N492A; Q495A; R497A; N498A; R499A; Q500A; K518A; K523A; K525A; H557A; R561A; K572A; R634A; Y651A; R654A; G655A; N658A; S662A; N667A; R686A; K692A; R694A; H700A; K751A; D786A; T787A; Y789A; T882A; K886A; N888A; A889A; L909A; N985A; N986A; R991A; R1015A; N44A; R45A; R51A; R55A; R59A; R60A; R116A; R165A; N169A; R208A; R209A; T238A; Y239A; K248A; Y256A; R314A; N394A; Q414A; K57A; R61A; H111A; K114A; V164A; R165A; L788A; S790A; R792A; N804A; Y868A; K870A; K878A; K879A; K881A; Y897A; R901A; K906A.

In some embodiments which do not form part of the claimed invention, variant SaCas9 proteins comprise one or more of the following additional mutations: Y211A, W229A, Y230A, R245A, T392A, N419A, L446A, Y651A, R654A, D786A, T787A, Y789A, T882A, K886A, N888A, A889A, L909A, N985A, N986A, R991A, R1015A, N44A, R45A, R51A, R55A, R59A, R60A, R116A, R165A, N169A, R208A, R209A, T238A, Y239A, K248A, Y256A, R314A, N394A, Q414A, K57A, R61A, H111A, K114A, V164A, R165A, L788A, S790A, R792A, N804A, Y868A, K870A, K878A, K879A, K881A, Y897A, R901A, K906A.

In some embodiments which do not form part of the claimed invention, the variant SaCas9 proteins comprise multiple substitution mutations: R245/T392/N419/R654 and Y221/R245/N419/R654 (quadruple variant mutants); N419/R654, R245/R654, Y221/R654, and Y221/N419 (double mutants); R245/N419/R654, Y211/N419/R654, and T392/N419/R654 (triple mutants). In some embodiments which do not form part of the claimed invention the mutants contain alanine in place of the wild type amino acid.

In some embodiments which do not form part of the claimed invention, the variant SaCas9 proteins also comprise one or more mutations that decrease nuclease activity selected from the group consisting of mutations at D10, E477, D556, H701, or D704; and at H557 or N580. In some embodiments, the mutations are: (i) D10A or D10N, (ii) H557A, H557N, or H557Y, (iii) N580A, and/or (iv) D556A.

In some embodiments which do not form part of the claimed invention, the variant SaCas9 proteins comprise one or more of the following mutations: E782K, K929R, N968K, or R1015H. Specifically, E782K/N968K/R1015H (KKH variant); E782K/K929R/R1015H (KRH variant); or E782K/K929R/N968K/R1015H (KRKH variant).

In some embodiments of the disclosure, the variant Cas9 proteins include mutations to one or more of the following regions to increase specificity:

| Functional Region | SpCas9 | SaCas9 |
|---|---|---|
| Residues contacting the DNA of the spacer region | L169; Y450; M495; N497; W659; R661; M694; Q695; H698; A728; Q926; E1108; V1015 | Y211; W229; Y230; R245; T392; N419; L446; Y651; R654 |
| Residues that potentially contact the DNA of the non-target strand | N14; S15; S55; S730; K775; S777; R778; R780; K782; R783; K789; K797; Q805; N808; K810; R832; Q844; S845; K848; S851; K855; R859; K862; K890; Q920; K961; S964; K968; K974; R976; N980; H982; K1003; K1014; S1040; N1041; N1044; K1047; K1059; R1060; K1200; H1241; Q1254; Q1256; K1289; K1296; K1297; K1300; H1311; K1325 | Q488A; N492A; Q495A; R497A; N498A; R499; Q500; K518; K523; K525; H557; R561; K572; R634; R654; G655; N658; S662; N667; R686; K692; R694; H700; K751 |
| Residues contacting the DNA of the PAM region (including direct PAM contacts) | R71; Y72; R78; R165; R403; T404; F405; K1107; S1109; R1114; S1116; K1118; D1135; S1136; K1200; S1216; E1219; R1333; R1335; T1337 | D786; T787; Y789; T882; K886; N888; A889; L909; N985; N986; R991; R1015 |
| Residues contacting the RNA of the spacer region | Y72; R75; K76; L101; S104; F105; R115; H116; I135; H160; K163; Y325; H328; R340; F351; D364; Q402; R403; I1110; K1113; R1122; Y1131 | N44; R45; R51; R55; R59; R60; R116; R165; N169; R208; R209; Y211; T238; Y239; K248; Y256; R314; N394; Q414 |
| Residues contacting the RNA of the repeat/anti-repeat region | R63; R66; R70; R71; R74; R78; R403; T404; N407; R447; I448; Y450; K510; Y515; R661; V1009; Y1013 | K57; R61; H111; K114; V164; R165; L788; S790; R792; N804; Y868; K870; K878; K879; K881; Y897; R901; K906 |
| Residues contacting the RNA stem loops | K30; K33; N46; R40; K44; E57; T62; R69; N77; L455; S460; R467; T472; 1473; H721; K742; K1097; V1100; T1102; F1105; K1123; K1124; E1225; Q1272; H1349; S1351; Y1356 | R47; K50; R54; R58; H62; R209; E213; S219; R452; K459; R774; N780; R781; L783 |

Also provided herein are fusion proteins comprising the isolated variant Cas9 proteins described herein fused to a heterologous functional domain, with an optional intervening linker, wherein the linker does not interfere with activity of the fusion protein. In some embodiments, the heterologous functional domain acts on DNA or protein, e.g., on chromatin. In some embodiments, the heterologous functional domain is a transcriptional activation domain. In some embodiments, the transcriptional activation domain is from VP64 or NF-κB p65. In some embodiments, the heterologous functional domain is a transcriptional silencer or transcriptional repression domain. In some embodiments, the transcriptional repression domain is a Kruppel-associated box (KRAB) domain, ERF repressor domain (ERD), or mSin3A interaction domain (SID). In some embodiments, the transcriptional silencer is Heterochromatin Protein 1 (HP1), e.g., HP1α or HP1β. In some embodiments, the heterologous functional domain is an enzyme that modifies the methylation state of DNA. In some embodiments, the enzyme that modifies the methylation state of DNA is a DNA methyltransferase (DNMT) or the entirety or the dioxygenase domain of a TET protein, e.g., a catalytic module comprising the cysteine-rich extension and the 20GFeDO domain encoded by 7 highly conserved exons, e.g., the Tet1 catalytic domain comprising amino acids 1580-2052, Tet2 comprising amino acids 1290-1905 and Tet3 comprising amino acids 966-1678. In some embodiments, the TET protein or TET-derived dioxygenase domain is from TET1. In some embodiments, the heterologous functional domain is an enzyme that modifies a histone subunit. In some embodiments, the enzyme that modifies a histone subunit is a histone acetyltransferase (HAT), histone deacetylase (HDAC), histone methyltransferase (HMT), or histone demethylase. In some embodiments, the heterologous functional domain is a biological tether. In some embodiments, the biological tether is MS2, Csy4 or lambda N protein. In some embodiments, the heterologous functional domain is FokI.

Also provided herein are nucleic acids, isolated nucleic acids encoding the variant Cas9 proteins described herein, as well as vectors comprising the isolated nucleic acids, optionally operably linked to one or more regulatory domains for expressing the variant Cas9 proteins described herein. Also provided herein are host cells, e.g., bacterial, yeast, insect, or mammalian host cells or transgenic animals (e.g., mice), comprising the nucleic acids described herein, and optionally expressing the variant Cas9 proteins described herein.

Also provided herein are isolated nucleic acids encoding the Cas9variants, as well as vectors comprising the isolated nucleic acids, optionally operably linked to one or more regulatory domains for expressing the variants, and host cells, e.g., mammalian host cells, comprising the nucleic acids, and optionally expressing the variant proteins.

Also provided herein are *in vitro* or *ex vivo* methods of altering the genome or epigenome of a cell, by expressing in the cell or contacting the cell with variant Cas9 proteins or fusion proteins as described herein, and at least one guide RNA having a region complementary to a selected portion of the genome of the cell with optimal nucleotide spacing at the genomic target site. The methods can include contacting the cell with a nucleic acid encoding the Cas9 protein and the guide RNA, e.g., in a single vector; contacting the cell with a nucleic acid encoding the Cas9 protein and a nucleic acid encoding the guide RNA, e.g., in multiple vectors; and contacting the cell with a complex of purified Cas9 protein and synthetic or purified gRNA, *inter alia.* In some embodiments, the cell stably expresses one or both of the gRNA or the variant protein/fusion protein, and the other element is transfected or introduced into the cell. For example, the cell may stably express a variant protein or fusion protein as described herein, and the methods can include contacting the cell with a synthetic gRNA, a purified recombinantly produced gRNA, or a nucleic acid encoding the gRNA. In some embodiments, the variant protein or fusion protein comprises one or more of a nuclear localization sequence, cell penetrating peptide sequence, and/or affinity tag.

Also provided herein are *in vitro* or *ex vivo* methods for altering, e.g., selectively altering, an isolated dsDNA molecule *in vitro* by contacting the dsDNA with a purified variant protein or fusion protein as described herein, and a guide RNA having a region complementary to a selected portion of the dsDNA molecule.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting. In case of conflict, the present specification, including definitions, will control.

Other features and advantages of the invention will be apparent from the following detailed description and figures, and from the claims.

### DESCRIPTION OF DRAWINGS

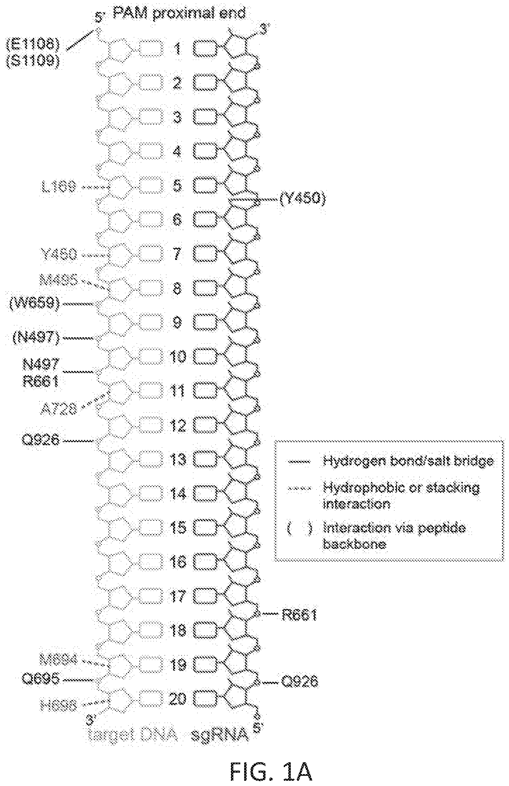

**FIGs. 1A-E** **| Identification and characterization of SpCas9 variants bearing mutations in residues that form non-specific DNA contacts.** A, Schematic depicting wild-type SpCas9 recognition of the target DNA:sgRNA duplex, based on PDB 4OOG and 4UN3 (adapted from refs. 31 and 32, respectively). B, Characterization of SpCas9 variants that contain alanine substitutions in positions that form hydrogen bonds to the DNA backbone. Wild-type SpCas9 and variants were assessed using the human cell EGFP disruption assay when programmed with a perfectly matched sgRNA or four other sgRNAs that encode mismatches to the target site. Error bars represent s.e.m. for n = 3; mean level of background EGFP loss represented by red dashed line (for this panel and panel C). C and D, On-target activities of wild-type SpCas9 and SpCas9-HF1 across 24 sites assessed by EGFP disruption assay (panel C) and 13 endogenous sites by T7E1 assay (panel D). Error bars represent s.e.m. for n = 3. E, Ratio of on-target activity of SpCas9-HF1 to wild-type SpCas9 (from panels C and D).

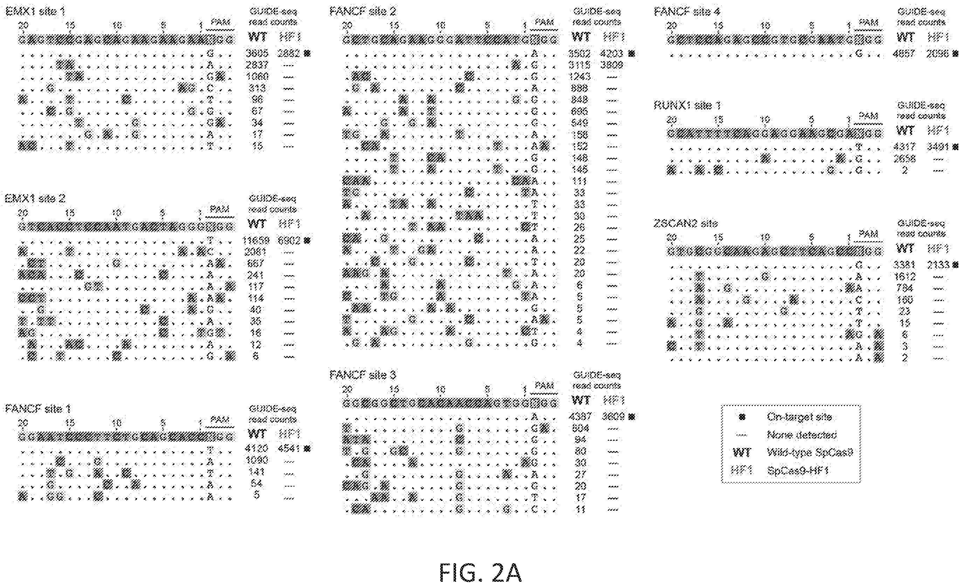

**FIG. 2A-C** **| Genome-wide specificities of wild-type SpCas9 and SpCas9-HF1 with sgRNAs for standard target sites.** A, Off-target sites of wild-type SpCas9 and SpCas9-HF 1 with eight sgRNAs targeted to endogenous human genes, as determined by GUIDE-seq. Read counts represent a measure of cleavage frequency at a given site; mismatched positions within the spacer or PAM are highlighted in color. B, Summary of the total number of genome-wide off-target sites identified by GUIDE-seq for wild-type SpCas9 and SpCas9-HF1 from the eight sgRNAs used in panel A. C, Off-target sites identified for wild-type SpCas9 and SpCas9-HF 1 for the eight sgRNAs, binned according to the total number of mismatches (within the protospacer and PAM) relative to the on-target site.

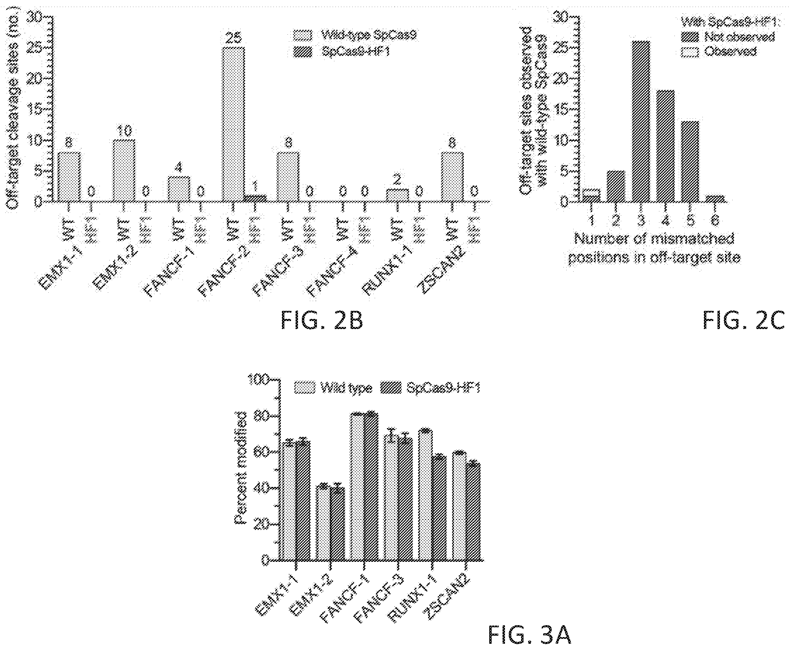

**FIG. 3A-C** **| Validation of SpCas9-HF1 specificity improvements by targeted deep sequencing of off-target sites identified by GUIDE-seq**. A, Mean on-target percent modification determined by deep sequencing for wild-type SpCas9 and SpCas9-HF1 with six sgRNAs from Fig. 2. Error bars represent s.e.m. for n = 3. B, Percentage of deep sequenced on-target sites and GUIDE-seq detected off-target sites that contain indel mutations. Triplicate experiments are plotted for wild-type SpCas9, SpCas9-HF1, and control conditions. Filled circles below the x-axis represent replicates for which no insertion or deletion mutations were observed. Off-target sites that could not be amplified by PCR are shown in red text with an asterisk. Hypothesis testing using a one-sided Fisher exact test with pooled read counts found significant differences (p < 0.05 after adjusting for multiple comparisons using the Benjamini-Hochberg method) for comparisons between SpCas9-HF1 and the control condition only at EMX1-1 off-target 1 and FANCF-3 off-target 1. Significant differences were also found between wild-type SpCas9 and SpCas9-HF1 at all off-target sites, and between wild-type SpCas9 and the control condition at all off-target sites except RUNX1-1 off-target 2. C, Scatter plot of the correlation between GUIDE-seq read counts (from Fig. 2A) and mean percent modification determined by deep sequencing at on- and off-target cleavage sites with wild-type SpCas9.

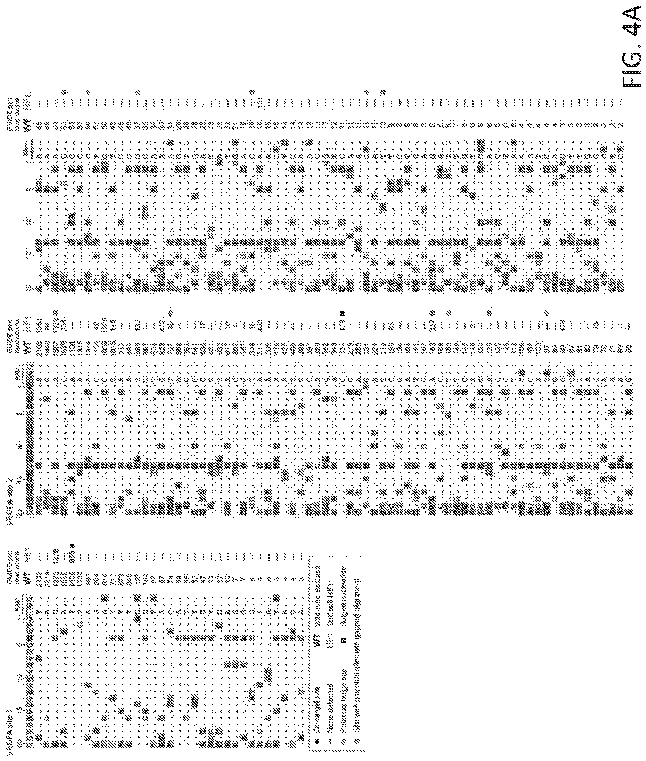

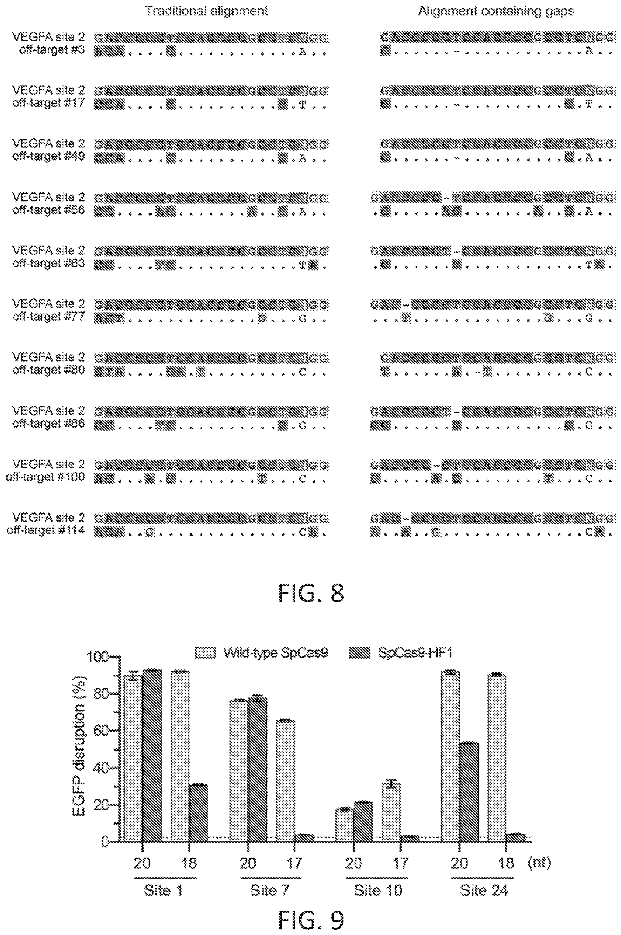

**FIG. 4A-C** **| Genome-wide specificities of wild-type SpCas9 and SpCas9-HF1 with sgRNAs for non-standard, repetitive sites**. A, GUIDE-seq specificity profiles of wild-type SpCas9 and SpCas9-HF1 using two sgRNAs known to cleave large numbers of off-target sites (Fu et al., Nat Biotechnol 31, 822-826 (2013); Tsai et al., Nat Biotechnol 33, 187-197 (2015)). GUIDE-seq read counts represent a measure of cleavage efficiency at a given site; mismatched positions within the spacer or PAM are highlighted in color; red circles indicate sites likely to have the indicated bulge (Lin et al., Nucleic Acids Res 42, 7473-7485 (2014)) at the sgRNA-DNA interface; blue circles indicate sites that may have an alternative gapped alignment relative to the one shown (see Fig. 8). B, Summary of the total number of genome-wide off-target sites identified by GUIDE-seq for wild-type SpCas9 and SpCas9-HF1 from the two sgRNAs used in panel A. C, Off-target sites identified with wild-type SpCas9 or SpCas9-HF1 for VEGFA sites 2 and 3, binned according to the total number of mismatches (within the protospacer and PAM) relative to the on-target site. Off-target sites marked with red circles in panel A are not included in these counts; sites marked with blue circles in panel A are counted with the number of mismatches in the nongapped alignment.

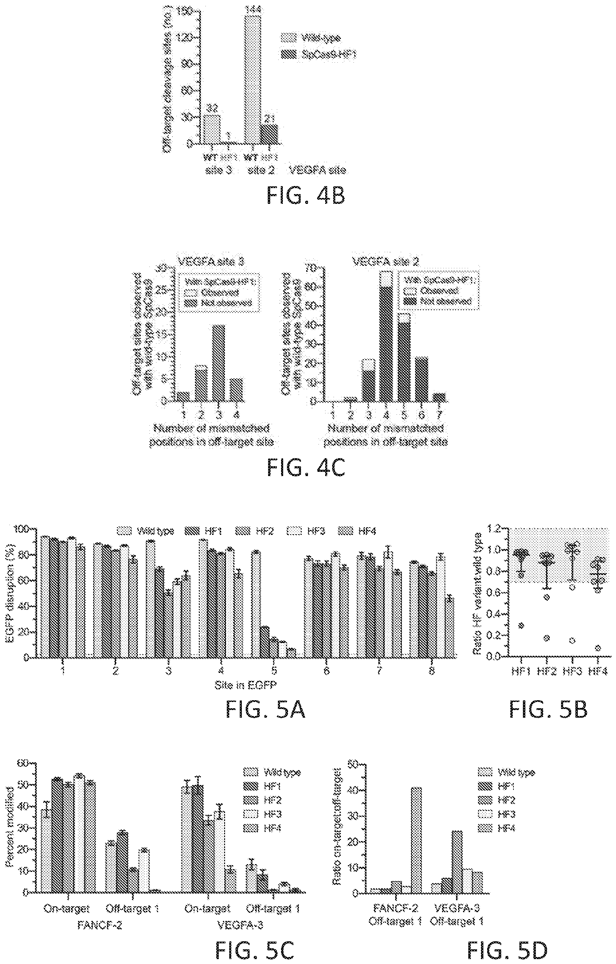

**FIG. 5A****-D| Activities of SpCas9-HF1 derivatives bearing additional substitutions.** A, Human cell EGFP disruption activities of wild-type SpCas9, SpCas9-HF1, and SpCas9-HF1-derivative variants with eight sgRNAs. SpCas9-HF1 harbors N497A, R661A, Q695, and Q926A mutations; HF2 = HF1 + D1135E; HF3 = HF1 + L169A; HF4 = HF1 + Y450A. Error bars represent s.e.m. for n = 3; mean level of background EGFP loss represented by the red dashed line. B, Summary of the on-target activity when using SpCas9-HF variants compared to wild-type SpCas9 with the eight sgRNAs from panel a. The median and interquartile range are shown; the interval showing >70% of wild-type activity is highlighted in green. C, Mean percent modification by SpCas9 and HF variants at the FANCF site 2 and VEGFA site 3 on-target sites, as well as off-target sites from Figs. 2A and 4A resistant to the effects of SpCas9-HF1. Percent modification determined by T7E1 assay; background indel percentages were subtracted for all experiments. Error bars represent s.e.m. for n = 3. D, Specificity ratios of wild-type SpCas9 and HF variants with the FANCF site 2 or VEGFA site 3 sgRNAs, plotted as the ratio of on-target to off-target activity (from panel C).

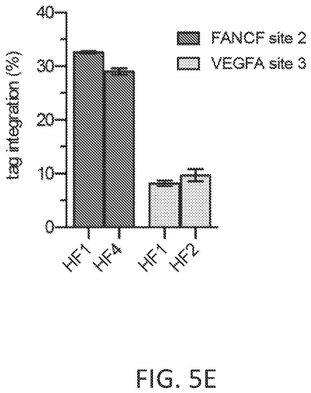

**FIGs. 5E-F** | Genome-wide specificities of SpCas9-HF1, -HF2, and -HF4 with sgRNAs that have off-target sites resistant to the effects of SpCas9-HF1. E, Mean GUIDE-seq tag integration at the intended on-target site for GUIDE-seq experiments in panel F. SpCas9-HF1 = N497A/R661A/Q695A/Q926A; HF2 = HF1 + D1135E; HF4 = HF1 + Y450A. Error bars represent s.e.m. for n = 3. F, GUIDE-seq identified off-target sites of SpCas9-HF1, -HF2, or -HF4 with either the FANCF site 2 or VEGFA site 3 sgRNAs. Read counts represent a measure of cleavage frequency at a given site; mismatched positions within the spacer or PAM are highlighted in color. The fold-improvement in off-target discrimination was calculated by normalizing the off-target read counts for an SpCas9-HF variant to the read counts at the on-target site prior to comparison between SpCas9-HF variants.

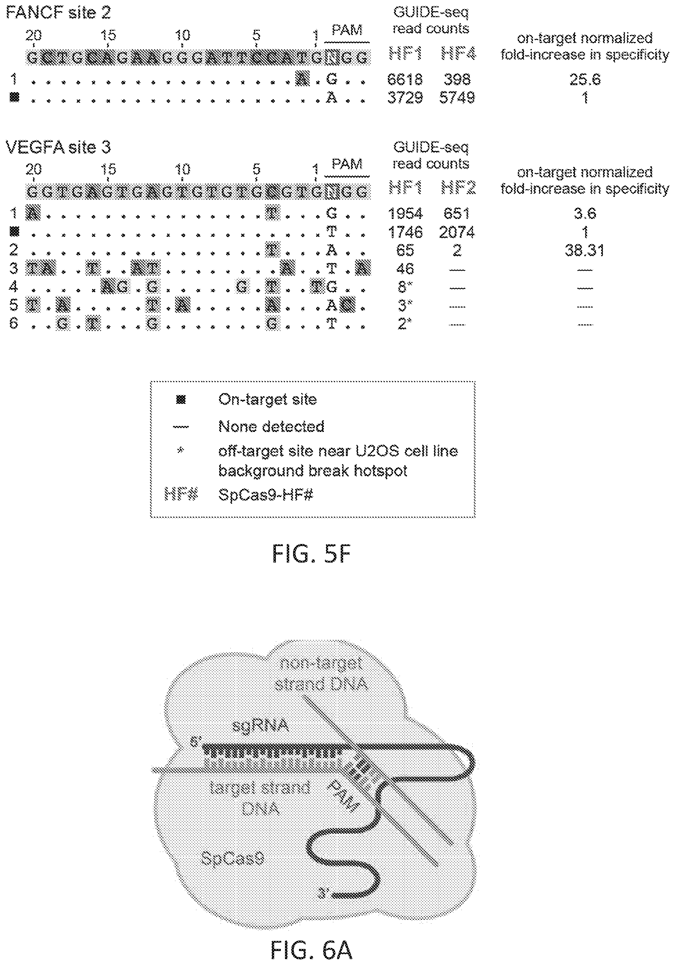

**FIG. 6A-B** **| SpCas9 interaction with the sgRNA and target DNA. A,** Schematic illustrating the SpCas9:sgRNA complex, with base pairing between the sgRNA and target DNA. B, Structural representation of the SpCas9: sgRNA complex bound to the target DNA, from PDB: 4UN3 (ref. 32). The four residues that form hydrogen bond contacts to the target-strand DNA backbone are highlighted in blue; the HNH domain is hidden for visualization purposes.

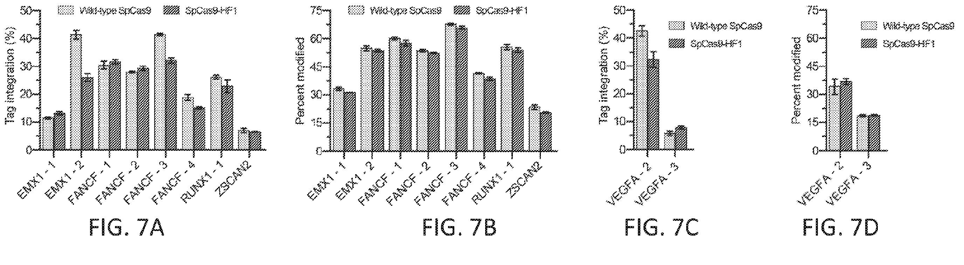

**FIG. 7A-D** **| On-target activity comparisons of wild-type and SpCas9-HF1 with various sgRNAs used for GUIDE-seq experiments.** A and C, Mean GUIDE-seq tag integration at the intended on-target site for GUIDE-seq experiments shown in Figs. 2A and 4A (panels 7A and 7C, respectively), quantified by restriction fragment length polymorphism assay. Error bars represent s.e.m. for n = 3. b and d, Mean percent modification at the intended on-target site for GUIDE-seq experiments shown in Figs. 2A and 4A (panels 7B and 7D, respectively), detected by T7E1 assay. Error bars represent s.e.m. for n = 3.
**FIG. 8** **| Potential alternate alignments for VEGFA site 2 off-target sites.** Ten VEGFA site 2 off-target sites identified by GUIDE-seq (left) that may potentially be recognized as off-target sites that contain single nucleotide gaps (Lin et al., Nucleic Acids Res 42, 7473-7485 (2014))) (right), aligned using Geneious (Kearse et al., Bioinformatics 28, 1647-1649 (2012)) version 8.1.6.
**FIG. 9** **| Activities of wild-type SpCas9 and SpCas9-HF1 with truncated sgRNAs14.** EGFP disruption activities of wild-type SpCas9 and SpCas9-HF1 using full-length or truncated sgRNAs targeted to four sites in EGFP. Error bars represent s.e.m. for n = 3; mean level of background EGFP loss in control experiments is represented by the red dashed line

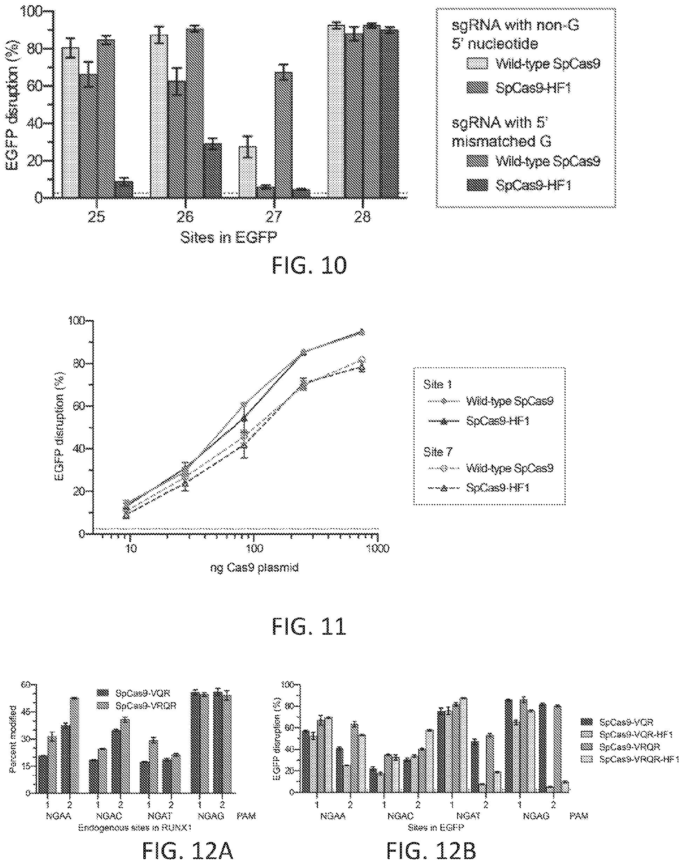

**FIG. 10** **| Wild-type SpCas9 and SpCas9-HF1 activities with sgRNAs bearing 5'-mismatched guanine bases.** EGFP disruption activities of wild-type SpCas9 and SpCas9-HF1 with sgRNAs targeted to four different sites. For each target site, sgRNAs either contain the matched non-guanine 5'-base or a 5'-guanine that is intentionally mismatched.
**FIG. 11** **| Titrating the amount of wild-type SpCas9 and SpCas9-HF1 expression plasmids.** Human cell EGFP disruption activities from transfections with varying amounts of wild-type and SpCas9-HF1 expression plasmids. For all transfections, the amount of sgRNA-containing plasmid was fixed at 250 ng. Two sgRNAs targeting separate sites were used; Error bars represent s.e.m. for n = 3; mean level of background EGFP loss in negative controls is represented by the red dashed line.

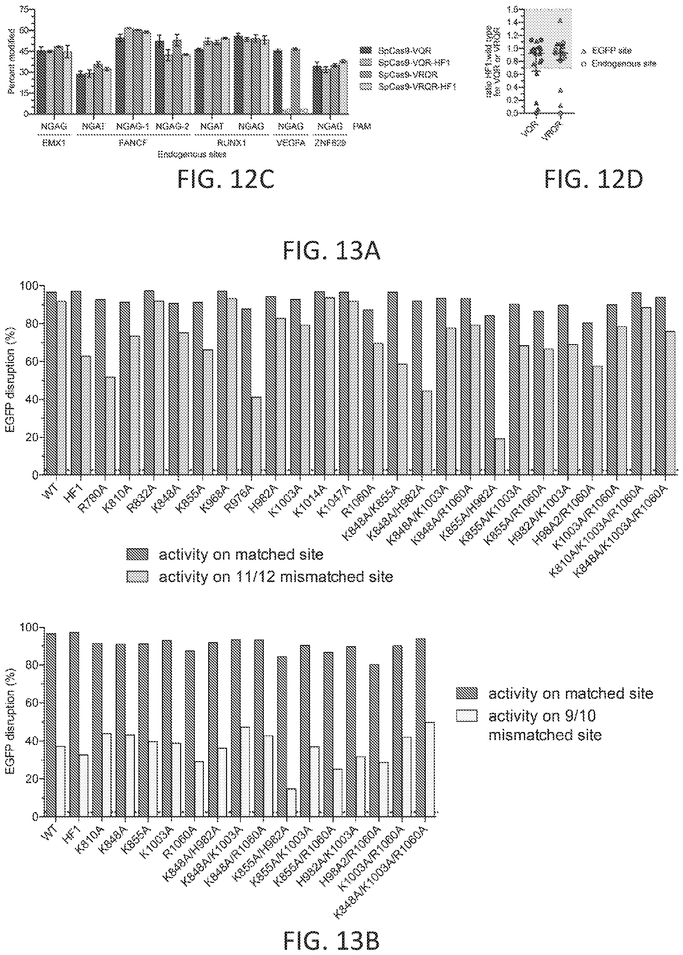

**FIG. 12A-D** **| Altering the PAM recognition specificity of SpCas9-HF1. A,** Comparison of the mean percent modification of on-target endogenous human sites by SpCas9-VQR (ref. 15) and an improved SpCas9-VRQR using 8 sgRNAs, quantified by T7E1 assay. Both variants are engineered to recognize an NGAN PAM Error bars represent s.e.m. for n = 2 or 3. B, On-target EGFP disruption activities of SpCas9-VQR and SpCas9-VRQR compared to their -HF1 counterparts using eight sgRNAs. Error bars represent s.e.m. for n = 3; mean level of background EGFP loss in negative controls represented by the red dashed line. C, Comparison of the mean on-target percent modification by SpCas9-VQR and SpCas9-VRQR compared to their -HF1 variants at eight endogenous human gene sites, quantified by T7E1 assay. Error bars represent s.e.m. for n = 3; ND, not detectable. D, Summary of the fold-change in on-target activity when using SpCas9-VQR or SpCas9-VRQR compared to their corresponding -HF1 variants (from panels B and C). The median and interquartile range are shown; the interval showing >70% of wild-type activity is highlighted in green.
**FIGs. 13A-B** **| Activities of wild-type SpCas9, SpCas9-HF1, and wild-type SpCas9 derivatives bearing one or more alanine substitutions at positions that can potentially contact the non-target DNA strand.** A and B, Nucleases were assessed using the EGFP disruption assay, with an sgRNA that is perfectly matched to a site in the *EGFP* gene as well as an sgRNA that is intentionally mismatched at positions 11 and 12 (panel A) or positions 9 and 10 (panel B). Mismatched positions are numbered with position 20 being the most PAM-distal position; the red dashed line represents background levels of EGFP disruption; HF1 = SpCas9 with N497A/R661A/Q695A/Q926A substitutions.

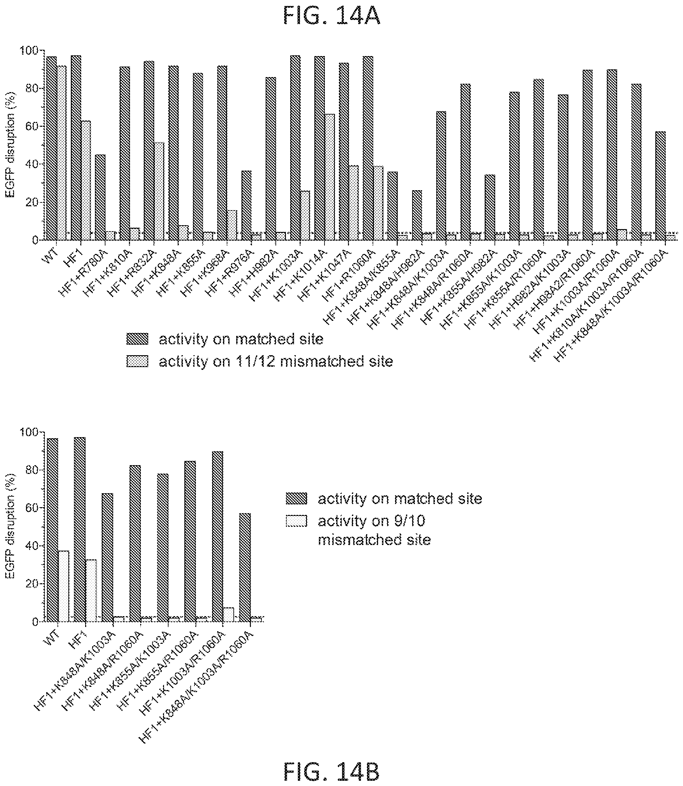

**FIGs. 14A-B** **| Activity of wild-type SpCas9, SpCas9-HF1, and SpCas9-HF1 derivatives bearing one or more alanine substitutions at positions that can potentially contact the non-target DNA strand.** A and B, Nucleases were assessed using the EGFP disruption assay, with an sgRNA that is perfectly matched to a site in the *EGFP* gene as well as an sgRNA that is intentionally mismatched at positions 11 and 12 (panel A) or positions 9 and 10 (panel B). Mismatched positions are numbered with position 20 being the most PAM-distal position; the red dashed line represents background levels of EGFP disruption; HF1 = SpCas9 with N497A/R661A/Q695A/Q926A substitutions.

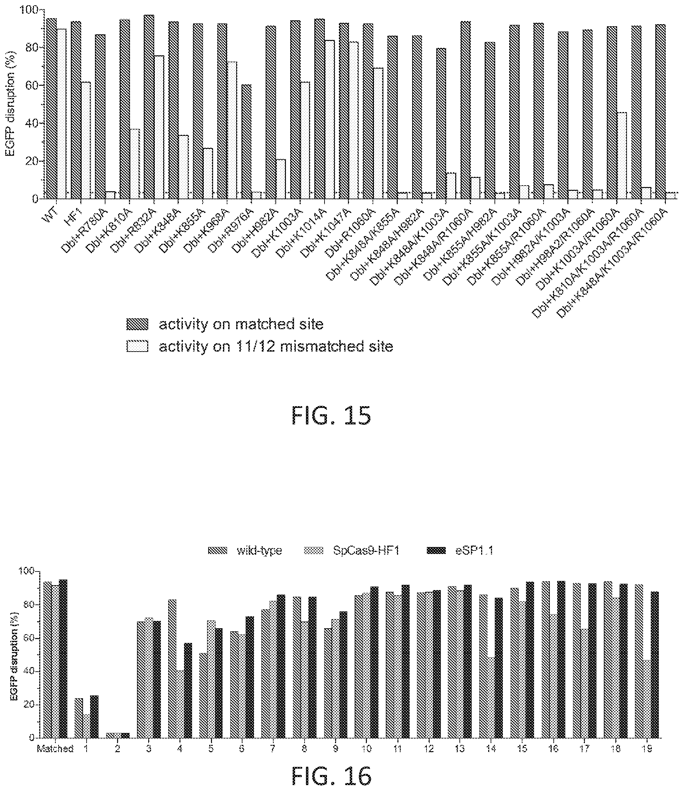

**FIG. 15** **| Activity of wild-type SpCas9, SpCas9-HF1, and SpCas9(Q695A/Q926A) derivatives bearing one or more alanine substitutions at positions that can potentially contact the non-target DNA strand.** Nucleases were assessed using the EGFP disruption assay, with an sgRNA that is perfectly matched to a site in the *EGFP* gene as well as an sgRNA that is intentionally mismatched at positions 11 and 12. Mismatched positions are numbered with position 20 being the most PAM-distal position; the red dashed line represents background levels of EGFP disruption; HF1 = SpCas9 with N497A/R661A/Q695A/Q926A substitutions; Dbl = SpCas9 with Q695A/Q926A substitutions.
**FIG. 16** **| Activities of wild-type SpCas9, SpCas9-HF1, and eSpCas9-1.1 using a matched sgRNA and sgRNAs with single mismatches at each position in the spacer.** Nucleases were assessed using the EGFP disruption assay, with an sgRNA that is perfectly matched to a site in the *EGFP* gene ("matched") as well as sgRNAs that are intentionally mismatched at the positions indicated. Mismatched positions are numbered with position 20 being the most PAM-distal position. SpCas9-HF1 = N497A/R661A/Q695A/Q926A, and eSP1.1 = K848A/K1003A/R1060A.

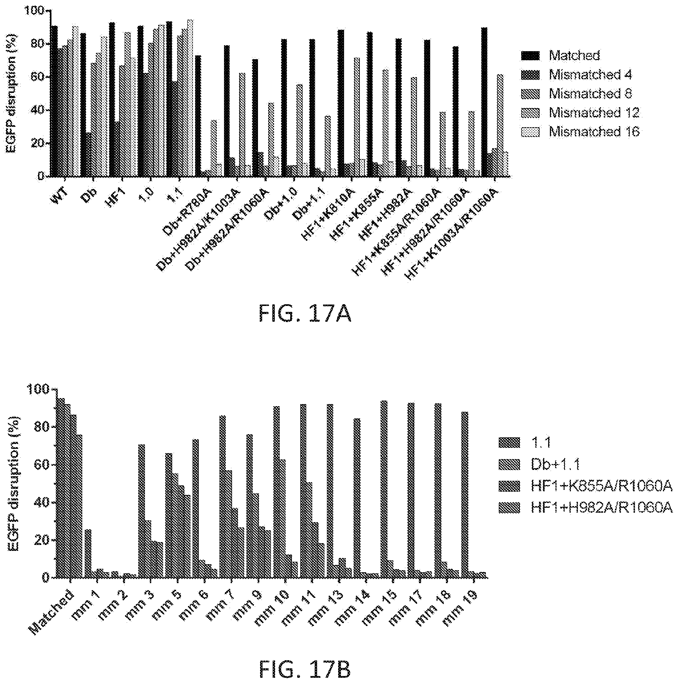

**FIGs. 17A-B** **| Activities of wild-type SpCas9 and variants using a matched sgRNA and sgRNAs with single mismatches at various positions in the spacer.** (**A**) The activities of SpCas9 nucleases containing combinations of alanine substitutions (directed to positions that may potentially contact the target or non-target DNA strands) were assessed using the EGFP disruption assay, with an sgRNA that is perfectly matched to a site in the *EGFP* gene ("matched") as well as sgRNAs that are intentionally mismatched at the indicated spacer positions. (**B**) A subset of these nucleases from (a) were tested using the remainder of all possible singly mismatched sgRNAs for the matched on-target site. Mismatched positions are numbered with position 20 being the most PAM-distal position. mm = mismatch, WT = wild-type, Db = Q695A/Q926A, HF1 = N497A/R661A/Q695A/Q926A, 1.0 = K810A/K1003A/R1060A, and 1.1 = K848A/K1003A/R1060A.

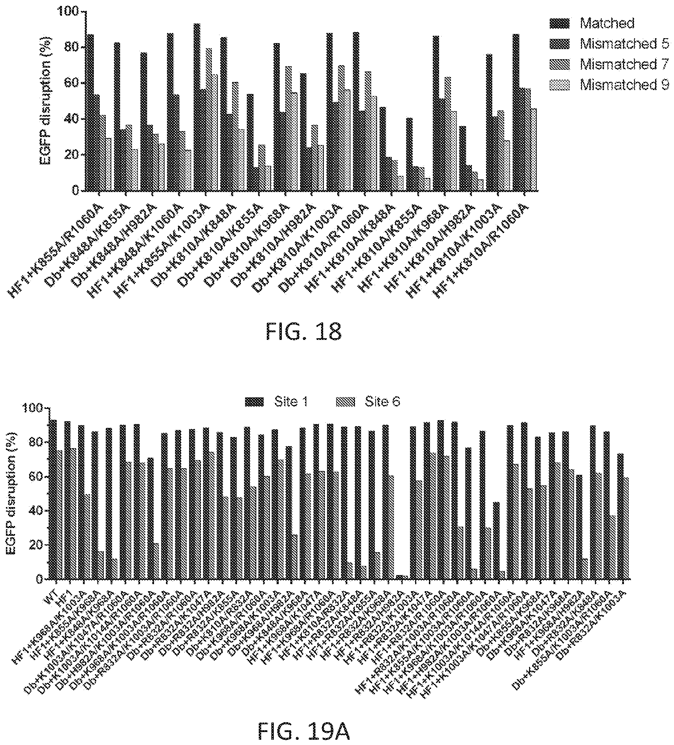

**FIG 18** **| Activities of wild-type SpCas9 and variants using a matched sgRNA and sgRNAs with mismatches at various individual positions in the spacer.** The activities of SpCas9 nucleases containing combinations of alanine substitutions (directed to positions that may potentially contact the target or non-target DNA strands), were assessed using the EGFP disruption assay with an sgRNA that is perfectly matched to a site in the *EGFP* gene ("matched") as well as sgRNAs that are intentionally mismatched at the indicated positions. Db = Q695A/Q926A, HF1 = N497A/R661A/Q695A/Q926A.
**FIGs. 19A-B** **| Activities of wild-type SpCas9 and variants using a matched sgRNA and sgRNAs with mismatches at various individual positions in the spacer**. (A) The on-target activities of SpCas9 nucleases containing combinations of alanine substitutions (directed to positions that may potentially contact the target or non-target DNA strands), were assessed using the EGFP disruption assay with two sgRNAs that are perfectly matched to a site in the *EGFP* gene. (**B**) A subset of these nucleases from (a) were tested with sgRNAs containing mismatches at positions 12, 14, 16, or 18 (of sgRNA 'site 1') in their spacer sequence to determine whether intolerance to mismatches was imparted by these substitutions. Db = Q695A/Q926A, HF1 = N497A/R661A/Q695A/Q926A.

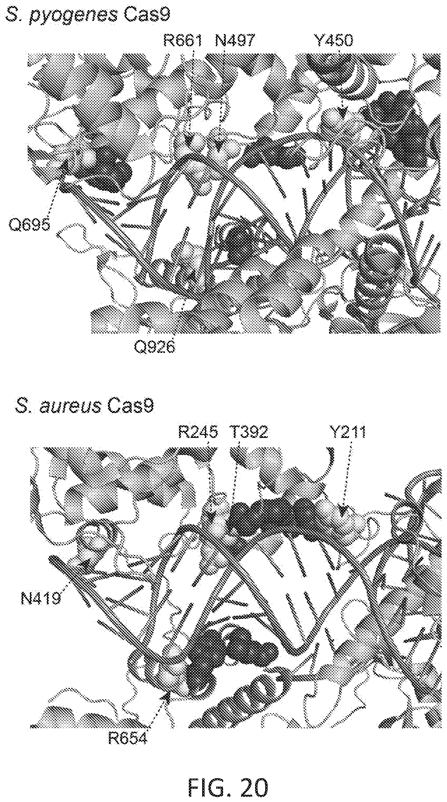

**FIG. 20** | Structural comparison of SpCas9 (top) and SaCas9 (bottom) illustrating the similarity between the positions of the mutations in the quadruple mutant constructs (shown in yellow sphere representation). Also, shown in pink sphere representation are other residues that contact the DNA backbone.

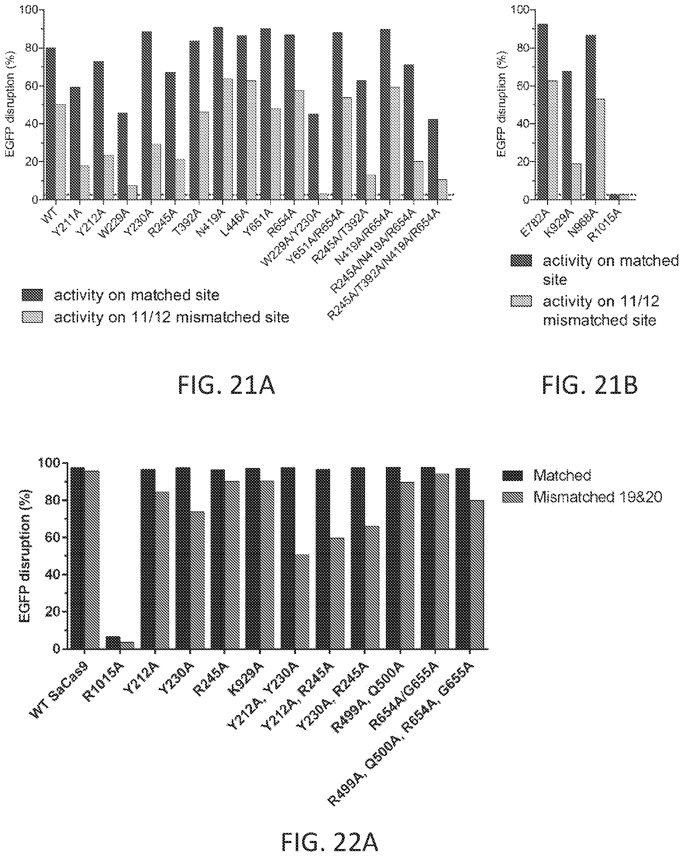

**FIGs. 21A-B** **| Activity of wild-type SaCas9 and SaCas9 derivatives bearing one or more alanine substitutions**. A and B, SaCas9 substitutions were directed to positions that may potentially contact the target DNA strand (panel A) or have previously been shown to influence PAM specificity (panel B). Nucleases were assessed using the EGFP disruption assay, with an sgRNA that is perfectly matched to a site in the *EGFP* gene as well as an sgRNA that is intentionally mismatched at positions 11 and 12. Mismatched positions are numbered with position 20 being the most PAM-distal position; the red dashed line represents background levels of EGFP disruption.
**FIGs. 22A-B** **| Activities of wild-type (WT) SaCas9 and SaCas9 derivatives bearing one or more alanine substitutions at residues that may potentially contact the target DNA strand. A** and **B**, Nucleases were assessed using the EGFP disruption assay, with an sgRNA that is perfectly matched to a site in the *EGFP* gene ("matched") and with an sgRNA that is intentionally mismatched at positions 19 and 20. Mismatched positions are numbered with position 20 being the most PAM-distal position.

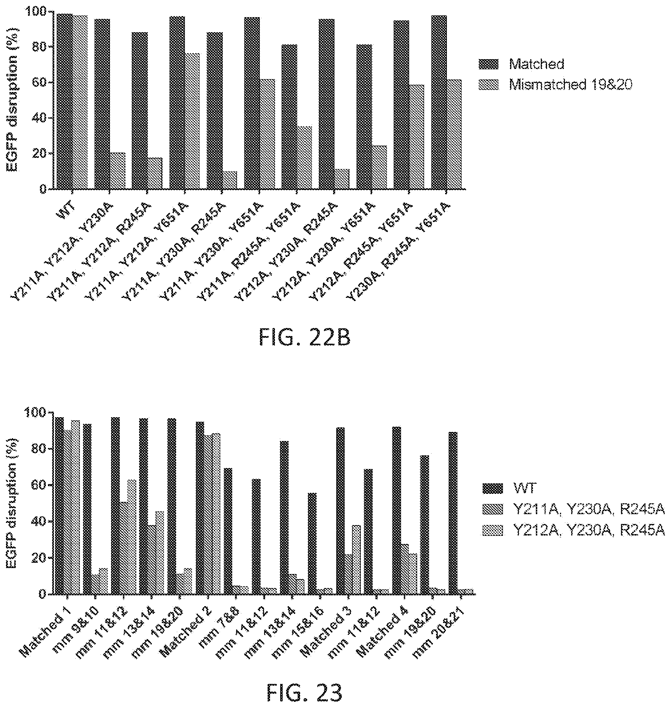

**FIG. 23** **| Activities of wild-type (WT) SaCas9 and SaCas9 variants bearing triple combinations of alanine substitutions at residues that may potentially contact the target DNA strand**. Nucleases were assessed using the EGFP disruption assay. Four different sgRNAs were used (matched #1-4), with each of the four target sites also being tested with mismatched sgRNAs known to be efficiently used by wild-type SaCas9. Mismatched sgRNAs for each site are shown to the right of each matched sgRNA (for example, the only mismatched sgRNA for matched site 3 is mm 11&12). Mismatched positions are numbered with position 21 being the most PAM-distal position; mm, mismatch.

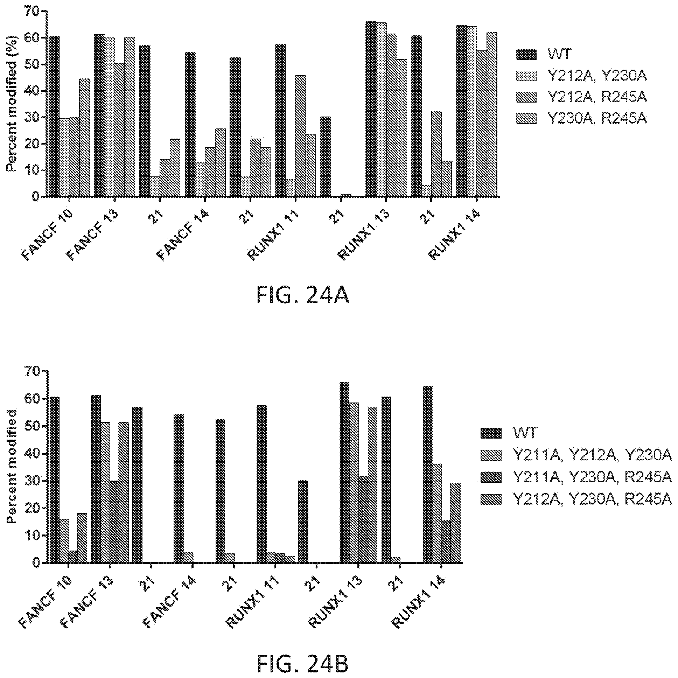

**FIGs. 24A-B** | **Activities of wild-type (WT) SaCas9 and SaCas9 derivatives bearing one or more alanine substitutions at residues that may potentially contact the target DNA strand. A** and **B,** SaCas9 variants bearing double **(A)** or triple **(B)** combinations substitutions were assessed against matched and singly mismatched endogenous human gene target sites using the T7E1 assay. Matched 'on-target' sites are named according to their gene target site sgRNA number from Kleinstiver et al., Nature Biotechnology 2015. Mismatched sgRNAs are numbered with the mismatch occurring at position 21, the most PAM-distal position; mismatched sgRNAs are derived from the matched on-target site that is listed to the left of the mismatched sgRNA.

### DETAILED DESCRIPTION

A limitation of the CRISPR-Cas9 nucleases is their potential to induce undesired "off-target" mutations at imperfectly matched target sites (see, for example, Tsai et al., Nat Biotechnol. 2015), in some cases with frequencies rivaling those observed at the intended on-target site (Fu et al., Nat Biotechnol. 2013). Previous work with CRISPR-Cas9 nucleases has suggested that reducing the number of sequence-specific interactions between the guide RNA (gRNA) and the spacer region of a target site can reduce mutagenic effects at off-target sites of cleavage in human cells (Fu et al., Nat Biotechnol. 2014).

This was earlier accomplished by truncating gRNAs at their 5' ends by 2 or 3 nts and it was hypothesized that the mechanism of this increased specificity was a decrease in the interaction energy of the gRNA/Cas9 complex so that it was poised with just enough energy to cleave the on-target site, making it less likely to have enough energy to cleave off-target sites where there would presumably be an energetic penalty due to mismatches in the target DNA site (WO2015/099850).

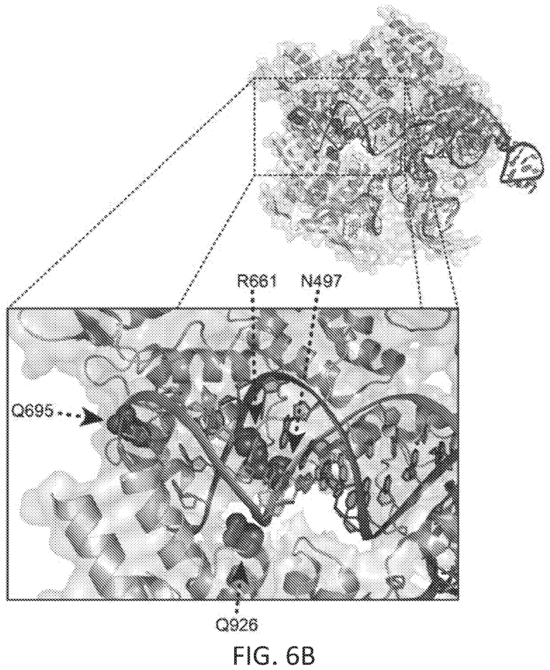

It was hypothesized that off-target effects (at DNA sites that are imperfect matches or mismatches with the intended target site for the guide RNA) of SpCas9 might be minimized by decreasing non-specific interactions with its target DNA site. SpCas9-sgRNA complexes cleave target sites composed of an NGG PAM sequence (recognized by SpCas9) (Deltcheva, E. et al. Nature 471, 602-607 (2011); Jinek, M. et al. Science 337, 816-821 (2012); Jiang, W., et al., Nat Biotechnol 31, 233-239 (2013); Sternberg, S.H., et al., Nature 507, 62-67 (2014)) and an adjacent 20 bp protospacer sequence (which is complementary to the 5' end of the sgRNA) (Jinek, M. et al. Science 337, 816-821 (2012); Jinek, M. et al. Elife 2, e00471 (2013); Mali, P. et al., Science 339, 823-826 (2013); Cong, L. et al., Science 339, 819-823 (2013)). It was previously theorized that the SpCas9-sgRNA complex may possess more energy than is needed for recognizing its intended target DNA site, thereby enabling cleavage of mismatched off-target sites (Fu, Y, et al., Nat Biotechnol 32, 279-284 (2014)). One can envision that this property might be advantageous for the intended role of Cas9 in adaptive bacterial immunity, giving it the capability to cleave foreign sequences that may become mutated. This excess energy model is also supported by previous studies demonstrating that off-target effects can be reduced (but not eliminated) by decreasing SpCas9 concentration (Hsu, P.D. et al. Nat Biotechnol 31, 827-832 (2013); Pattanayak, V. et al. Nat Biotechnol 31, 839-843 (2013)) or by reducing the complementarity length of the sgRNA (Fu, Y., et al., Nat Biotechnol 32, 279-284 (2014), although other interpretations for this effect have also been proposed (Josephs, E.A. et al. Nucleic Acids Res 43, 8924-8941 (2015); Sternberg, S.H., et al. Nature 527, 110-113 (2015); Kiani, S. et al. Nat Methods 12, 1051-1054 (2015))). Structural data suggests that the SpCas9-sgRNA-target DNA complex may be stabilized by several SpCas9-mediated DNA contacts, including direct hydrogen bonds made by four SpCas9 residues (N497, R661, Q695, Q926) to the phosphate backbone of the target DNA strand (Nishimasu, H. et al. Cell 156, 935-949 (2014); Anders, C., et al. Nature 513, 569-573 (2014)) **(****Fig. 1a** and **Figs. 6a** **and** **6b****).** The present inventors envisioned that disruption of one or more of these contacts might energetically poise the SpCas9-sgRNA complex at a level just sufficient to retain robust on-target activity but with a diminished ability to cleave mismatched off-target sites.

As described herein, Cas9 proteins can be engineered to show increased specificity, theoretically by reducing the binding affinity of Cas9 for DNA. Several variants of the widely used *Streptococcus pyogenes* Cas9 (SpCas9) were engineered by introducing individual alanine substitutions into various residues in SpCas9 that might be expected to interact with phosphates on the DNA backbone using structural information, bacterial selection-based directed evolution, and combinatorial design. The variants were further tested for cellular activity using a robust *E.coli-based* screening assay to assess the cellular activities of these variants; in this bacterial system, cell survival depended on cleavage and subsequent destruction of a selection plasmid containing a gene for the toxic gyrase poison ccdB and a 23 base pair sequence targeted by a gRNA and SpCas9, and led to identification of residues that were associated with retained or lost activity. In addition, another SpCas9 variant was identified and characterized, which exhibited improved target specificity in human cells.

Furthermore, activities of single alanine substitution mutants of SpCas9 as assessed in the bacterial cell-based system indicated that survival percentages between 50-100% usually indicated robust cleavage, whereas 0% survival indicated that the enzyme had been functionally compromised. Additional mutations of SpCas9 were then assayed in bacteria to include: R63A, R66A, R69A, R70A, R71A, Y72A, R74A, R75A, K76A, N77A, R78A, R115A, H160A, K163A, R165A, L169A, R403A,T404A, F405A, N407A, R447A, N497A, I448A, Y450A, S460A, M495A, K510A, Y515A, R661A, M694A, Q695A, H698A, Y1013A, V1015A, R1122A, K1123A, K1124A, K1158A, K1185A, K1200A, S1216A, Q1221A, K1289A, R1298A, K1300A, K1325A, R1333A, K1334A, R1335A, and T1337A. With the exception of 2 mutants (R69A and F405A) that had < 5% survival in bacteria, all of these additional single mutations appeared to have little effect on the on-target activity of SpCas9 (>70% survival in the bacterial screen).

To further determine whether the variants of Cas9 identified in the bacterial screen functioned efficiently in human cells, various alanine substitution Cas9 mutants were tested using a human U2OS cell-based EGFP-disruption assay. In this assay, successful cleavage of a target site in the coding sequence of a single integrated, constitutively expressed EGFP gene led to the induction of indel mutations and disruption of EGFP activity, which was quantitatively assessed by flow cytometry (see, for example, Reyon et al., Nat Biotechnol. 2012 May;30(5):460-5).

These experiments show that the results obtained in the bacterial cell-based assay correlate well with nuclease activities in human cells, suggesting that these engineering strategies could be extended to Cas9s from other species and different cells. Thus these findings provide support for SpCas9 and SaCas9 variants, referred to collectively herein as "variants" or "the variants".

All of the variants described herein can be rapidly incorporated into existing and widely used vectors, e.g., by simple site-directed mutagenesis, and because they require only a small number of mutations, the variants should also work with other previously described improvements to the SpCas9 platform (e.g., truncated sgRNAs (Tsai et al., Nat Biotechnol 33, 187-197 (2015); Fu et al., Nat Biotechnol 32, 279-284 (2014)), nickase mutations (Mali et al., Nat Biotechnol 31, 833-838 (2013); Ran et al., Cell 154, 1380-1389 (2013)), FokI-dCas9 fusions (Guilinger et al., Nat Biotechnol 32, 577-582 (2014); Tsai et al., Nat Biotechnol 32, 569-576 (2014); WO2014144288); and engineered CRISPR-Cas9 nucleases with altered PAM specificities (Kleinstiver et al., Nature. 2015 Jul 23;523(7561):481-5).

Thus, provided herein are Cas9 variants, including SpCas9 variants. The SpCas9 wild type sequence is as follows:

The SpCas9 variants described herein can include the amino acid sequence of SEQ ID NO: 1, with mutations (i.e., replacement of the native amino acid with a different amino acid, e.g., alanine, glycine, or serine), at one or more of the following positions: N497, R661, Q695, Q926 (or at positions analogous thereto). The SpCas9 variants of the claimed invention have mutations Q695A and Q926A in SEQ ID NO: 1 and are at least 95% identical to the amino acid sequence of SEQ ID NO: 1, e.g., have differences at up to 5% of the residues of SEQ ID NO: 1 replaced, e.g., with conservative mutations, in addition to the mutations described herein. In preferred embodiments, the variant retains desired activity of the parent, e.g., the nuclease activity (except where the parent is a nickase or a dead Cas9), and/or the ability to interact with a guide RNA and target DNA).

To determine the percent identity of two nucleic acid sequences, the sequences are aligned for optimal comparison purposes (e.g., gaps can be introduced in one or both of a first and a second amino acid or nucleic acid sequence for optimal alignment and non-homologous sequences can be disregarded for comparison purposes). The length of a reference sequence aligned for comparison purposes is at least 80% of the length of the reference sequence, and in some embodiments is at least 90% or 100%. The nucleotides at corresponding amino acid positions or nucleotide positions are then compared. When a position in the first sequence is occupied by the same nucleotide as the corresponding position in the second sequence, then the molecules are identical at that position (as used herein nucleic acid "identity" is equivalent to nucleic acid "homology"). The percent identity between the two sequences is a function of the number of identical positions shared by the sequences, taking into account the number of gaps, and the length of each gap, which need to be introduced for optimal alignment of the two sequences. Percent identity between two polypeptides or nucleic acid sequences is determined in various ways that are within the skill in the art, for instance, using publicly available computer software such as Smith Waterman Alignment (Smith, T. F. and M. S. Waterman (1981) J Mol Biol 147:195-7); "BestFit" (Smith and Waterman, Advances in Applied Mathematics, 482-489 (1981)) as incorporated into GeneMatcher Plus^{™}, Schwarz and Dayhof (1979) Atlas of Protein Sequence and Structure, Dayhof, M.O., Ed, pp 353-358; BLAST program (Basic Local Alignment Search Tool; (Altschul, S. F., W. Gish, et al. (1990) J Mol Biol 215: 403-10), BLAST-2, BLAST-P, BLAST-N, BLAST-X, WU-BLAST-2, ALIGN, ALIGN-2, CLUSTAL, or Megalign (DNASTAR) software. In addition, those skilled in the art can determine appropriate parameters for measuring alignment, including any algorithms needed to achieve maximal alignment over the length of the sequences being compared. In general, for proteins or nucleic acids, the length of comparison can be any length, up to and including full length (e.g., 5%, 10%, 20%, 30%, 40%, 50%, 60%, 70%, 80%, 90%, 95%, or 100%). For purposes of the present compositions and methods, at least 80% of the full length of the sequence is aligned.

For purposes of the present invention, the comparison of sequences and determination of percent identity between two sequences can be accomplished using a Blossum 62 scoring matrix with a gap penalty of 12, a gap extend penalty of 4, and a frameshift gap penalty of 5.

Conservative substitutions typically include substitutions within the following groups: glycine, alanine; valine, isoleucine, leucine; aspartic acid, glutamic acid, asparagine, glutamine; serine, threonine; lysine, arginine; and phenylalanine, tyrosine.

In some embodiments, the SpCas9 variants include one of the following sets of mutations: N497A/R661A/Q695/Q926A (quadruple alanine mutant); Q695A/Q926A (double alanine mutant); R661A/Q695A/Q926A and N497A/Q695A/Q926A (triple alanine mutants). In some embodiments, the additional substitution mutations at L169 and/or Y450 might be added to these double-, triple, and quadruple mutants or added to single mutants bearing substitutions at Q695A and Q926A. In some embodiments, the mutants have alanine in place of the wild type amino acid. In some embodiments, the mutants, other than those at positions Q695A and Q926A,G have any amino acid other than arginine or lysine (or the native amino acid).

In some embodiments, the SpCas9 variants also include one of the following mutations, which reduce or destroy the nuclease activity of the Cas9: D10, E762, D839, H983, or D986 and H840 or N863, e.g., D10A/D10N and H840A/H840N/H840Y, to render the nuclease portion of the protein catalytically inactive; substitutions at these positions could be alanine (as they are in Nishimasu al., Cell 156, 935-949 (2014)), or other residues, e.g., glutamine, asparagine, tyrosine, serine, or aspartate, e.g., E762Q, H983N, H983Y, D986N, N863D, N863S, or N863H (see WO 2014/152432). In some embodiments, the variant includes mutations at D10A or H840A (which creates a single-strand nickase), or mutations at D10A and H840A (which abrogates nuclease activity; this mutant is known as dead Cas9 or dCas9).

The SpCas9 N497A/R661A/Q695A/R926A mutations have analogous residues in Staphylococcus aureus Cas9 (SaCas9); see FIG. 20. Mutations to the residues contacting the DNA or RNA backbone are expected to increase the specificity of SaCas9 as we've observed for SpCas9. Thus, also provided herein, which does not form part of the claimed invention, are SaCas9 variants.

The SaCas9 wild type sequence is as follows:

SaCas9 variants described herein include the amino acid sequence of SEQ ID NO:2, with mutations at one, two, three, four, five, or all six of the following positions: Y211, W229, R245, T392, N419, and/or R654, e.g., comprising a sequence that is at least 80% identical to the amino acid sequence of SEQ ID NO:2 with mutations at one, two, three, four five or six of the following positions: Y211, W229, R245, T392, N419, and/or R654.

In some embodiments which do not form part of the claimed invention, the variant SaCas9 proteins also comprise one or more of the following mutations: Y211A; W229A; Y230A; R245A; T392A; N419A; L446A; Y651A; R654A; D786A; T787A; Y789A; T882A; K886A; N888A; A889A; L909A; N985A; N986A; R991A; R1015A; N44A; R45A; R51A; R55A; R59A; R60A; R116A; R165A; N169A; R208A; R209A; Y211A; T238A; Y239A; K248A; Y256A; R314A; N394A; Q414A; K57A; R61A; H111A; K114A; V164A; R165A; L788A; S790A; R792A; N804A; Y868A; K870A; K878A; K879A; K881A; Y897A; R901A; K906A.

In some embodiments which do not form part of the claimed invention, variant SaCas9 proteins comprise one or more of the following additional mutations: Y211A, W229A, Y230A, R245A, T392A, N419A, L446A, Y651A, R654A, D786A, T787A, Y789A, T882A, K886A, N888A, A889A, L909A, N985A, N986A, R991A, R1015A, N44A, R45A, R51A, R55A, R59A, R60A, R116A, R165A, N169A, R208A, R209A, Y211A, T238A, Y239A, K248A, Y256A, R314A, N394A, Q414A, K57A, R61A, H111A, K114A, V164A, R165A, L788A, S790A, R792A, N804A, Y868A, K870A, K878A, K879A, K881A, Y897A, R901A, K906A.

In some embodiments which do not form part of the claimed invention, the variant SaCas9 proteins comprise multiple substitution mutations: R245/T392/N419/R654 and Y221/R245/N419/R654 (quadruple variant mutants); N419/R654, R245/R654, Y221/R654, and Y221/N419 (double mutants); R245/N419/R654, Y211/N419/R654, and T392/N419/R654 (triple mutants). In some embodiments which do not form part of the claimed invention, the mutants contain alanine in place of the wild type amino acid.

In some embodiments which do not form part of the claimed invention, the variant SaCas9 proteins also comprise mutations at E782K, K929R, N968K, and/or R1015H. For example, the KKH variant (E782K/N968K/R1015H), the KRH variant (E782K/K929R/R1015H), or the KRKH variant (E782K/K929R/N968K/R1015H)]

In some embodiments which do not form part of the claimed invention, the variant SaCas9 proteins also comprise one or more mutations that decrease nuclease activity selected from the group consisting of mutations at D10, E477, D556, H701, or D704; and at H557 or N580.

In some embodiments which do not form part of the claimed invention, the mutations are: (i) D10A or D10N, (ii) H557A, H557N, or H557Y, (iii) N580A, and/or (iv) D556A.

Also provided herein are isolated nucleic acids encoding the Cas9 variants, vectors comprising the isolated nucleic acids, optionally operably linked to one or more regulatory domains for expressing the variant proteins, and host cells, e.g., mammalian host cells, comprising the nucleic acids, and optionally expressing the variant proteins.

The variants described herein can be used for altering the genome of a cell; the methods generally include expressing the variant proteins in the cells, along with a guide RNA having a region complementary to a selected portion of the genome of the cell. Methods for selectively altering the genome of a cell are known in the art, see, e.g., US 8,993,233; US 20140186958; US 9,023,649; WO/2014/099744; WO 2014/089290; WO2014/144592; WO144288; WO2014/204578; WO2014/152432; WO2115/099850; US8,697,359; US20160024529; US20160024524; US20160024523; US20160024510; US20160017366; US20160017301; US20150376652; US20150356239; US20150315576; US20150291965; US20150252358; US20150247150; US20150232883; US20150232882; US20150203872; US20150191744; US20150184139; US20150176064; US20150167000; US20150166969; US20150159175; US20150159174; US20150093473; US20150079681; US20150067922; US20150056629; US20150044772; US20150024500; US20150024499; US20150020223;; US20140356867; US20140295557; US20140273235; US20140273226; US20140273037; US20140189896; US20140113376; US20140093941; US20130330778; US20130288251; US20120088676; US20110300538; US20110236530; US20110217739; US20110002889; US20100076057; US20110189776; US20110223638; US20130130248; US20150050699; US20150071899; US20150050699; ; US20150045546; US20150031134; US20150024500; US20140377868; US20140357530; US20140349400; US20140335620; US20140335063; US20140315985; US20140310830; US20140310828; US20140309487; US20140304853; US20140298547; US20140295556; US20140294773; US20140287938; US20140273234; US20140273232; US20140273231; US20140273230; US20140271987; US20140256046; US20140248702; US20140242702; US20140242700; US20140242699; US20140242664; US20140234972; US20140227787; US20140212869; US20140201857; US20140199767; US20140189896; US20140186958; US20140186919; US20140186843; US20140179770; US20140179006; US20140170753; WO/2008/108989; WO/2010/054108; WO/2012/164565; WO/2013/098244; WO/2013/176772; US 20150071899; Makarova et al., "Evolution and classification of the CRISPR-Cas systems" 9(6) Nature Reviews Microbiology 467-477 (1-23) (Jun. 2011); Wiedenheft et al., "RNA-guided genetic silencing systems in bacteria and archaea" 482 Nature 331-338 (Feb. 16, 2012); Gasiunas et al., "Cas9-crRNAribonucleoprotein complex mediates specific DNA cleavage for adaptive immunity in bacteria" 109(39) Proceedings of the National Academy of Sciences USAE2579-E2586 (Sep. 4, 2012); Jinek et al., "A Programmable Dual-RNA-Guided DNA Endonuclease in Adaptive Bacterial Immunity" 337 Science 816-821 (Aug. 17, 2012); Carroll, "A CRISPR Approach to Gene Targeting" 20(9) Molecular Therapy 1658-1660 (Sep. 2012); U.S. Appl. No. 61/652,086, filed May 25, 2012; Al-Attar et al., Clustered Regularly Interspaced Short Palindromic Repeats (CRISPRs): The Hallmark of an Ingenious Antiviral Defense Mechanism in Prokaryotes, Biol Chem. (2011) vol. 392, Issue 4, pp. 277-289; Hale et al., Essential Features and Rational Design of CRISPR RNAs That Function With the Cas RAMP Module Complex to Cleave RNAs, Molecular Cell, (2012) vol. 45, Issue 3, 292-302.

The variant proteins described herein can be used in place of or in addition to any of the Cas9 proteins described in the foregoing references, or in combination with mutations described therein. In addition, the variants described herein can be used in fusion proteins in place of the wild-type Cas9 or other Cas9 mutations (such as the dCas9 or Cas9 nickase described above) as known in the art, e.g., a fusion protein with a heterologous functional domains as described in US 8,993,233; US 20140186958; US 9,023,649; WO/2014/099744; WO 2014/089290; WO2014/144592; WO144288; WO2014/204578; WO2014/152432; WO2115/099850; US8,697,359; US2010/0076057; US2011/0189776; US2011/0223638; US2013/0130248; WO/2008/108989; WO/2010/054108; WO/2012/164565; WO/2013/098244; WO/2013/176772; US20150050699; US 20150071899 and WO 2014/124284. For example, the variants, preferably comprising one or more nuclease-reducing, -altering, or -killing mutation, can be fused on the N or C terminus of the Cas9 to a transcriptional activation domain or other heterologous functional domains (e.g., transcriptional repressors (e.g., KRAB, ERD, SID, and others, e.g., amino acids 473-530 of the ets2 repressor factor (ERF) repressor domain (ERD), amino acids 1-97 of the KRAB domain of KOX1, or amino acids 1-36 of the Mad mSIN3 interaction domain (SID); see Beerli et al., PNAS USA 95:14628-14633 (1998)) or silencers such as Heterochromatin Protein 1 (HP1, also known as swi6), e.g., HP1α or HPIβ; proteins or peptides that could recruit long non-coding RNAs (IncRNAs) fused to a fixed RNA binding sequence such as those bound by the MS2 coat protein, endoribonuclease Csy4, or the lambda N protein; enzymes that modify the methylation state of DNA (e.g., DNA methyltransferase (DNMT) or TET proteins); or enzymes that modify histone subunits (e.g., histone acetyltransferases (HAT), histone deacetylases (HDAC), histone methyltransferases (e.g., for methylation of lysine or arginine residues) or histone demethylases (e.g., for demethylation of lysine or arginine residues)) as are known in the art can also be used. A number of sequences for such domains are known in the art, e.g., a domain that catalyzes hydroxylation of methylated cytosines in DNA. Exemplary proteins include the Ten-Eleven-Translocation (TET)1-3 family, enzymes that converts 5-methylcytosine (5-mC) to 5-hydroxymethylcytosine (5-hmC) in DNA.

**Sequences for human TET1-3 are known in the art and are shown in the following table:**

| | **GenBank Accession Nos.** | |
|---|---|---|
| Gene | Amino Acid | Nucleic Acid |
| TET1 | NP_085128.2 | NM_030625.2 |
| TET2^{∗} | NP_001120680.1 (var 1) | NM_001127208.2 |
| | NP_060098.3 (var 2) | NM_017628.4 |
| TET3 | NP_659430.1 | NM_144993.1 |

| | | |
|---|---|---|
| ^{∗} Variant (1) represents the longer transcript and encodes the longer isoform (a). Variant (2) differs in the 5' UTR and in the 3' UTR and coding sequence compared to variant 1. The resulting isoform (b) is shorter and has a distinct C-terminus compared to isoform a. | | |

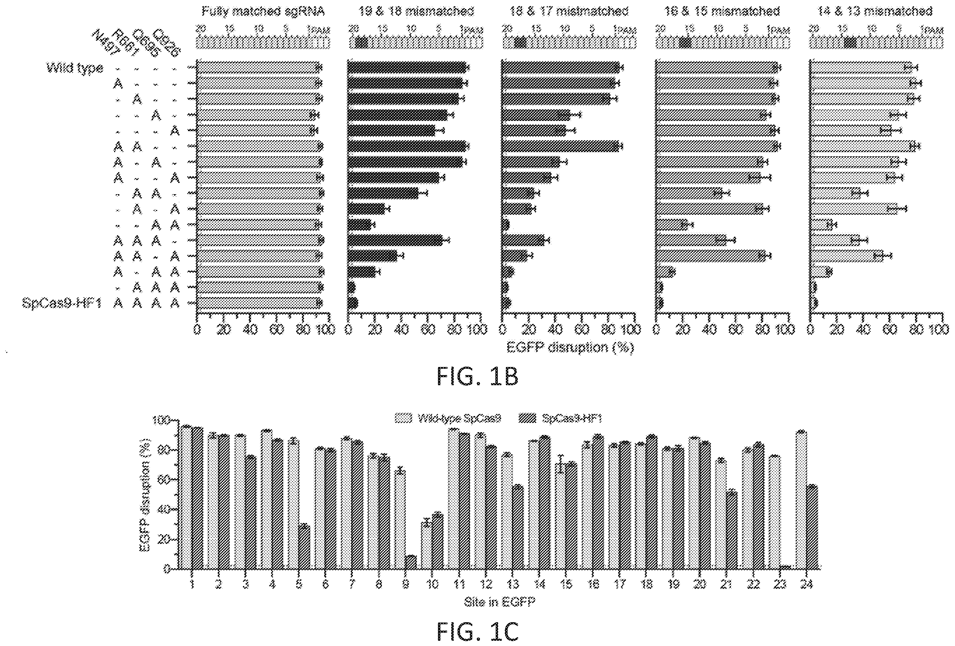

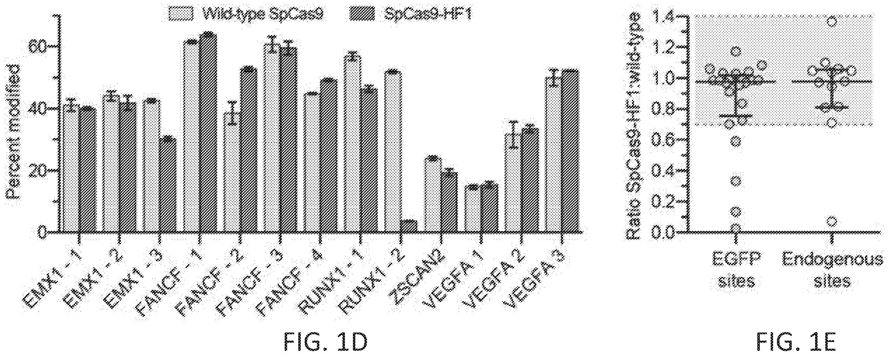

In some embodiments, all or part of the full-length sequence of the catalytic domain can be included, e.g., a catalytic module comprising the cysteine-rich extension and the 2OGFeDO domain encoded by 7 highly conserved exons, e.g., the Tet1 catalytic domain comprising amino acids 1580-2052, Tet2 comprising amino acids 1290-1905 and Tet3 comprising amino acids 966-1678. See, e.g., Fig. 1 of Iyer et al., Cell Cycle. 2009 Jun 1;8(11):1698-710. Epub 2009 Jun 27, for an alignment illustrating the key catalytic residues in all three Tet proteins, and the supplementary materials thereof (available at ftp site ftp.ncbi.nih.gov/pub/aravind/DONS/supplementary_material_DONS.html) for full length sequences (see, e.g., seq 2c); in some embodiments, the sequence includes amino acids 1418-2136 of Tet1 or the corresponding region in Tet2/3.

Other catalytic modules can be from the proteins identified in Iyer et al., 2009.

In some embodiments, the heterologous functional domain is a biological tether, and comprises all or part of (e.g., DNA binding domain from) the MS2 coat protein, endoribonuclease Csy4, or the lambda N protein. These proteins can be used to recruit RNA molecules containing a specific stem-loop structure to a locale specified by the dCas9 gRNA targeting sequences. For example, a dCas9 variant fused to MS2 coat protein, endoribonuclease Csy4, or lambda N can be used to recruit a long non-coding RNA (lncRNA) such as XIST or HOTAIR; see, e.g., Keryer-Bibens et al., Biol. Cell 100:125-138 (2008), that is linked to the Csy4, MS2 or lambda N binding sequence. Alternatively, the Csy4, MS2 or lambda N protein binding sequence can be linked to another protein, e.g., as described in Keryer-Bibens et al., supra, and the protein can be targeted to the dCas9 variant binding site using the methods and compositions described herein. In some embodiments, the Csy4 is catalytically inactive. In some embodiments, the Cas9 variant, preferably a dCas9 variant, is fused to FokI as described in US 8,993,233; US 20140186958; US 9,023,649; WO/2014/099744; WO 2014/089290; WO2014/144592; WO144288; WO2014/204578; WO2014/152432;WO2115/099850; US8,697,359; US2010/0076057; US2011/0189776; US2011/0223638; US2013/0130248; WO/2008/108989; WO/2010/054108; WO/2012/164565; WO/2013/098244; WO/2013/176772; US20150050699; US 20150071899 and WO 2014/204578.

In some embodiments, the fusion proteins include a linker between the dCas9 variant and the heterologous functional domains. Linkers that can be used in these fusion proteins (or between fusion proteins in a concatenated structure) can include any sequence that does not interfere with the function of the fusion proteins. In preferred embodiments, the linkers are short, e.g., 2-20 amino acids, and are typically flexible (i.e., comprising amino acids with a high degree of freedom such as glycine, alanine, and serine). In some embodiments, the linker comprises one or more units consisting of GGGS (SEQ ID NO:3) or GGGGS (SEQ ID NO:4), e.g., two, three, four, or more repeats of the GGGS (SEQ ID NO:5) or GGGGS (SEQ ID NO:6) unit. Other linker sequences can also be used.

In some embodiments, the variant protein includes a cell-penetrating peptide sequence that facilitates delivery to the intracellular space, e.g., HIV-derived TAT peptide, penetratins, transportans, or hCT derived cell-penetrating peptides, see, e.g., Caron et al., (2001) Mol Ther. 3(3):310-8; Langel, Cell-Penetrating Peptides: Processes and Applications (CRC Press, Boca Raton FL 2002); El-Andaloussi et al., (2005) Curr Pharm Des. 11(28):3597-611; and Deshayes et al., (2005) Cell Mol Life Sci. 62(16): 1839-49.

Cell penetrating peptides (CPPs) are short peptides that facilitate the movement of a wide range of biomolecules across the cell membrane into the cytoplasm or other organelles, e.g. the mitochondria and the nucleus. Examples of molecules that can be delivered by CPPs include therapeutic drugs, plasmid DNA, oligonucleotides, siRNA, peptide-nucleic acid (PNA), proteins, peptides, nanoparticles, and liposomes. CPPs are generally 30 amino acids or less, are derived from naturally or non-naturally occurring protein or chimeric sequences, and contain either a high relative abundance of positively charged amino acids, e.g. lysine or arginine, or an alternating pattern of polar and non-polar amino acids. CPPs that are commonly used in the art include Tat (Frankel et al., (1988) Cell. 55:1189-1193, Vives et al., (1997) J. Biol. Chem. 272:16010-16017), penetratin (Derossi et al., (1994) J. Biol. Chem. 269:10444-10450), polyarginine peptide sequences (Wender et al., (2000) Proc. Natl. Acad. Sci. USA 97:13003-13008, Futaki et al., (2001) J. Biol. Chem. 276:5836-5840), and transportan (Pooga et al., (1998) Nat. Biotechnol. 16:857-861).

CPPs can be linked with their cargo through covalent or non-covalent strategies. Methods for covalently joining a CPP and its cargo are known in the art, e.g. chemical cross-linking (Stetsenko et al., (2000) J. Org. Chem. 65:4900-4909, Gait et al. (2003) Cell. Mol. Life. Sci. 60:844-853) or cloning a fusion protein (Nagahara et al., (1998) Nat. Med. 4:1449-1453). Non-covalent coupling between the cargo and short amphipathic CPPs comprising polar and non-polar domains is established through electrostatic and hydrophobic interactions.

CPPs have been utilized in the art to deliver potentially therapeutic biomolecules into cells. Examples include cyclosporine linked to polyarginine for immunosuppression (Rothbard et al., (2000) Nature Medicine 6(11): 1253-1257), siRNA against cyclin B1 linked to a CPP called MPG for inhibiting tumorigenesis (Crombez et al., (2007) Biochem Soc. Trans. 35:44-46), tumor suppressor p53 peptides linked to CPPs to reduce cancer cell growth (Takenobu et al., (2002) Mol. Cancer Ther. 1(12):1043-1049, Snyder et al., (2004) PLoS Biol. 2:E36), and dominant negative forms of Ras or phosphoinositol 3 kinase (PI3K) fused to Tat to treat asthma (Myou et al., (2003) J. Immunol. 171:4399-4405).

CPPs have been utilized in the art to transport contrast agents into cells for imaging and biosensing applications. For example, green fluorescent protein (GFP) attached to Tat has been used to label cancer cells (Shokolenko et al., (2005) DNA Repair 4(4):511-518). Tat conjugated to quantum dots have been used to successfully cross the blood-brain barrier for visualization of the rat brain (Santra et al., (2005) Chem. Commun. 3144-3146). CPPs have also been combined with magnetic resonance imaging techniques for cell imaging (Liu et al., (2006) Biochem. and Biophys. Res. Comm. 347(1): 133-140). See also Ramsey and Flynn, Pharmacol Ther. 2015 Jul 22. pii: S0163-7258(15)00141-2.

Alternatively, or in addition, the variant proteins can include a nuclear localization sequence, e.g., SV40 large T antigen NLS (PKKKRRV (SEQ ID NO:7)) and nucleoplasmin NLS (KRPAATKKAGQAKKKK (SEQ ID NO:8)). Other NLSs are known in the art; see, e.g., Cokol et al., EMBO Rep. 2000 Nov 15; 1(5): 411-415; Freitas and Cunha, Curr Genomics. 2009 Dec; 10(8): 550-557.

In some embodiments, the variants include a moiety that has a high affinity for a ligand, for example GST, FLAG or hexahistidine sequences. Such affinity tags can facilitate the purification of recombinant variant proteins.

For methods in which the variant proteins are delivered to cells, the proteins can be produced using any method known in the art, e.g., by in vitro translation, or expression in a suitable host cell from nucleic acid encoding the variant protein; a number of methods are known in the art for producing proteins. For example, the proteins can be produced in and purified from yeast, *E. coli,* insect cell lines, plants, transgenic animals, or cultured mammalian cells; see, e.g., Palomares et al., "Production of Recombinant Proteins: Challenges and Solutions," Methods Mol Biol. 2004;267:15-52. In addition, the variant proteins can be linked to a moiety that facilitates transfer into a cell, e.g., a lipid nanoparticle, optionally with a linker that is cleaved once the protein is inside the cell. See, e.g., LaFountaine et al., Int J Pharm. 2015 Aug 13;494(1):180-194.

### Expression Systems

To use the Cas9 variants described herein, it may be desirable to express them from a nucleic acid that encodes them. This can be performed in a variety of ways. For example, the nucleic acid encoding the Cas9 variant can be cloned into an intermediate vector for transformation into prokaryotic or eukaryotic cells for replication and/or expression. Intermediate vectors are typically prokaryote vectors, e.g., plasmids, or shuttle vectors, or insect vectors, for storage or manipulation of the nucleic acid encoding the Cas9 variant for production of the Cas9 variant. The nucleic acid encoding the Cas9 variant can also be cloned into an expression vector, for administration to a plant cell, animal cell, preferably a mammalian cell or a human cell, fungal cell, bacterial cell, or protozoan cell.

To obtain expression, a sequence encoding a Cas9 variant is typically subcloned into an expression vector that contains a promoter to direct transcription. Suitable bacterial and eukaryotic promoters are well known in the art and described, e.g., in Sambrook et al., Molecular Cloning, ALaboratory Manual (3d ed. 2001); Kriegler, Gene Transfer and Expression: A Laboratory Manual (1990); and Current Protocols in Molecular Biology (Ausubel et al., eds., 2010). Bacterial expression systems for expressing the engineered protein are available in, e.g., *E. coli, Bacillus* sp., and *Salmonella* (Palva et al., 1983, Gene 22:229-235). Kits for such expression systems are commercially available. Eukaryotic expression systems for mammalian cells, yeast, and insect cells are well known in the art and are also commercially available.

The promoter used to direct expression of a nucleic acid depends on the particular application. For example, a strong constitutive promoter is typically used for expression and purification of fusion proteins. In contrast, when the Cas9 variant is to be administered in vivo for gene regulation, either a constitutive or an inducible promoter can be used, depending on the particular use of the Cas9 variant. In addition, a preferred promoter for administration of the Cas9 variant can be a weak promoter, such as HSV TK or a promoter having similar activity. The promoter can also include elements that are responsive to transactivation, e.g., hypoxia response elements, Gal4 response elements, lac repressor response element, and small molecule control systems such as tetracycline-regulated systems and the RU-486 system (see, e.g., Gossen & Bujard, 1992, Proc. Natl. Acad. Sci. USA, 89:5547; Oligino et al., 1998, Gene Ther., 5:491-496; Wang et al., 1997, Gene Ther., 4:432-441; Neering et al., 1996, Blood, 88:1147-55; and Rendahl et al., 1998, Nat. Biotechnol., 16:757-761).

In addition to the promoter, the expression vector typically contains a transcription unit or expression cassette that contains all the additional elements required for the expression of the nucleic acid in host cells, either prokaryotic or eukaryotic. A typical expression cassette thus contains a promoter operably linked, e.g., to the nucleic acid sequence encoding the Cas9 variant, and any signals required, e.g., for efficient polyadenylation of the transcript, transcriptional termination, ribosome binding sites, or translation termination. Additional elements of the cassette may include, e.g., enhancers, and heterologous spliced intronic signals.

The particular expression vector used to transport the genetic information into the cell is selected with regard to the intended use of the Cas9 variant, e.g., expression in plants, animals, bacteria, fungus, protozoa, etc. Standard bacterial expression vectors include plasmids such as pBR322 based plasmids, pSKF, pET23D, and commercially available tag-fusion expression systems such as GST and LacZ.

Expression vectors containing regulatory elements from eukaryotic viruses are often used in eukaryotic expression vectors, e.g., SV40 vectors, papilloma virus vectors, and vectors derived from Epstein-Barr virus. Other exemplary eukaryotic vectors include pMSG, pAV009/A+, pMTO10/A+, pMAMneo-5, baculovirus pDSVE, and any other vector allowing expression of proteins under the direction of the SV40 early promoter, SV40 late promoter, metallothionein promoter, murine mammary tumor virus promoter, Rous sarcoma virus promoter, polyhedrin promoter, or other promoters shown effective for expression in eukaryotic cells.

The vectors for expressing the Cas9 variants can include RNA Pol III promoters to drive expression of the guide RNAs, e.g., the H1, U6 or 7SK promoters. These human promoters allow for expression of Cas9 variants in mammalian cells following plasmid transfection.

Some expression systems have markers for selection of stably transfected cell lines such as thymidine kinase, hygromycin B phosphotransferase, and dihydrofolate reductase. High yield expression systems are also suitable, such as using a baculovirus vector in insect cells, with the gRNA encoding sequence under the direction of the polyhedrin promoter or other strong baculovirus promoters.

The elements that are typically included in expression vectors also include a replicon that functions in *E. coli,* a gene encoding antibiotic resistance to permit selection of bacteria that harbor recombinant plasmids, and unique restriction sites in nonessential regions of the plasmid to allow insertion of recombinant sequences.

Standard transfection methods are used to produce bacterial, mammalian, yeast or insect cell lines that express large quantities of protein, which are then purified using standard techniques (see, e.g., Colley et al., 1989, J. Biol. Chem., 264:17619-22; Guide to Protein Purification, in Methods in Enzymology, vol. 182 (Deutscher, ed., 1990)). Transformation of eukaryotic and prokaryotic cells are performed according to standard techniques (see, e.g., Morrison, 1977, J. Bacteriol. 132:349-351; Clark-Curtiss & Curtiss, Methods in Enzymology 101:347-362 (Wu et al., eds, 1983).

Any of the known procedures for introducing foreign nucleotide sequences into host cells may be used. These include the use of calcium phosphate transfection, polybrene, protoplast fusion, electroporation, nucleofection, liposomes, microinjection, naked DNA, plasmid vectors, viral vectors, both episomal and integrative, and any of the other well-known methods for introducing cloned genomic DNA, cDNA, synthetic DNA or other foreign genetic material into a host cell (see, e.g., Sambrook et al., supra). It is only necessary that the particular genetic engineering procedure used be capable of successfully introducing at least one gene into the host cell capable of expressing the Cas9 variant.

The present methods can also include modifying gDNA by introducing purified Cas9 protein with a gRNA into cells as a ribonuclear protein (RNP) complex, as well as introducing a gRNA plus mRNA encoding the Cas9 protein. The gRNA can be synthetic gRNA or a nucleic acid (e.g., in an expression vector) encoding the guide RNA.

The present invention also includes the vectors and cells comprising the vectors.

### EXAMPLES

The invention is further described in the following examples, which do not limit the scope of the invention described in the claims. Any examples falling outside the scope of the claims are provided for comparative purposes only.

### Methods

### Bacterial-based positive selection assay for evolving SpCas9 variants

Competent *E.coli* BW25141(λDE3)²³ containing a positive selection plasmid (with embedded target site) were transformed with Cas9/sgRNA-encoding plasmids. Following a 60 minute recovery in SOB media, transformations were plated on LB plates containing either chloramphenicol (non-selective) or chloramphenicol + 10 mM arabinose (selective).

To identify additional positions that might be critical for genome wide target specificity, a bacterial selection system previously used to study properties of homing endonucleases (hereafter referred to as the positive selection) (Chen & Zhao, Nucleic Acids Res 33, e154 (2005); Doyon et al., J Am Chem Soc 128, 2477-2484 (2006)) was adapted.

In the present adaptation of this system, Cas9-mediated cleavage of a positive selection plasmid encoding an inducible toxic gene enables cell survival, due to subsequent degradation and loss of the linearized plasmid. After establishing that SpCas9 can function in the positive selection system, both wild-type and the variants were tested for their ability to cleave a selection plasmid harboring a target site selected from the known human genome. These variants were introduced into bacteria with a positive selection plasmid containing a target site and plated on selective medium. Cleavage of the positive selection plasmid was estimated by calculating the survival frequency: colonies on selective plates / colonies on non-selective plates **(see** **FIG. 1****,** **5-6****). A subset of plasmids used in this study (sequences shown below)**

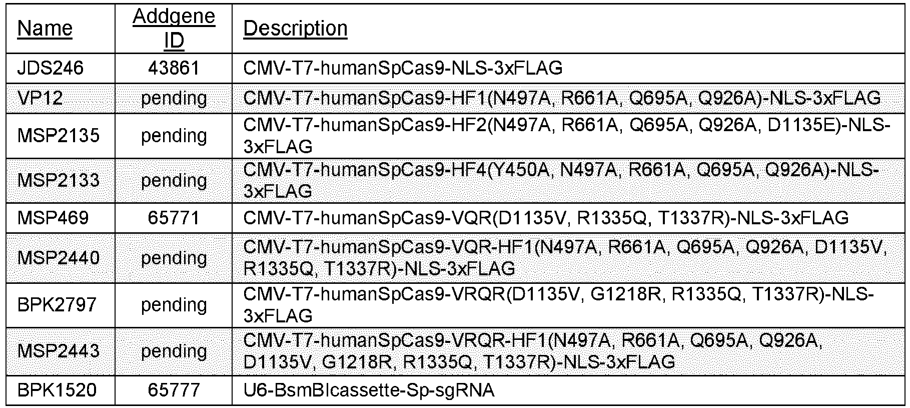

### Human cell culture and transfection

U2OS.EGFP cells harboring a single integrated copy of a constitutively expressed EGFP-PEST reporter gene¹⁵ were cultured in Advanced DMEM media (Life Technologies) supplemented with 10% FBS, 2 mM GlutaMax (Life Technologies), penicillin/streptomycin, and 400 µg/ml of G418 at 37 °C with 5% CO₂. Cells were co-transfected with 750 ng of Cas9 plasmid and 250 ng of sgRNA plasmid (unless otherwise noted) using the DN-100 program of a Lonza 4D-nucleofector according to the manufacturer's protocols. Cas9 plasmid transfected together with an empty U6 promoter plasmid was used as a negative control for all human cell experiments. (see **FIGs. 2****,** **7-10****).**

### Human cell EGFP disruption assay

EGFP disruption experiments were performed as previously described¹⁶. Transfected cells were analyzed for EGFP expression ~52 hours post-transfection using a Fortessa flow cytometer (BD Biosciences). Background EGFP loss was gated at approximately 2.5% for all experiments (see **FIGs. 2****,** **7****).**

### T7E1 assay, targeted deep-sequencing, and GUIDE-seq to quantify nuclease-induced mutation rates

T7E1 assays were performed as previously described for human cells (Kleinstiver, B.P. et al., Nature 523, 481-485 (2015)). For U2OS.EGFP human cells, genomic DNA was extracted from transfected cells ~72 hours post-transfection using the Agencourt DNAdvance Genomic DNA Isolation Kit (Beckman Coulter Genomics). Roughly 200 ng of purified PCR product was denatured, annealed, and digested with T7E1 (New England BioLabs). Mutagenesis frequencies were quantified using a Qiaxcel capillary electrophoresis instrument (QIagen), as previously described for human cells (Kleinstiver et al., Nature 523, 481-485 (2015); Reyon et al,. Nat Biotechnol 30, 460-465 (2012)).

GUIDE-seq experiments were performed as previously described (Tsai et al., Nat Biotechnol 33, 187-197 (2015)). Briefly, phosphorylated, phosphorothioate-modified double-stranded oligodeoxynucleotides (dsODNs) were transfected into U2OS cells with Cas9 nuclease along with Cas9 and sgRNA expression plasmids, as described above. dsODN-specific amplification, high-throughput sequencing, and mapping were performed to identify genomic intervals containing DSB activity. For wild-type versus double or quadruple mutant variant experiments, off-target read counts were normalized to the on-target read counts to correct for sequencing depth differences between samples. The normalized ratios for wild-type and variant SpCas9 were then compared to calculate the fold-change in activity at off-target sites. To determine whether wild-type and SpCas9 variant samples for GUIDE-seq had similar oligo tag integration rates at the intended target site, restriction fragment length polymorphism (RFLP) assays were performed by amplifying the intended target loci with Phusion Hot-Start Flex from 100 ng of genomic DNA (isolated as described above). Roughly 150 ng of PCR product was digested with 20 U of NdeI (New England BioLabs) for 3 hours at 37 °C prior to clean-up using the Agencourt Ampure XP kit. RFLP results were quantified using a Qiaxcel capillary electrophoresis instrument (QIagen) to approximate oligo tag integration rates. T7E1 assays were performed for a similar purpose, as described above.

### Example 1

One potential solution to address targeting specificity of CRISPR-Cas9 RNA guided gene editing would be to engineer Cas9 variants with novel mutations.

Based on these earlier results, it was hypothesized (without wishing to be bound by theory) that the specificity of CRISPR-Cas9 nucleases might be significantly increased by reducing the non-specific binding affinity of Cas9 for DNA, mediated by the binding to the phosphate groups on the DNA or hydrophobic or base stacking interactions with the DNA. This approach would have the advantage of not decreasing the length of the target site recognized by the gRNA/Cas9 complex, as in the previously described truncated gRNA approach. It was reasoned that non-specific binding affinity of Cas9 for DNA might be reduced by mutating amino acid residues that contact phosphate groups on the target DNA.

An analogous approach has been used to create variants of non-Cas9 nucleases such as TALENs (see, for example, Guilinger et al., Nat. Methods. 11: 429 (2014)).

In an initial test of the hypothesis, the present inventors attempted to engineer a reduced affinity variant of the widely used *S. pyogenes* Cas9 (SpCas9) by introducing individual alanine substitutions into various residues in SpCas9 that might be expected to interact with phosphates on the DNA backbone. An E.coli-based screening assay was used to assess the activities of these variants (Kleinstiver et al., Nature. 2015 Jul 23;523(7561):481-5). In this bacterial system, cell survival depended on cleavage (and subsequent destruction) of a selection plasmid containing a gene for the toxic gyrase poison ccdB and a 23 base pair sequence targeted by a gRNA and SpCas9. Results of this experiment identified residues that retained or lost activity **(Table 1)**.

**Table 1: Activities of single alanine substitution mutants of Cas9 as assessed in the bacterial cell-based system shown in FIG. 1.**

| **mutation** | **% survival** | | **mutation** | **% survival** | | **mutation** | **% survival** |
|---|---|---|---|---|---|---|---|
| R63A | 84.2 | | Q926A | 53.3 | | K1158A | 46.5 |
| R66A | 0 | | K1107A | 47.4 | | K1185A | 19.3 |
| R70A | 0 | | E1108A | 40.0 | | K1200A | 24.5 |
| R74A | 0 | | S1109A | 96.6 | | S1216A | 100.4 |
| R78A | 56.4 | | K1113A | 51.8 | | Q1221A | 98.8 |
| R165A | 68.9 | | R1114A | 47.3 | | K1289A | 55.2 |
| R403A | 85.2 | | S1116A | 73.8 | | R1298A | 28.6 |
| N407A | 97.2 | | K1118A | 48.7 | | K1300A | 59.8 |
| N497A | 72.6 | | D1135A | 67.2 | | K1325A | 52.3 |
| K510A | 79.0 | | S1136A | 69.2 | | R1333A | 0 |
| Y515A | 34.1 | | K1151A | 0 | | K1334A | 87.5 |
| R661A | 75.0 | | K1153A | 76.6 | | R1335A | 0 |
| Q695A | 69.8 | | K1155A | 44.6 | | T1337A | 64.6 |

Survival percentages between 50-100% usually indicated robust cleavage, whereas 0% survival indicated that the enzyme has been functionally compromised. Additional mutations that were assayed in bacteria (but are not shown in the table above) include: R69A, R71A, Y72A, R75A, K76A, N77A, R115A, H160A, K163A, L169A, T404A, F405A, R447A, I448A, Y450A, S460A, M495A, M694A, H698A, Y1013A, V1015A, R1122A, K1123A, and K1124A. With the exception of R69A and F405A (which had < 5% survival in bacteria), all of these additional single mutations appeared to have little effect on the on-target activity of SpCas9 (>70% survival in the bacterial screen).

15 different SpCas9 variants bearing all possible single, double, triple and quadruple combinations of the N497A, R661A, Q695A, and Q926A mutations were constructed to test whether contacts made by these residues might be dispensable for on-target activity **(****Fig. 1b****)**. For these experiments, a previously described human cell-based assay was used in which cleavage and induction of insertion or deletion mutations **(indels)** by non-homologous end-joining **(NHEJ)-mediated** repair within a single integrated EGFP reporter gene leads to loss of cell fluorescence (Reyon, D. et al., Nat Biotechnol. 30, 460-465, 2012). Using a EGFP-targeted sgRNA previously shown to efficiently disrupt EGFP expression in human cells when paired with wild-type SpCas9 (Fu, Y. et al., Nat Biotechnol 31, 822-826 (2013), all 15 SpCas9 variants possessed EGFP disruption activities comparable to that of wild-type SpCas9 **(****Fig. 1b**, grey bars). Thus, substitution of one or all of these residues did not reduce on-target cleavage efficiency of SpCas9 with this EGFP-targeted sgRNA.

Next, experiments were performed to assess the relative activities of all 15 SpCas9 variants at mismatched target sites. To do this, the EGFP disruption assay was repeated with derivatives of the EGFP-targeted sgRNA used in the previous experiment that contain pairs of substituted bases at positions 13 and 14, 15 and 16, 17 and 18, and 18 and 19 (numbering starting with 1 for the most PAM-proximal base and ending with 20 for the most PAM-distal base; **Fig. 1b****)**. This analysis revealed that one of the triple mutants (R661A/Q695A/Q926A) and the quadruple mutant (N497A/R661A/Q695A/Q926A) both showed levels of EGFP disruption equivalent to that of background with all four of the mismatched sgRNAs **(****Fig. 1b****,** colored bars). Notably, among the 15 variants, those possessing the lowest activities with the mismatched sgRNAs all harbored the Q695A and Q926A mutations. Based on these results and similar data from an experiment using a sgRNA for another EGFP target site, the quadruple mutant (N497A/R661A/Q695A/Q926A) was chosen for additional analysis and designated it as **SpCas9-HF1** (for high-fidelity variant #1).

### On-target activities of SpCas9-HF1

To determine how robustly SpCas9-HF 1 functions at a larger number of on-target sites, direct comparisons were performed between this variant and wild-type SpCas9 using additional sgRNAs. In total, 37 different sgRNAs were tested: 24 targeted to EGFP (assayed with the EGFP disruption assay) and 13 targeted to endogenous human gene targets (assayed using the T7 Endonuclease I **(T7EI)** mismatch assay). 20 of the 24 sgRNAs tested with the EGFP disruption assay **(****Fig. 1c**) and 12 of the 13 sgRNAs tested on endogenous human gene sites **(****Fig. 1d****)** showed activities with SpCas9-HF1 that were at least 70% as active as wild-type SpCas9 with the same sgRNA **(****Fig. 1e****)**. Indeed, SpCas9-HF1 showed highly comparable activities (90-140%) to wild-type SpCas9 with the vast majority of sgRNAs (Fig. **1e**). Three of the 37 sgRNAs tested showed essentially no activity with SpCas9-HF1 and examination of these target sites did not suggest any obvious differences in the characteristics of these sequences compared to those for which high activities were seen **(Table 3).** Overall, SpCas9-HF1 possessed comparable activities (greater than 70% of wild-type SpCas9 activities) for 86% (32/37) of the sgRNAs tested.

**Table 3: List of sgRNA targets**

| ***S. pyogenes sgRNAs*** | | | | | | |
|---|---|---|---|---|---|---|
| | | | | | | |

| **EGFP** | | | | | | |
|---|---|---|---|---|---|---|
| **Prep Name** | **Name** | **Spacer length (nt)** | **Spacer Sequence** | **SEQ ID NO:** | **Sequence with extended PAM** | **SEQ ID NO:** |
| FYF1 320 | NGG site 1 | 20 | | 9. | | 10. |
| FYF1 641 | NGG site 1 | 18 | | 11. | | 12. |
| CK10 12 | NGG site 1-13&14 | 20 | | 13. | | 14. |
| FYF1 429 | NGG site 1-15&16 | 20 | | 15. | | 16. |
| FYF1 430 | NGG site 1-17&18 | 20 | | 17. | | 18. |
| FYF1 347 | NGG site 1-18&19 | 20 | | 19. | | 20. |
| BPK1 345 | NGG site 2 | 20 | | 21. | | 22. |
| BPK1 350 | NGG site 3 | 20 | | 23. | | 24. |
| BPK1 353 | NGG site 4 | 20 | | 25. | | 26. |
| MSP7 92 | NGG site 5 | 20 | | 27. | | 28. |
| MSP7 95 | NGG site 6 | 20 | | 29. | | 30. |
| FYF1 328 | NGG site 7 | 20 | | 31. | | 32. |
| JAF1 001 | NGG site 7 | 17 | | 33. | | 34. |
| BPK1 365 | NGG site 8 | 20 | | 35. | | 36. |
| MSP7 94 | NGG site 9 | 20 | | 37. | | 38. |
| FYF1 327 | NGG site 10 | 20 | | 39. | | 40. |
| JAF9 97 | NGG site 10 | 17 | | 41. | | 42. |
| BPK1 347 | NGG site 11 | 20 | | 43. | | 44. |
| BPK1 369 | NGG site 12 | 20 | | 45. | | 46. |
| MSP2 545 | NGG site 13 | 20 | | 47. | | 48. |
| MSP2 546 | NGG site 14 | 20 | | 49. | | 50. |
| MSP2 547 | NGG site 15 | 20 | | 51. | | 52. |
| MSP2 548 | NGG site 16 | 20 | | 53. | | 54. |
| MSP2 549 | NGG site 17 | 20 | | 55. | | 56. |
| MSP2 550 | NGG site 18 | 20 | | 57. | | 58. |
| MSP2 551 | NGG site 19 | 20 | | 59. | | 60. |
| MSP2 553 | NGG site 20 | 20 | | 61. | | 62. |
| MSP2 554 | NGG site 21 | 20 | | 63. | | 64. |
| MSP2 555 | NGG site 22 | 20 | | 65. | | 66. |
| 555 MSP2 556 | site 22 NGG site 23 | 20 | | 67. | | 68. |
| FYF1 331 | NGG site 24 | 20 | | 69. | | 70. |
| FYF1 560 | NGG site 24 | 18 | | 71. | | 72. |
| BPK1 348 | NGG site 25-no5'G | 20 | | 73. | | 74. |
| BPK1 349 | NGG site 25-mm 5' G | 20 | | 75. | | 76. |
| BPK1 351 | NGG site 26-no 5' G | 20 | | 77. | | 78. |
| BPK1 352 | NGG site 26-mm 5' G | 20 | | 79. | | 80. |
| BPK1 373 | NGG site 27-no5'G | 20 | | 81. | | 82. |
| BPK1 375 | NGG site 27-mm 5' G | 20 | | 83. | | 84. |
| BPK1 377 | NGG site 28-no 5' G | 20 | | 85. | | 86. |
| BPK1 361 | NGG site 28-mm 5' G | 20 | | 87. | | 88. |
| BPK1 468 | NGAA site 1 | 20 | | 89. | | 90. |
| MSP8 07 | NGAA site 2 | 20 | | 91. | | 92. |
| MSP1 70 | NGAC site 1 | 20 | | 93. | | 94. |
| MSP7 90 | NGAC site 2 | 20 | | 95. | | 96. |
| MSP1 71 | NGAT 1 site 1 | 20 | | 97. | | 98. |
| MSP1 69 | NGAT site 2 | 20 | | 99. | | 100. |
| MSP1 68 | NGAG site 1 | 20 | | 101. | | 102. |
| MSP3 66 | NGAG site 2 | 20 | | 103. | | 104. |
| Endogenous genes | | | | | | |
| ***EMX1*** | | | | | | |

| **Prep Name** | **Name** | **Spacer length (nt)** | **Spacer Sequence** | **SEQ ID NO:** | **Sequence with extended PAM** | **SEQ ID NO:** |
|---|---|---|---|---|---|---|
| FYF1 548 | NGG site 1 | 20 | | 105. | | 106. |
| MSP8 09 | NGG site 2 | 20 | | 107. | | 108. |
| VC47 5 | NGG site 3 | 20 | | 109. | | 110. |
| MSP8 14 ^{∗}1 | NGA site 1 | 20 | | 111. | | 112. |
| | | | | | | |
| *FANCF* | | | | | | |

| **Prep Name** | **Name** | **Spacer length (nt)** | **Spacer Sequence** | **SEQ ID NO:** | **Sequence with extended PAM** | **SEQ ID NO:** |
|---|---|---|---|---|---|---|
| DR34 8 | NGG site 1 | 20 | | 113. | | 114. |
| MSP8 15 | NGG site 2 | 20 | | 115. | | 116. |
| MSP8 16 | NGG site 3 | 20 | | 117. | | 118. |
| MSP8 17 | NGG site 4 | 20 | | 119. | | 120. |
| MSP8 18 ^{∗}2 | NGA site 1 | 20 | | 121. | | 122. |
| MSP8 20 ^{∗}3 | NGA site 2 | 20 | | 123. | | 124. |
| MSP8 85 ^{∗}4 | NGA site 3 | 20 | | 125. | | 126. |
| | | | | | | |
| *RUNX1* | | | | | | |

| **Prep Name** | **Name** | **Spacer length (nt)** | **Spacer Sequence** | **SEQ ID NO:** | **Sequence with extended PAM** | **SEQ ID NO:** |
|---|---|---|---|---|---|---|
| MSP8 22 | NGG site 1 | 20 | | 127. | | 128. |
| MSP8 25 | NGG site 2 | 20 | | 129. | | 130. |
| MSP8 26 ^{∗}5 | NGA site 1 | 20 | | 131. | | 132. |
| MSP8 28 ^{∗}6 | NGA site 2 | 20 | | 133. | | 134. |
| MSP1 725 | NGAA site 1 | 20 | | 135. | | 136. |
| MSP1 726 | NGAA site 2 | 20 | | 137. | | 138. |
| MSP1 728 | NGAC site 1 | 20 | | 139. | | 140. |
| MSP1 730 | NGAC site 2 | 20 | | 141. | | 142. |
| MSP1 732 | NGAT site 1 | 20 | | 143. | | 144. |
| MSP8 29 | NGAT site 2 | 20 | | 145. | | 146. |
| MSP1 734 | NGAG site 1 | 20 | | 147. | | 148. |
| MSP8 28 | NGAG site 2 | 20 | | 149. | | 150. |
| | | | | | | |
| ***ZSCAN2*** | | | | | | |

| **Prep Name** | **Name** | **Spacer length (nt)** | **Spacer Sequence** | **SEQ ID NO:** | **Sequence with extended PAM** | **SEQ ID NO:** |
|---|---|---|---|---|---|---|
| NN67 5 | NGG site | 20 | | 151. | | 152. |
| | | | | | | |
| ***VEGFA*** | | | | | | |

| **Prep Name** | **Name** | **Spacer length (nt)** | **Spacer Sequence** | **SEQ ID NO:** | **Sequence with extended PAM** | **SEQ ID NO:** |
|---|---|---|---|---|---|---|
| VC29 7 | NGG site 1 | 20 | | 153. | | 154. |
| VC29 9 | NGG site 2 | 20 | | 155. | | 156. |
| VC22 8 | NGG site 3 | 20 | | 157. | | 158. |
| BPK1 846 ^{∗}7 | NGA site 1 | 20 | | 159. | | 160. |
| | | | | | | |
| *ZNF629* | | | | | | |

| **Prep Name** | **Name** | **Spacer length (nt)** | **Spacer Sequence** | **SEQ ID NO:** | **Sequence with extended PAM** | **SEQ ID NO:** |
|---|---|---|---|---|---|---|
| NN67 5 ^{∗}8 | NGA site | 20 | | 161. | | 162. |

| | | | | | | |
|---|---|---|---|---|---|---|
| ^{∗}1, NGA EMX1 site 4 from Kleinstiver et al., Nature 2015 ^{∗}2, NGA FANCF site 1 from Kleinstiver et al., Nature 2015 ^{∗}3, NGA FANCF site 3 from Kleinstiver et al., Nature 2015 ^{∗}4, NGA FANCF site 4 from Kleinstiver et al., Nature 2015 ^{∗}5, NGA RUNX1 site 1 from Kleinstiver et al., Nature 2015 ^{∗}6, NGA RUNX1 site 3 from Kleinstiver et al., Nature 2015 ^{∗}7, NGA VEGFA site 1 from Kleinstiver et al., Nature 2015 ^{∗}8, NGA ZNF629 site from Kleinstiver et al., Nature 2015 | | | | | | |

### Genome-wide specificity of SpCas9-HF1

To test whether SpCas9-HF1 exhibited reduced off-target effects in human cells, the genome-wide unbiased identification of double-stranded breaks enabled by sequencing **(GUIDE-seq)** method was used. GUIDE-seq uses integration of a short double-stranded oligodeoxynucleotide **(dsODN)** tag into double-strand breaks to enable amplification and sequencing of adjacent genomic sequence, with the number of tag integrations at any given site providing a quantitative measure of cleavage efficiency (Tsai, S.Q. et al,Nat Biotechnol 33, 187-197 (2015)). GUIDE-seq was used to compare the spectrum of off-target effects induced by wild-type SpCas9 and SpCas9-HF1 using eight different sgRNAs targeted to various sites in the endogenous human *EMX1, FANCF, RUNX1,* and *ZSCAN2* genes. The sequences targeted by these sgRNAs are unique and have variable numbers of predicted mismatched sites in the reference human genome **(Table 2).** Assessment of on-target dsODN tag integration (by restriction fragment length polymorphism **(RFLP)** assay) and indel formation (by T7EI assay) for the eight sgRNAs revealed comparable on-target activities with wild-type SpCas9 and SpCas9-HF1 **(****Figs. 7a** and **7b****,** respectively). GUIDE-seq experiments showed that seven of the eight sgRNAs induced cleavage at multiple genome-wide off-target sites (ranging from 2 to 25 per sgRNA) with wild-type SpCas9, whereas the eighth sgRNA (for FANCF site 4) did not produce any detectable off-target sites **(****Figs. 2a** and **2b****).** However, six of the seven sgRNAs that induced indels with wild-type SpCas9 showed a strikingly complete absence of GUIDE-seq detectable off-target events with SpCas9-HF1 **(****Figs. 2a** and **2b****);** and the remaining seventh sgRNA (for FANCF site 2) induced only a single detectable genome-wide off-target cleavage event, at a site harboring one mismatch within the protospacer seed sequence **(****Fig. 2a****).** Collectively, the off-target sites that were not detected when using SpCas9-HF1 harbored one to six mismatches in the protospacer and/or PAM sequence **(****Fig. 2c****).** As with wild-type SpCas9, the eighth sgRNA (for FANCF site 4) did not yield any detectable off-target cleavage events when tested with SpCas9-HF1 (**Fig. 2a****).**

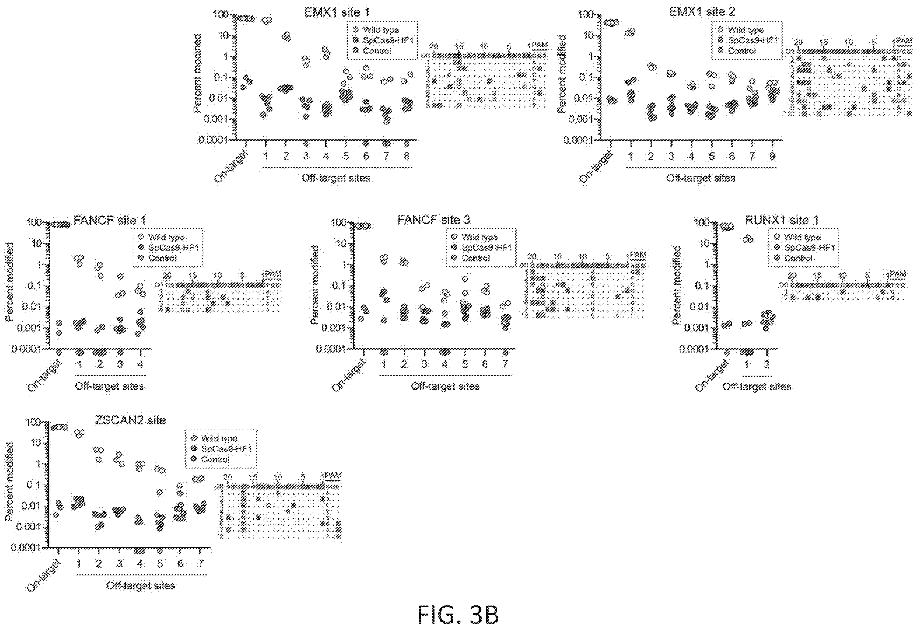

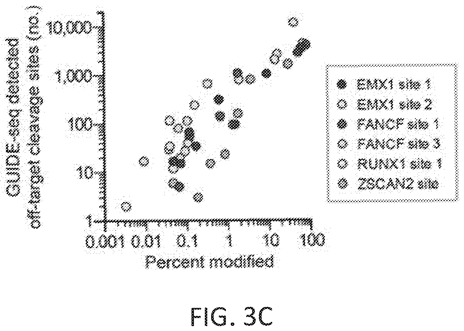

To confirm the GUIDE-seq findings, targeted amplicon sequencing was used to more directly measure the frequencies of NHEJ-mediated indel mutations induced by wild-type SpCas9 and SpCas9-HF1. For these experiments, human cells were transfected only with sgRNA- and Cas9-encoding plasmids (i.e., without the GUIDE-seq tag). Next-generation sequencing was then used to examine 36 of the 40 off-target sites that had been identified with wild-type SpCas9 for six sgRNAs in the GUIDE-seq experiments (four of the 40 sites could not be examined because they could not be specifically amplified from genomic DNA). These deep sequencing experiments showed that: (1) wild-type SpCas9 and SpCas9-HF1 induced comparable frequencies of indels at each of the six sgRNA on-target sites **(****Figs. 3a** and **3b****);** (2) wild-type SpCas9, as expected showed statistically significant evidence of indel mutations at 35 of the 36 off-target sites **(****Fig. 3b****)** at frequencies that correlated well with GUIDE-seq read counts for these same sites **(****Fig. 3c****);** and (3) the frequencies of indels induced by SpCas9-HF1 at 34 of the 36 off-target sites were indistinguishable from the background level of indels observed in samples from control transfections **(****Fig. 3b****).** For the two off-target sites that appeared to have statistically significant mutation frequencies with SpCas9-HF1 relative to the negative control, the mean frequencies of indels were 0.049% and 0.037%, levels at which it is difficult to determine whether these are due to sequencing/PCR error or are *bona fide* nuclease-induced indels. Based on these results, it was concluded that SpCas9-HF1 can completely or nearly completely reduce off-target mutations that occur across a range of different frequencies with wild-type SpCas9 to undetectable levels.

Next the capability of SpCas9-HF1 to reduce genome-wide off-target effects of sgRNAs that target atypical homopolymeric or repetitive sequences was assessed. Although many now try to avoid on-target sites with these characteristics due to their relative lack of orthogonality to the genome, it was desirable to explore whether SpCas9-HF1 might reduce off-target indels even for these challenging targets. Therefore, previously characterized sgRNAs (Fu, Y. et al., Nat Biotechnol 3 1,Tsai, S.Q. et al., Nat Biotechnol 33, 187-197 (2015) were used that target either a cytosinerich homopolymeric sequence or a sequence containing multiple TG repeats in the human *VEGFA* gene (VEGFA site 2 and VEGFA site 3, respectively) **(Table 2).** In control experiments, each of these sgRNAs induced comparable levels of GUIDE-seq ds ODN tag incorporation **(****Fig. 7c****)** and indel mutations **(****Fig. 7d****)** with both wild-type SpCas9 and SpCas9-HF1, demonstrating that SpCas9-HF1 was not impaired in on-target activity with either of these sgRNAs. Importantly, GUIDE-seq experiments revealed that SpCas9-HF1 was highly effective at reducing off-target sites of these sgRNAs, with 123/144 sites for VEGFA site 2 and 31/32 sites for VEGFA site 3 not detected **(****Figs. 4a** and **4b****).** Examination of these off-target sites not detected with SpCas9-HF1 showed that they each possessed a range of total mismatches within their protospacer and PAM sequences: 2 to 7 mismatches for the VEGFA site 2 sgRNA and 1 to 4 mismatches for the VEGFA site 3 sgRNA **(****Fig. 4c****);** also, nine of these offtargets for VEGFA site 2 may have a potential bulged base (Lin, Y. et al,. Nucleic Acids Res 42, 7473-7485 (2014).at the sgRNA-DNA interface **(****Fig. 4a** and **Fig. 8****).** The sites that were not detected with SpCas9-HF1 possessed 2 to 6 mismatches for the VEGFA site 2 sgRNA and 2 mismatches in the single site for the VEGFA site 3 sgRNA **(****Fig. 4c****),** with three off-target sites for VEGFA site 2 sgRNA again having a potential bulge **(****Fig. 8****).** Collectively, these results demonstrated that SpCas9-HF1 can be highly effective at reducing off-target effects of sgRNAs targeted to simple repeat sequences and can also have substantial impacts on sgRNAs targeted to homopolymeric sequences.

**Table 2| Summary of potential mismatched sites in the reference human genome for the ten sgRNAs examined by GUIDE-seq**

| | | **mismatches to on-target site*** | | | | | | |
|---|---|---|---|---|---|---|---|---|
| **site** | **spacer with PAM** | **1** | **2** | **3** | **4** | **5** | **6** | **total** |
| EMX1-1 | GAGTCCGAGCAGAAGAAGAAGGG (SEQ ID NO:163) | 0 | 1 | 18 | 273 | 2318 | 15831 | 18441 |
| EMX1-2 | GTCACCTCCAATGACTAGGGTGG (SEQ ID NO:164) | 0 | 0 | 3 | 68 | 780 | 6102 | 6953 |
| FANCF-1 | GGAATCCCTTCTGCAGCACCTGG (SEQ ID NO:165) | 0 | 1 | 18 | 288 | 1475 | 9611 | 11393 |
| FANCF-2 | GCTGCAGAAGGGATTCCATGAGG (SEQ ID NO:166) | 1 | 1 | 29 | 235 | 2000 | 13047 | 15313 |
| FANCF-3 | GGCGGCTGCACAACCAGTGGAGG (SEQ ID NO:167) | 0 | 0 | 11 | 79 | 874 | 6651 | 7615 |
| FANCF-4 | GCTCCAGAGCCGTGCGAATGGGG (SEQ ID NO:168) | 0 | 0 | 6 | 59 | 639 | 5078 | 5782 |
| RUNX1-1 | GCATTTTCAGGAGGAAGCGATGG(SEQ ID NO:169) | 0 | 2 | 6 | 189 | 1644 | 11546 | 13387 |
| ZSCAN2 | GTGCGGCAAGAGCTTCAGCCGGG(SEQ ID NO:170) | 0 | 3 | 12 | 127 | 1146 | 10687 | 11975 |
| VEGFA2 | GACCCCCTCCACCCCGCCTCCGG(SEQ ID NO:171) | 0 | 2 | 35 | 456 | 3905 | 17576 | 21974 |
| VEGFA3 | GGTGAGTGAGTGTGTGCGTGTGG (SEQ ID NO:172) | 1 | 17 | 383 | 6089 | 13536 | 35901 | 55927 |

| | | | | | | | | |
|---|---|---|---|---|---|---|---|---|
| * determined using Cas-OFFinder (Bae et al., Bioinformatics 30, 1473-1475 (2014)) | | | | | | | | |

**Table 4: Oligonucleotides used in the study**

| **description of T7E1 primers** | **sequence** | **SEQ ID NO:** |
|---|---|---|
| forward primer to amplify EMX1 in U2OS human cells | | 173. |
| reverse primer to amplify EMX1 in U2OS human cells | | 174. |
| forward primer to amplify FANCF in U2OS human cells | | 175. |
| reverse primer to amplify FANCF in U2OS human cells | | 176. |
| forward primer to amplify RUNX1 in U2OS human cells | | 177. |
| reverse primer to amplify RUNX1 in U2OS human cells | | 178. |
| forward primer to amplify VEGFA in U2OS human cells | | 179. |
| reverse primer to amplify VEGFA in U2OS human cells | | 180. |
| forward primer to amplify VEGFA (NGG site 2) in U2OS human cells | | 181. |
| reverse primer to amplify VEGFA (NGG site 2) in U2OS human cells | | 182. |
| forward primer to amplify ZSCAN2 in U2OS human cells | | 183. |
| reverse primer to amplify ZSCAN2 in U2OS human cells | | 184. |
| forward primer to amplify ZNF629 in U2OS human cells | | 185. |
| reverse primer to amplify ZNF629 in U2OS human cells | | 186. |

| **description of deep sequencing primers** | **sequence** | **SEQ ID NO:** |
|---|---|---|
| forward primer to amplify EMX1-1 on-target | | 187. |
| reverse primer to amplify EMX1-1 on-target | | 188. |
| forward primer to amplify EMX1-1-GUIDE seq-OT#1 | | 189. |
| _ reverse primer to amplify EMX1-1-GUIDE _seq-OT#1 | | 190. |
| forward primer to amplify EMX1-1-GUIDE _seq-OT#2 | | 191. |
| reverse primer to amplify EMX1-1-GUIDE _seq-OT#2 | | 192. |
| forward primer to amplify EMX1-1-GUIDE _seq-OT#3 | | 193. |
| reverse primer to amplify EMX1-1-GUIDE _seq-OT#3 | | 194. |
| forward primer to amplify EMX1-1-GUIDE seq-OT#4 | | 195. |
| _ reverse primer to amplify EMX1-1-GUIDE _seq-OT#4 | | 196. |
| forward primer to amplify EMX1-1-GUIDE _seq-OT#5 | | 197. |
| reverse primer to amplify EMX1-1-GUIDE seq-OT#5 | | 198. |
| _ forward primer to amplify EMX1-1-GUIDE _seq-OT#6 | | 199. |
| reverse primer to amplify EMX1-1-GUIDE _seq-OT#6 | | 200. |
| forward primer to amplify EMX1-1-GUIDE _seq-OT#7 | | 201. |
| reverse primer to amplify EMX1-1-GUIDE _seq-OT#7 | | 202. |
| forward primer to amplify EMX1-1-GUIDE _seq-OT#8 | | 203. |
| reverse primer to amplify EMX1-1-GUIDE _seq-OT#8 | | 204. |
| forward primer to amplify EMX1-2 on-target | | 205. |
| reverse primer to amplify EMX1-2 on-target | | 206. |
| forward primer to amplify EMX1-2-GUIDE _seq-OT#1 | | 207. |
| reverse primer to amplify EMX1-2-GUIDE _seq-OT#1 | | 208 |
| forward primer to amplify EMX1-2-GUIDE _seq-OT#2 | | 209 |
| reverse primer to amplify EMX1-2-GUIDE _seq-OT#2 | | 210 |
| forward primer to amplify EMX1-2-GUIDE _seq-OT#3 | | 211 |
| reverse primer to amplify EMX1-2-GUIDE _seq-OT#3 | | 212 |
| forward primer to amplify EMX1-2-GUIDE _seq-OT#4 | | 213 |
| reverse primer to amplify EMX1-2-GUIDE _seq-OT#4 | | 214 |
| forward primer to amplify EMX1-2-GUIDE _seq-OT#5 | | 215 |
| reverse primer to amplify EMX1-2-GUIDE _seq-OT#5 | | 216 |
| forward primer to amplify EMX1-2-GUIDE _seq-OT#6 | | 217 |
| reverse primer to amplify EMX1-2-GUIDE _seq-OT#6 | | 218 |
| forward primer to amplify EMX1-2-GUIDE _seq-OT#7 | | 219 |
| reverse primer to amplify EMX1-2-GUIDE _seq-OT#7 | | 220 |
| forward primer to amplify EMX1-2-GUIDE _seq-OT#9 | | 221 |
| reverse primer to amplify EMX1-2-GUIDE _seq-OT#9 | | 222 |
| forward primer to amplify FANCF-1 on-target | | 223 |
| reverse primer to amplify FANCF-1 on-target | | 224 |
| forward primer to amplify FANCF-1-GUIDE _seq-OT#1 | | 225 |
| reverse primer to amplify FANCF-1-GUIDE _seq-OT#1 | | 226 |
| forward primer to amplify FANCF-1-GUIDE _seq-OT#2 | | 227 |
| reverse primer to amplify FANCF-1-GUIDE _seq-OT#2 | | 228 |
| forward primer to amplify FANCF-1-GUIDE _seq-OT#3 | | 229 |
| reverse primer to amplify FANCF-1-GUIDE _seq-OT#3 | | 230 |
| forward primer to amplify FANCF-1-GUIDE _seq-OT#4 | | 231 |
| reverse primer to amplify FANCF-1-GUIDE _seq-OT#4 | | 232 |
| forward primer to amplify FANCF-3 on-target | | 233 |
| reverse primer to amplify FANCF-3 on-target | | 234 |
| forward primer to amplify FANCF-3-GUIDE _seq-OT#1 | | 235 |
| reverse primer to amplify FANCF-3-GUIDE _seq-OT#1 | | 236 |
| forward primer to amplify FANCF-3-GUIDE _seq-OT#2 | | 237 |
| reverse primer to amplify FANCF-3-GUIDE _seq-OT#2 | | 238 |
| forward primer to amplify FANCF-3-GUIDE _seq-OT#3 | | 239 |
| reverse primer to amplify FANCF-3-GUIDE _seq-OT#3 | | 240 |
| forward primer to amplify FANCF-3-GUIDE _seq-OT#4 | | 241 |
| reverse primer to amplify FANCF-3-GUIDE _seq-OT#4 | | 242 |
| forward primer to amplify FANCF-3-GUIDE _seq-OT#5 | | 243 |
| reverse primer to amplify FANCF-3-GUIDE _seq-OT#5 | | 244 |
| forward primer to amplify FANCF-3-GUIDE _seq-OT#6 | | 245 |
| reverse primer to amplify FANCF-3-GUIDE _seq-OT#6 | | 246 |
| forward primer to amplify FANCF-3-GUIDE _seq-OT#7 | | 247 |
| reverse primer to amplify FANCF-3-GUIDE _seq-OT#7 | | 248 |
| forward primer to amplify RUNX1-1 on-target | | 249 |
| reverse primer to amplify RUNX1-1 on-target | | 250 |
| forward primer to amplify RUNX1-1-GUIDE _seq-OT#1 | | 251 |
| reverse primer to amplify RUNX1-1-GUIDE _seq-OT#1 | | 252 |
| forward primer to amplify RUNX1-1-GUIDE _seq-OT#2 | | 253 |
| reverse primer to amplify RUNX1-1-GUIDE _seq-OT#2 | | 254 |
| forward primer to amplify ZSCAN2 on-target | | 255 |
| reverse primer to amplify ZSCAN2 on-target | | 256 |
| forward primer to amplify ZSCAN2-GUIDE _seq-OT#1 | | 257 |
| reverse primer to amplify ZSCAN2-GUIDE _seq-OT#1 | | 258 |
| forward primer to amplify ZSCAN2-GUIDE _seq-OT#2 | | 259 |
| reverse primer to amplify ZSCAN2-GUIDE _seq-OT#2 | | 260 |
| forward primer to amplify ZSCAN2-GUIDE _seq-OT#3 | | 261 |
| reverse primer to amplify ZSCAN2-GUIDE _seq-OT#3 | | 262 |
| forward primer to amplify ZSCAN2-GUIDE _seq-OT#4 | | 263 |
| reverse primer to amplify ZSCAN2-GUIDE _seq-OT#4 | | 264 |
| forward primer to amplify ZSCAN2-GUIDE _seq-OT#5 | | 265 |
| reverse primer to amplify ZSCAN2-GUIDE _seq-OT#5 | | 266 |
| forward primer to amplify ZSCAN2-GUIDE _seq-OT#6 | | 267 |
| reverse primer to amplify ZSCAN2-GUIDE _seq-OT#6 | | 268 |
| forward primer to amplify ZSCAN2-GUIDE _seq-OT#7 | | 269 |
| reverse primer to amplify ZSCAN2-GUIDE _seq-OT#7 | | 270 |

### Refining the specificity of SpCas9-HF1

Previously described methods such as truncated gRNAs (Fu, Y. et al., Nat Biotechnol 32, 279-284 (2014)) and the SpCas9-D1 135E variant (Kleinstiver, B.P. et al., Nature 523, 481-485 (2015)) can partially reduce SpCas9 off-target effects, and the present inventors wondered whether these might be combined with SpCas9-HF1 to further improve its genome-wide specificity. Testing of SpCas9-HF1 with matched full-length and truncated sgRNAs targeted to four sites in the human cell-based EGFP disruption assay revealed that shortening sgRNA complementarity length substantially impaired on-target activities **(****Fig. 9****).** By contrast, SpCas9-HF1 with an additional D1135E mutation (a variant referred to herein as SpCas9-HF2) retained 70% or more activity of wild-type SpCas9 with six of eight sgRNAs tested using a human cell-based EGFP disruption assay **(****Figs. 5a** and **5b****).** SpCas9-HF3 and SpCas9-HF4 variants were also created harboring L169A or Y450A mutations, respectively, at positions whose side chains mediated hydrophobic non-specific interactions with the target DNA on its PAM proximal end (Nishimasu, H. et al., Cell 156, 935-949 (2014); Jiang, F., et al., Science 348, 1477-1481 (2015)). SpCas9-HF3 and SpCas9-HF4 retained 70% or more of the activities observed with wild-type SpCas9 with the same six out of eight EGFP-targeted sgRNAs (**Figs. 5a** and **5b**).

To determine whether SpCas9-HF2, -HF3, and -HF4 could reduce indel frequencies at two off-target sites (for the FANCF site 2 and VEGFA site 3 sgRNAs) that were resistant to SpCas9-HF1, further experiments were performed. For the FANCF site 2 off-target, which bears a single mismatch in the seed sequence of the protospacer, SpCas9-HF4 reduced indel mutation frequencies to near background level as judged by T7EI assay while also beneficially increasing on-target activity (**Fig. 5c**), resulting in the greatest increase in specificity among the three variants (**Fig. 5d**). For the VEGFA site 3 off-target site, which bears two protospacer mismatches (one in the seed sequence and one at the nucleotide most distal from the PAM sequence), SpCas9-HF2 showed the greatest reduction in indel formation while showing only modest effects on on-target mutation efficiency (**Fig. 5c**), leading to the greatest increase in specificity among the three variants tested (**Fig. 5d**). Taken together, these results demonstrate the potential for reducing off-target effects that are resistant to SpCas9-HF1 by introducing additional mutations at other residues that mediate non-specific DNA contacts or that may alter PAM recognition.

To generalize the T7E1 assay findings described above that show SpCas9-HF4 and SpCas9-HF2 have improved discrimination relative to SpCas9-HF1 against off-targets of the FANCF site 2 and VEGFA site 3 sgRNAs, respectively, the genome-wide specificities of these variants were examined using GUIDE-seq. Using an RFLP assay, it was determined that SpCas9-HF4 and SpCas9-HF2 had similar on-target activities to SpCas9-HF1, as assayed by GUIDE-seq tag integration rates (FIG. 5E). When analyzing the GUIDE-seq data, no new off-target sites were identified for SpCas9-HF2 or SpCas9-HF4 (FIG. 5F). Compared to SpCas9-HF1, off-target activities at all sites were either rendered undetectable by GUIDE-seq or substantially decreased. Relative to SpCas9-HF1, SpCas9-HF4 had nearly 26-fold better specificity against the single FANCF site 2 off-target site that remained recalcitrant to the specificity improvements of SpCas9-HF1 (FIG. 5F). SpCas9-HF2 had nearly 4-fold improved specificity relative to SpCas9-HF1 for the high-frequency VEGFA site 3 off-target, while also dramatically reducing (>38-fold) or eliminating GUIDE-seq detectable events at other low-frequency off-target sites. Of note, the genomic position of 3 of these low frequency sites identified for SpCas9-HF1 are adjacent to previously characterized background U2OS cell breakpoint hotspots. Collectively, these results suggest that the SpCas9-HF2 and SpCas9-HF4 variants can improve the genome-wide specificity of SpCas9-HF1.

SpCas9-HF1 robustly and consistently reduced off-target mutations when using sgRNAs designed against standard, non-repetitive target sequences. The two off-target sites that were most resistant to SpCas9-HF 1 have only one and two mismatches in the protospacer. Together, these observations suggest that off-target mutations might be minimized to undetectable levels by using SpCas9-HF1 and targeting non-repetitive sequences that do not have closely related sites bearing one or two mismatches elsewhere in the genome (something that can be easily accomplished using existing publicly available software programs (Bae, S., et al, Bioinformatics 30, 1473-1475 (2014)). One parameter that users should keep in mind is that SpCas9-HF1 may not be compatible with the common practice of using a G at the 5' end of the gRNA that is mismatched to the protospacer sequence. Testing of four sgRNAs bearing a 5' G mismatched to its target site showed three of the four had diminished activities with SpCas9-HF1 compared to wild-type SpCas9 (**Fig. 10**), perhaps reflecting the ability of SpCas9-HF1 to better discriminate a partially matched site.

Further biochemical work can confirm or clarify the precise mechanism by which SpCas9-HF1 achieves its high genome-wide specificity. It does not appear that the four mutations introduced alter the stability or steady-state expression level of SpCas9 in the cell, because titration experiments with decreasing concentrations of expression plasmids suggested that wild-type SpCas9 and SpCas9-HF1 behaved comparably as their concentrations are lowered (**Fig. 11**). Instead, the simplest mechanistic explanation is that these mutations decreased the energetics of interaction between the Cas9-sgRNA and the target DNA, with the energy of the complex at a level just sufficient to retain on-target activity but lowered it enough to make off-target site cleavage inefficient or non-existent. This mechanism is consistent with the non-specific interactions observed between the residues mutated and the target DNA phosphate backbone in structural data (Nishimasu, H. et al., Cell 156, 935-949 (2014); Anders, C et. Al., Nature 513, 569-573 (2014)). A somewhat similar mechanism has been proposed to explain the increased specificities of transcription activator-like effector nucleases bearing substitutions at positively charged residues (Guilinger, J.P. et al., Nat Methods 11, 429-435 (2014)).

It was possible that SpCas9-HF1 might also be combined with other mutations that have been shown to alter Cas9 function. For example, an SpCas9 mutant bearing three amino acid substitutions (D1135V/R1335Q/T1337R, also known as the SpCas9-VQR variant), recognizes sites with NGAN PAMs (with relative efficiencies for NGAG>NGAT=NGAA>NGAC) (Kleinstiver, B.P. et al, Nature 523, 481-485 (2015)) and a recently identified quadruple SpCas9 mutant (D1135V/G1218R/R1335Q/T1337R, referred to as the SpCas9-VRQR variant) has improved activities relative to the VQR variant on sites with NGAH (H = A, C, or T) PAMs (**Fig. 12a**). Introduction of the four mutations (N497A/R661A/Q695A/Q926A) from SpCas9-HF1 into SpCas9-VQR and SpCas9-VRQR created SpCas9-VQR-HF1 and SpCas9-VRQR-HF1, respectively. Both HF versions of these nucleases showed on-target activities comparable (i.e., 70% or more) to their non-HF counterparts with five of eight sgRNAs targeted to the EGFP reporter gene and with seven of eight sgRNAs targeted to endogenous human gene sites (**Figs. 12b-12d**).

More broadly, these results illuminate a general strategy for the engineering of additional high-fidelity variants of CRISPR-associated nucleases. Adding additional mutations at non-specific DNA contacting residues further reduced some of the very small number of residual off-target sites that persist with SpCas9-HF1. Thus, variants such as SpCas9-HF2, SpCas9-HF3, SpCas9-HF4, and others can be utilized in a customized fashion depending on the nature of the off-target sequences. Furthermore, success with engineering high-fidelity variants of SpCas9 suggests that the approach of mutating non-specific DNA contacts can be extended to other naturally occurring and engineered Cas9 orthologues (Ran, F.A. et al., Nature 520, 186-191 (2015), Esvelt, K.M. et al., Nat Methods 10, 1116-1121 (2013); Hou, Z. et al., Proc Natl Acad Sci U S A (2013); Fonfara, I. et al., Nucleic Acids Res 42, 2577-2590 (2014); Kleinstiver, B.P. et al, Nat Biotechnol (2015) as well as newer CRISPR-associated nucleases (Zetsche, B. et al., Cell 163, 759-771 (2015); Shmakov, S. et al., Molecular Cell 60, 385-397) that are being discovered and characterized with increasing frequency.

### Example 2

Described herein are SpCas9 variants with alanine substitutions in residues that contact the target strand DNA, including N497A, Q695A, R661A, and Q926A. Beyond these residues, the present inventors sought to determine whether the specificity of these variants, e.g., the SpCas9-HF1 variant (N497A/R661A/Q695A/Q926A), might be further improved by adding substitutions in positively-charged SpCas9 residues that appear to make contacts with the non-target DNA strand: R780, K810, R832, K848, K855, K968, R976, H982, K1003, K1014, K1047, and/or R1060 (see Slaymaker et al., Science. 2016 Jan 1;351(6268):84-8).

The activities of wild-type SpCas9 derivatives bearing single alanine substitutions at these positions and combinations thereof were initially tested using the EGFP disruption assay with a perfectly matched sgRNA designed to a site in the *EGFP* gene (to assess on-target activities) and the same sgRNA bearing intentional mismatches at positions 11 and 12 with position 1 being the most PAM-proximal base (to assess activities at mismatched sites, as would be found at off-target sites) (**Figure 13A**). (Note that the derivatives bearing the triple substitutions K810A/K1003A/R1060A or K848A/K1003A/R1060A are the same as recently described variants known as eSpCas9(1.0) and eSpCas9(1.1), respectively; see ref. 1). As expected, wild-type SpCas9 had robust on-target and mismatched-target activities. As a control, we also tested SpCas9-HF1 in this experiment and found that it maintained on-target activity while reducing mismatched-target activity as expected **(****Figure 13A****).** All of the wild-type SpCas9 derivatives bearing one or more alanine substitutions at positions that might potentially contact the non-target DNA strand showed on-target activities comparable to wild-type SpCas9 **(****Figure 13A****).** Interestingly, some of these derivatives also showed reduced cleavage with the mismatched 11/12 sgRNA relative to the activity observed with wild-type SpCas9, suggesting that a subset of the substitutions in these derivatives confer enhanced specificity against this mismatched site relative to wild-type SpCas9 **(****Figure 13A****).** However, none of these single substitutions or combinations of substitutions were sufficient to completely eliminate activities observed the 11/12 mismatched sgRNA. When we tested wild-type SpCas9, SpCas9-HF1, and these same wild-type SpCas9 derivatives using an additional sgRNA bearing mismatches at positions 9 and 10

**(****Figure 13B****),** only minimal changes in mismatched-target activities were observed for most derivatives. Again, this demonstrated that single, double, or even triple substitutions (equivalent to the previously described eSpCas9(1.0) and (1.1) variants) at these potential non-target strand contacting residues are insufficient to eliminate activities at imperfectly matched DNA sites. Collectively, these data demonstrate that the wild-type SpCas9 variants retain on-target activity with a matched sgRNA and that the substitutions contained in these derivatives on their own (in the context of wild-type SpCas9) are not sufficient to eliminate nuclease activities on two different mismatched DNA sites (**Figures 13A** and **13B**).

Given these results, it was hypothesized that SpCas9-HF1 derivatives bearing one or more additional amino acid substitutions at residues that might contact the non-target DNA strand might further improve specificity relative to the parental SpCas9-HF1 protein. Therefore, various SpCas9-HF1-derviatives bearing combinations of single, double, or triple alanine substitutions were tested in the human cell-based EGFP disruption assay using a perfectly matched sgRNA (to test on-target activities) and the same sgRNA bearing mismatches at positions 11 and 12 (to assess activities at a mismatched target site, as would be found for off-target sites). These sgRNAs are the same ones that were used for **Figures 13A-B****.** This experiment revealed most of the SpCas9-HF1-derivative variants we tested showed comparable on-target activities to those observed with both wild-type SpCas9 and SpCas9-HF1 **(****Figure 14A****).** With the 11/12 mismatched sgRNA, some of the SpCas9-HF1 derivatives tested (such as SpCas9-HF1 + R832A and SpCas9-HF1 + K1014A) did not show an appreciable change in cleavage with the mismatched sgRNA. However, importantly, most of the SpCas9-HF1 derivatives had substantially lower activity with the 11/12 mismatched sgRNA than what was observed with SpCas9-HF1, eSpCas9(1.0), or eSpCas9(1.1), suggesting that certain combinations of these new variants have reduced mismatched-target activities and thus improved specificities **(****Figure 14A****).** Of the 16 SpCas9-HF1 derivatives that reduced mismatched-target activities with the 11/12 mismatched sgRNA to near background levels, 9 appeared to have only minimal effects on on-target activity (assessed using the perfectly matched sgRNA; **Figure 14A**). Additional testing of a subset of these SpCas9-HF1 derivatives in the EGFP disruption assay using an sgRNA intentionally mismatched at positions 9 and 10 (**Figure 14B**) also revealed that these variants possessed lower activities with this mismatched sgRNA than what was observed either with SpCas9-HF1 **(****Figure 14b****),** with eSpCas9(1.1) **(****Figure 13A****),** or with the same substitutions added to wild-type SpCas9 nuclease **(****Figure 13B****).** Importantly, five variants showed background level off-target activity in this assay with the 9/10 mismatched sgRNA.

Next, whether these alanine substitutions of the non-target strand could be combined with the SpCas9 variant that contains only the Q695A and Q926A substitutions from our SpCas9-HF1 variant (here "double" variant) was tested. Because many of the HF1 derivatives tested above showed an observable (and undesirable) decrease in on-target activity, it was hypothesized that combining only the two most important substitutions from SpCas9-HF1 (Q695A and Q926A; see **Figure 1B****)** with one or more non-target strand contacting substitutions might rescue on-target activity but still maintain the gains in specificity observed when these substitutions were added to the SpCas9-HF1 variant. Therefore, various SpCas9(Q695A/Q926A) derivatives bearing combinations of single, double, or triple alanine substitutions at potential non-target DNA strand interacting positions were tested in the human cell-based EGFP disruption assay using the same perfectly matched sgRNA targeted to EGFP described above (to test on-target activities) and the same sgRNA bearing mismatches at positions 11 and 12 (to assess activities at a mismatched target site, as would be found for off-target sites) that were used for **Figures 13A-B****.** This experiment revealed most of the SpCas9(Q695A/Q926A) derivative variants tested showed comparable on-target activities to those observed with both wild-type SpCas9 and SpCas9-HF1 **(****Figure 15****).** Importantly, many of the SpCas9-HF1 derivatives had substantially lower activity with the 11/12 mismatched sgRNA compared with what was observed with SpCas9-HF1, eSpCas9(1.0), or eSpCas9(1.1) suggesting that certain combinations of these new variants have reduced mismatched-target activities and thus improved specificities **(****Figure 15****).** Of the 13 SpCas9(Q695A/Q926A) derivatives that reduced mismatched-target activities with the 11/12 mismatched sgRNA to near background levels, only 1 appeared to have a substantial effect on on-target activity (assessed using the perfectly matched sgRNA; **Figure 15****).**

Overall, these data demonstrate that the addition of one, two, or three alanine substitutions to SpCas9-HF1 or SpCas9(Q695A/Q926A) at positions that might contact the non-target DNA strand can lead to new variants with improved abilities to discriminate against mismatched off-target sites (relative to to their parental clones or the recently described eSpCas9(1.0) or (1.1). Importantly, these same substitutions in the context of wild-type SpCas9 do not appear to provide any substantial specificity benefit.

To better define and compare the tolerances of SpCas9-HF1 and eSpCas9-1.1 to mismatches at the sgRNA-target DNA complementarity interface, their activities were examined using sgRNAs containing single mismatches at all possible positions in the spacer complementarity region. Both the SpCas9-HF1 and eSPCas9-1.1 variants had similar activities on most singly mismatched sgRNAs when compared to wild-type SpCas9, with a few exceptions where SpCas9-HF1 outperformed eSpCas9-1.1 **(****Figure 16****).**

Next we tested the single nucleotide mismatch tolerance of some variants containing combinations of amino acid substitutions from either the double mutant (Db = Q695A/Q926A), SpCas9-HF1 (N497A/R661A/Q695A/Q926A), eSpCas9-1.0 (1.0 = K810A/K1003A/R1060A), or eSpCas9-1.1(1.1 = K848A/K1003A/R1060A) with additional alanine substitutions in residues that contact the target strand DNA or that potentially contact the non-target strand DNA **(****Figures 17A-B****).** On-target activity was assessed using a perfectly matched sgRNA, while single nucleotide mismatch tolerance was assessed using sgRNAs bearing such mismatches at positions 4, 8, 12, or 16 in the spacer sequence **(****Figure 17A****).** A number of these variants maintained on-target activity with substantial reductions in activities observed with the mismatched sgRNAs. Three of these variants
(Q695A/K848A/Q926A/K1003A/R1060A,
N497A/R661A/Q695A/K855A/Q926A/R1060A, and
N497A/R661A/Q695A/Q926A/H982A/R1060A) were further tested with the remaining single mismatch sgRNAs (containing mismatches at positions 1-3, 5-7, 9-11, 13-15, and 17-20). These variants demonstrated a more robust intolerance to single nucleotide substitutions in the sgRNA compared with eSpCas9-1.1, demonstrating the improved specificity profile of these new variants **(****Figure 17B****).** Additional variant nucleases containing alternative combinations of amino acid substitutions were tested using sgRNAs containing mismatches at positions 5, 7, and 9 in the spacer (these particular mismatched sgRNAs were used because earlier variants appeared to tolerate mismatches at these positions) **(****Figure 18****).** A number of these nucleases had improved specificities against the mismatched sites, with only marginal reductions in on-target activities **(****Figure 18****).**

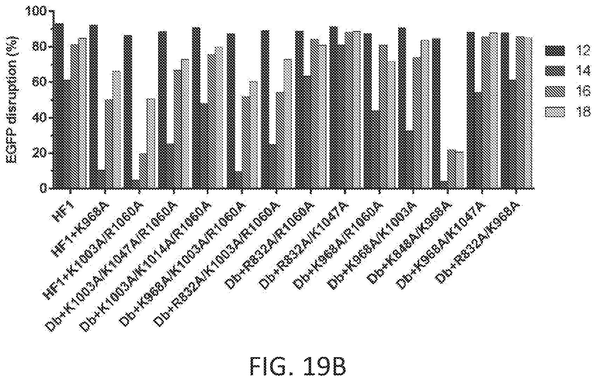

To further determine whether additional combinations of mutations could convey specificity improvements, a greatly expanded panel of nuclease variants with two additional matched sgRNAs was tested to examine on-target activity in our EGFP disruption activity **(****Figure 19A****).** A number of these variants maintained robust on-target activities, suggesting that they may be useful for generating further mprovements to specificity **(****Figure 19B****).** A number of these variants were tested with sgRNAs containing single substitutions at positions 12, 14, 16, or 18 to determine whether specificity improvements were observed and were found to exhibit greater intolerance to single nucleotide mismatches at these positions **(****Figure 19B****).**

### Example 3

Taking an analogous strategy with *Staphylococcus aureus* Cas9 (SaCas9) as we had done with SpCas9, experiments were performed to improve the specificity of SaCas9 by introducing alanine substitutions in residues that are known to contact the target DNA strand **(****Figure 20** and **Figure 21A****),** residues that may contact the non-target DNA (ongoing experiments), and residues that we have previously shown can influence PAM specificity **(****Figure 21B****).** Residues that may contact the target strand DNA backbone include: Y211, Y212, W229, Y230, R245, T392, N419, L446, Y651, and R654; residues that may contact the non-target strand DNA include: Q848, N492, Q495, R497, N498, R499, Q500, K518, K523, K525, H557, R561, K572, R634, R654, G655, N658, S662, N668, R686, K692, R694, H700, K751; and residues that contact the PAM include: E782, D786, T787, Y789, T882, K886, N888, A889, L909, K929, N985, N986, R991, and R1015. In a preliminary experiment, single alanine substitutions (or some combinations thereof) in either target strand DNA contacting residues or PAM contacting residues **(****Figures 21A and B****,** respectively) had variable effects on on-target EGFP disruption activity (using a perfectly matched sgRNA) and were unable to eliminate off-target cleavage (when using an sgRNA mismatched at positions 11 and 12). Interestingly, SpCas9 mutations in the HF1 were unable to completely abolish off-target activity with a similarly mismatched target/sgRNA pair, suggesting that variants containing combinations of target strand/non-target strand substitutions may be necessary to improve specificity at such sites (as we observed with SpCas9).

To further assess the strategy of mutating potential target strand DNA contacts to improve SaCas9 specificity, the potential of single, double, triple, and quadruple combinations of mutations to tolerate mismatches at positions 19 and 20 in an sgRNA was examined **(****Figures 22A** **and** **B****).** These combinations revealed that alanine substitutions at Y230 and R245, when combined with other substitutions, can increase specificity as judged by the capability to better discriminate against mismatched sites.

Next the on-target gene disruption activities of two of these triple alanine substitution variants (Y211A/Y230A/R245A and Y212A/Y230A/R245A) were examined at 4 on-target sites in EGFP (matched sites #1-4; **Figure 23****).** These variants maintained robust on-target activities for matched sites 1 and 2 but showed approximately 60-70% loss of on-target activity with matched sites 3 and 4. Both of these triple alanine substitution variants dramatically improved specificity relative to wild-type SaCas9 as judged by using sgRNAs bearing double mismatches at various positions in the spacers of target sites 1-4 **(****Figure 23****).**

SaCas9 variants bearing double and triple combinations **(****Figures 24A and B****, respectively)** of these alanine substitutions were tested on six endogenous sites for on target activities and improvements in specificity assessed using an sgRNA containing a single mismatch at position 21 (the most PAM distal position expected to be a challenging mismatch to discriminate against). In some cases, on-target activities with the matched sgRNA were maintained with the variants while 'off-target' activities with the sgRNA mismatched at position 21 were eliminated **(****Figures 24A and B****).** In other cases, marginal to complete loss of activity was observed with the matched sgRNA.

### References

1. Sander, J.D. & Joung, J.K. CRISPR-Cas systems for editing, regulating and targeting genomes. Nat Biotechnol 32, 347-355 (2014).
2. Hsu, P.D., Lander, E.S. & Zhang, F. Development and applications of CRISPR-Cas9 for genome engineering. Cell 157, 1262-1278 (2014).
3. Doudna, J.A. & Charpentier, E. Genome editing. The new frontier of genome engineering with CRISPR-Cas9. Science 346, 1258096 (2014).
4. Barrangou, R. & May, A.P. Unraveling the potential of CRISPR-Cas9 for gene therapy. Expert Opin Biol Ther 15, 311-314 (2015).
5. Jinek, M. et al. A programmable dual-RNA-guided DNA endonuclease in adaptive bacterial immunity. Science 337, 816-821 (2012).
6. Sternberg, S.H., Redding, S., Jinek, M., Greene, E.C. & Doudna, J.A. DNA interrogation by the CRISPR RNA-guided endonuclease Cas9. Nature 507, 62-67 (2014).
7. Hsu, P.D. et al. DNA targeting specificity of RNA-guided Cas9 nucleases. Nat Biotechnol 31, 827-832 (2013).
8. Tsai, S.Q. et al. GUIDE-seq enables genome-wide profiling of off-target cleavage by CRISPR-Cas nucleases. Nat Biotechnol 33, 187-197 (2015).
9. Hou, Z. et al. Efficient genome engineering in human pluripotent stem cells using Cas9 from Neisseria meningitidis. Proc Natl Acad Sci U S A (2013).
10. Fonfara, I. et al. Phylogeny of Cas9 determines functional exchangeability of dual-RNA and Cas9 among orthologous type II CRISPR-Cas systems. Nucleic Acids Res 42, 2577-2590 (2014).
11. Esvelt, K.M. et al. Orthogonal Cas9 proteins for RNA-guided gene regulation and editing. Nat Methods 10, 1116-1121 (2013).
12. Cong, L. et al. Multiplex genome engineering using CRISPR/Cas systems. Science 339, 819-823 (2013).
13. Horvath, P. et al. Diversity, activity, and evolution of CRISPR loci in Streptococcus thermophilus. JBacteriol 190, 1401-1412 (2008).
14. Anders, C., Niewoehner, O., Duerst, A. & Jinek, M. Structural basis of PAM-dependent target DNA recognition by the Cas9 endonuclease. Nature 513, 569-573 (2014).
15. Reyon, D. et al. FLASH assembly of TALENs for high-throughput genome editing. Nat Biotechnol 30, 460-465 (2012).
16. Fu, Y. et al. High-frequency off-target mutagenesis induced by CRISPR-Cas nucleases in human cells. Nat Biotechnol 31, 822-826 (2013).
17. Chen, Z. & Zhao, H. A highly sensitive selection method for directed evolution of homing endonucleases. Nucleic Acids Res 33, e154 (2005).
18. Doyon, J.B., Pattanayak, V., Meyer, C.B. & Liu, D.R. Directed evolution and substrate specificity profile of homing endonuclease I-SceI. J Am Chem Soc 128, 2477-2484 (2006).
19. Jiang, W., Bikard, D., Cox, D., Zhang, F. & Marraffini, L.A. RNA-guided editing of bacterial genomes using CRISPR-Cas systems. Nat Biotechnol 31, 233-239 (2013).
20. Mali, P. et al. RNA-guided human genome engineering via Cas9. Science 339, 823-826 (2013).
21. Hwang, W.Y. et al. Efficient genome editing in zebrafish using a CRISPR-Cas system. Nat Biotechnol 31, 227-229 (2013).
22. Chylinski, K., Le Rhun, A. & Charpentier, E. The tracrRNA and Cas9 families of type II CRISPR-Cas immunity systems. RNA Biol 10, 726-737 (2013).
23. Kleinstiver, B.P., Fernandes, A.D., Gloor, G.B. & Edgell, D.R. A unified genetic, computational and experimental framework identifies functionally relevant residues of the homing endonuclease I-BmoI. Nucleic Acids Res 38, 2411-2427 (2010).
24. Gagnon, J.A. et al. Efficient mutagenesis by Cas9 protein-mediated oligonucleotide insertion and large-scale assessment of single-guide RNAs. PLoS One 9, e98186 (2014).

### Sequences

**SEQ ID NO:271** - **JDS246:** CMV-T7-humanSpCas9-NLS-3xFLAG Human codon optimized *S*. *pyogenes* Cas9 in normal font, NLS double underlined, 3xFLAG tag in **bold:**
SEQ **ID NO:272** - **VP12:** CMV-T7-humanSpCas9-HF1(N497A, R661A, Q695A, Q926A)-NLS-3xFLAG Human codon optimized *S*. *pyogenes* Cas9 in normal font, modified codons in lower case, NLS double underlined, 3xFLAG tag in **bold:**
**SEQ ID NO:273** - **MSP2135:** CMV-T7-humanSpCas9-HF2(N497A, R661A, Q695A, Q926A, D1135E)-NLS-3xFLAG Human codon optimized *S*. *pyogenes* Cas9 in normal font, modified codons in lower case, NLS double underlined, 3xFLAG tag in **bold:**
**SEQ ID NO:274** - **MSP2133:** CMV-T7-humanSpCas9-HF4(Y450A, N497A, R661A, Q695A, Q926A)-NLS-3xFLAG Human codon optimized *S*. *pyogenes* Cas9 in normal font, modified codons in lower case, NLS double underlined, 3xFLAG tag in **bold:**
**SEQ ID NO:275** - **MSP469:** CMV-T7-humanSpCas9-VQR(D1135V, R1335Q, T1337R)-NLS-3xFLAG Human codon optimized *S*. *pyogenes* Cas9 in normal font, modified codons in lower case, NLS double underlined, 3xFLAG tag in **bold:**
**SEQ ID NO:276** - **MSP2440:** CMV-T7-humanSpCas9-VQR-HF1(N497A, R661A, Q695A, Q926A, D1135V, R1335Q, T1337R)-NLS-3xFLAG Human codon optimized *S*. *pyogenes* Cas9 in normal font, modified codons in lower case, NLS double underlined, 3xFLAG tag in **bold:**
**SEQ ID NO:277** - **BPK2797:** CMV-T7-humanSpCas9-VRQR(D1135V, G1218R, R1335Q, T1337R)-NLS-3xFLAG Human codon optimized *S*. *pyogenes* Cas9 in normal font, modified codons in lower case, NLS double underlined, 3xFLAG tag in **bold:**
**SEQ ID NO:278** - **MSP2443:** CMV-T7-humanSpCas9-VRQR-HF1(N497A, R661A, Q695A, Q926A, D1135V, G1218R, R1335Q, T1337R)-NLS-3xFLAG Human codon optimized *S*. *pyogenes* Cas9 in normal font, modified codons in lower case, NLS double underlined, 3xFLAG tag in **bold:**
**SEQ ID NO:279** - **BPK1520:** U6-BsmBIcassette-Sp-sgRNA U6 promoter in normal font, BsmBI sites *italicised, S. pyogenes* sgRNA in lower case, U6 terminator double underlined:

### SEQUENCE LISTING

<110> THE GENERAL HOSPITAL CORPORATION
<120> ENGINEERED CRISPR-CAS9 NUCLEASES
<130> 29539-0189W01
<150> US 15/015,947
   <151> 2016-02-04
<150> US 62/258,280
   <151> 2015-11-20
<150> US 62/216,033
   <151> 2015-09-09
<150> US 62/211,553
   <151> 2015-08-28
<150> US 62/271,938
   <151> 2015-12-28
<160> 279
<170> PatentIn version 3.5
<210> 1
   <211> 1368
   <212> PRT
   <213> Streptococcus pyogenes
<400> 1
<210> 2
   <211> 1053
   <212> PRT
   <213> Staphylococcus aureus
<400> 2
   Met Lys Arg Asn Tyr Ile Leu Gly Leu Asp Ile Gly Ile Thr Ser Val
   1               5                   10                  15
   Gly Tyr Gly Ile Ile Asp Tyr Glu Thr Arg Asp Val Ile Asp Ala Gly
               20                  25                  30
   Val Arg Leu Phe Lys Glu Ala Asn Val Glu Asn Asn Glu Gly Arg Arg
           35                  40                  45
   Ser Lys Arg Gly Ala Arg Arg Leu Lys Arg Arg Arg Arg His Arg Ile
       50                  55                  60
   Gln Arg Val Lys Lys Leu Leu Phe Asp Tyr Asn Leu Leu Thr Asp His
   65                  70                  75                  80
   Ser Glu Leu Ser Gly Ile Asn Pro Tyr Glu Ala Arg Val Lys Gly Leu
                   85                  90                  95
   Ser Gln Lys Leu Ser Glu Glu Glu Phe Ser Ala Ala Leu Leu His Leu
               100                 105                 110
   Ala Lys Arg Arg Gly Val His Asn Val Asn Glu Val Glu Glu Asp Thr
           115                 120                 125
   Gly Asn Glu Leu Ser Thr Lys Glu Gln Ile Ser Arg Asn Ser Lys Ala
       130                 135                 140
   Leu Glu Glu Lys Tyr Val Ala Glu Leu Gln Leu Glu Arg Leu Lys Lys
   145                 150                 155                 160
   Asp Gly Glu Val Arg Gly Ser Ile Asn Arg Phe Lys Thr Ser Asp Tyr
                   165                 170                 175
   Val Lys Glu Ala Lys Gln Leu Leu Lys Val Gln Lys Ala Tyr His Gln
               180                 185                 190
   Leu Asp Gln Ser Phe Ile Asp Thr Tyr Ile Asp Leu Leu Glu Thr Arg
           195                 200                 205
   Arg Thr Tyr Tyr Glu Gly Pro Gly Glu Gly Ser Pro Phe Gly Trp Lys
       210                 215                 220
   Asp Ile Lys Glu Trp Tyr Glu Met Leu Met Gly His Cys Thr Tyr Phe
   225                 230                 235                 240
   Pro Glu Glu Leu Arg Ser Val Lys Tyr Ala Tyr Asn Ala Asp Leu Tyr
                   245                 250                 255
   Asn Ala Leu Asn Asp Leu Asn Asn Leu Val Ile Thr Arg Asp Glu Asn
               260                 265                 270
   Glu Lys Leu Glu Tyr Tyr Glu Lys Phe Gln Ile Ile Glu Asn Val Phe
           275                 280                 285
   Lys Gln Lys Lys Lys Pro Thr Leu Lys Gln Ile Ala Lys Glu Ile Leu
       290                 295                 300
   Val Asn Glu Glu Asp Ile Lys Gly Tyr Arg Val Thr Ser Thr Gly Lys
   305                 310                 315                 320
   Pro Glu Phe Thr Asn Leu Lys Val Tyr His Asp Ile Lys Asp Ile Thr
                   325                 330                 335
   Ala Arg Lys Glu Ile Ile Glu Asn Ala Glu Leu Leu Asp Gln Ile Ala
               340                 345                 350
   Lys Ile Leu Thr Ile Tyr Gln Ser Ser Glu Asp Ile Gln Glu Glu Leu
           355                 360                 365
   Thr Asn Leu Asn Ser Glu Leu Thr Gln Glu Glu Ile Glu Gln Ile Ser
       370                 375                 380
   Asn Leu Lys Gly Tyr Thr Gly Thr His Asn Leu Ser Leu Lys Ala Ile
   385                 390                 395                 400
   Asn Leu Ile Leu Asp Glu Leu Trp His Thr Asn Asp Asn Gln Ile Ala
                   405                 410                 415
   Ile Phe Asn Arg Leu Lys Leu Val Pro Lys Lys Val Asp Leu Ser Gln
               420                 425                 430
   Gln Lys Glu Ile Pro Thr Thr Leu Val Asp Asp Phe Ile Leu Ser Pro
           435                 440                 445
   Val Val Lys Arg Ser Phe Ile Gln Ser Ile Lys Val Ile Asn Ala Ile
       450                 455                 460
   Ile Lys Lys Tyr Gly Leu Pro Asn Asp Ile Ile Ile Glu Leu Ala Arg
   465 470 475 480
   Glu Lys Asn Ser Lys Asp Ala Gln Lys Met Ile Asn Glu Met Gln Lys
   485 490 495
   Arg Asn Arg Gln Thr Asn Glu Arg Ile Glu Glu Ile Ile Arg Thr Thr
   500 505 510
   Gly Lys Glu Asn Ala Lys Tyr Leu Ile Glu Lys Ile Lys Leu His Asp
   515 520 525
   Met Gln Glu Gly Lys Cys Leu Tyr Ser Leu Glu Ala Ile Pro Leu Glu
   530 535 540
   Asp Leu Leu Asn Asn Pro Phe Asn Tyr Glu Val Asp His Ile Ile Pro
   545 550 555 560
   Arg Ser Val Ser Phe Asp Asn Ser Phe Asn Asn Lys Val Leu Val Lys
   565 570 575
   Gln Glu Glu Asn Ser Lys Lys Gly Asn Arg Thr Pro Phe Gln Tyr Leu
   580 585 590
   Ser Ser Ser Asp Ser Lys Ile Ser Tyr Glu Thr Phe Lys Lys His Ile
   595 600 605
   Leu Asn Leu Ala Lys Gly Lys Gly Arg Ile Ser Lys Thr Lys Lys Glu
   610 615 620
   Tyr Leu Leu Glu Glu Arg Asp Ile Asn Arg Phe Ser Val Gln Lys Asp
   625 630 635 640
   Phe Ile Asn Arg Asn Leu Val Asp Thr Arg Tyr Ala Thr Arg Gly Leu
   645 650 655
   Met Asn Leu Leu Arg Ser Tyr Phe Arg Val Asn Asn Leu Asp Val Lys
   660 665 670
   Val Lys Ser Ile Asn Gly Gly Phe Thr Ser Phe Leu Arg Arg Lys Trp
   675 680 685
   Lys Phe Lys Lys Glu Arg Asn Lys Gly Tyr Lys His His Ala Glu Asp
   690 695 700
   Ala Leu Ile Ile Ala Asn Ala Asp Phe Ile Phe Lys Glu Trp Lys Lys
   705 710 715 720
   Leu Asp Lys Ala Lys Lys Val Met Glu Asn Gln Met Phe Glu Glu Lys
   725 730 735
   Gln Ala Glu Ser Met Pro Glu Ile Glu Thr Glu Gln Glu Tyr Lys Glu
   740 745 750
   Ile Phe Ile Thr Pro His Gln Ile Lys His Ile Lys Asp Phe Lys Asp
   755 760 765
   Tyr Lys Tyr Ser His Arg Val Asp Lys Lys Pro Asn Arg Glu Leu Ile
   770 775 780
   Asn Asp Thr Leu Tyr Ser Thr Arg Lys Asp Asp Lys Gly Asn Thr Leu
   785 790 795 800
   Ile Val Asn Asn Leu Asn Gly Leu Tyr Asp Lys Asp Asn Asp Lys Leu
   805 810 815
   Lys Lys Leu Ile Asn Lys Ser Pro Glu Lys Leu Leu Met Tyr His His
   820 825 830
   Asp Pro Gln Thr Tyr Gln Lys Leu Lys Leu Ile Met Glu Gln Tyr Gly
   835 840 845
   Asp Glu Lys Asn Pro Leu Tyr Lys Tyr Tyr Glu Glu Thr Gly Asn Tyr
   850 855 860
   Leu Thr Lys Tyr Ser Lys Lys Asp Asn Gly Pro Val Ile Lys Lys Ile
   865 870 875 880
   Lys Tyr Tyr Gly Asn Lys Leu Asn Ala His Leu Asp Ile Thr Asp Asp
   885 890 895
   Tyr Pro Asn Ser Arg Asn Lys Val Val Lys Leu Ser Leu Lys Pro Tyr
   900 905 910
   Arg Phe Asp Val Tyr Leu Asp Asn Gly Val Tyr Lys Phe Val Thr Val
   915 920 925
   Lys Asn Leu Asp Val Ile Lys Lys Glu Asn Tyr Tyr Glu Val Asn Ser
   930 935 940
   Lys Cys Tyr Glu Glu Ala Lys Lys Leu Lys Lys Ile Ser Asn Gln Ala
   945 950 955 960
   Glu Phe Ile Ala Ser Phe Tyr Asn Asn Asp Leu Ile Lys Ile Asn Gly
   965 970 975
   Glu Leu Tyr Arg Val Ile Gly Val Asn Asn Asp Leu Leu Asn Arg Ile
               980                 985                 990
   Glu Val Asn Met Ile Asp Ile Thr  Tyr Arg Glu Tyr Leu  Glu Asn Met
           995                 1000                 1005
   Asn Asp  Lys Arg Pro Pro Arg  Ile Ile Lys Thr Ile  Ala Ser Lys
       1010                 1015                 1020
   Thr Gln  Ser Ile Lys Lys Tyr  Ser Thr Asp Ile Leu  Gly Asn Leu
       1025                 1030                 1035
   Tyr Glu  Val Lys Ser Lys Lys  His Pro Gln Ile Ile  Lys Lys Gly
       1040                 1045                 1050
<210> 3
   <211> 4
   <212> PRT
   <213> Artificial Sequence
<220>
   <223> Description of Artificial Sequence: Synthetic peptide
<400> 3
   Gly Gly Gly Ser
   1
<210> 4
   <211> 5
   <212> PRT
   <213> Artificial Sequence
<220>
   <223> Description of Artificial Sequence: Synthetic peptide
<400> 4
   Gly Gly Gly Gly Ser
   1               5
<210> 5
   <211> 4
   <212> PRT
   <213> Artificial Sequence
<220>
   <223> Description of Artificial Sequence: Synthetic peptide
<400> 5
   Gly Gly Gly Ser
   1
<210> 6
   <211> 5
   <212> PRT
   <213> Artificial Sequence
<220>
   <223> Description of Artificial Sequence: Synthetic peptide
<400> 6
   Gly Gly Gly Gly Ser
   1               5
<210> 7
   <211> 7
   <212> PRT
   <213> Artificial Sequence
<220>
   <223> Description of Artificial Sequence: Synthetic peptide
<400> 7
   Pro Lys Lys Lys Arg Arg Val
   1               5
<210> 8
   <211> 16
   <212> PRT
   <213> Artificial Sequence
<220>
   <223> Description of Artificial Sequence: Synthetic peptide
<400> 8
   Lys Arg Pro Ala Ala Thr Lys Lys Ala Gly Gln Ala Lys Lys Lys Lys
   1               5                   10                  15
<210> 9
   <211> 20
   <212> DNA
   <213> Artificial Sequence
<220>
   <223> Description of Artificial Sequence: Synthetic oligonucleotide
<400> 9
   gggcacgggc agcttgccgg 20
<210> 10
   <211> 24
   <212> DNA
   <213> Artificial Sequence
<220>
   <223> Description of Artificial Sequence: Synthetic oligonucleotide
<400> 10
   gggcacgggc agcttgccgg tggt 24
<210> 11
   <211> 18
   <212> DNA
   <213> Artificial Sequence
<220>
   <223> Description of Artificial Sequence: Synthetic oligonucleotide
<400> 11
   gcacgggcag cttgccgg 18
<210> 12
   <211> 22
   <212> DNA
   <213> Artificial Sequence
<220>
   <223> Description of Artificial Sequence: Synthetic oligonucleotide
<400> 12
   gcacgggcag cttgccggtg gt 22
<210> 13
   <211> 20
   <212> DNA
   <213> Artificial Sequence
<220>
   <223> Description of Artificial Sequence: Synthetic oligonucleotide
<400> 13
   gggcacccgc agcttgccgg 20
<210> 14
   <211> 24
   <212> DNA
   <213> Artificial Sequence
<220>
   <223> Description of Artificial Sequence: Synthetic oligonucleotide
<400> 14
   gggcacccgc agcttgccgg tggt 24
<210> 15
   <211> 20
   <212> DNA
   <213> Artificial Sequence
<220>
   <223> Description of Artificial Sequence: Synthetic oligonucleotide
<400> 15
   gggctggggc agcttgccgg 20
<210> 16
   <211> 24
   <212> DNA
   <213> Artificial Sequence
<220>
   <223> Description of Artificial Sequence: Synthetic oligonucleotide
<400> 16
   gggctggggc agcttgccgg tggt 24
<210> 17
   <211> 20
   <212> DNA
   <213> Artificial Sequence
<220>
   <223> Description of Artificial Sequence: Synthetic oligonucleotide
<400> 17
   ggcgacgggc agcttgccgg 20
<210> 18
   <211> 24
   <212> DNA
   <213> Artificial Sequence
<220>
   <223> Description of Artificial Sequence: Synthetic oligonucleotide
<400> 18
   ggcgacgggc agcttgccgg tggt 24
<210> 19
   <211> 20
   <212> DNA
   <213> Artificial Sequence
<220>
   <223> Description of Artificial Sequence: Synthetic oligonucleotide
<400> 19
   gcccacgggc agcttgccgg 20
<210> 20
   <211> 24
   <212> DNA
   <213> Artificial Sequence
<220>
   <223> Description of Artificial Sequence: Synthetic oligonucleotide
<400> 20
   gcccacgggc agcttgccgg tggt 24
<210> 21
   <211> 20
   <212> DNA
   <213> Artificial Sequence
<220>
   <223> Description of Artificial Sequence: Synthetic oligonucleotide
<400> 21
   gtcgccctcg aacttcacct 20
<210> 22
   <211> 24
   <212> DNA
   <213> Artificial Sequence
<220>
   <223> Description of Artificial Sequence: Synthetic oligonucleotide
<400> 22
   gtcgccctcg aacttcacct cggc 24
<210> 23
   <211> 20
   <212> DNA
   <213> Artificial Sequence
<220>
   <223> Description of Artificial Sequence: Synthetic oligonucleotide
<400> 23
   gtaggtcagg gtggtcacga 20
<210> 24
   <211> 24
   <212> DNA
   <213> Artificial Sequence
<220>
   <223> Description of Artificial Sequence: Synthetic oligonucleotide
<400> 24
   gtaggtcagg gtggtcacga gggt 24
<210> 25
   <211> 20
   <212> DNA
   <213> Artificial Sequence
<220>
   <223> Description of Artificial Sequence: Synthetic oligonucleotide
<400> 25
   ggcgagggcg atgccaccta 20
<210> 26
   <211> 24
   <212> DNA
   <213> Artificial Sequence
<220>
   <223> Description of Artificial Sequence: Synthetic oligonucleotide
<400> 26
   ggcgagggcg atgccaccta cggc 24
<210> 27
   <211> 20
   <212> DNA
   <213> Artificial Sequence
<220>
   <223> Description of Artificial Sequence: Synthetic oligonucleotide
<400> 27
   ggtcgccacc atggtgagca 20
<210> 28
   <211> 24
   <212> DNA
   <213> Artificial Sequence
<220>
   <223> Description of Artificial Sequence: Synthetic oligonucleotide
<400> 28
   ggtcgccacc atggtgagca aggg 24
<210> 29
   <211> 20
   <212> DNA
   <213> Artificial Sequence
<220>
   <223> Description of Artificial Sequence: Synthetic oligonucleotide
<400> 29
   ggtcagggtg gtcacgaggg 20
<210> 30
   <211> 24
   <212> DNA
   <213> Artificial Sequence
<220>
   <223> Description of Artificial Sequence: Synthetic oligonucleotide
<400> 30
   ggtcagggtg gtcacgaggg tggg 24
<210> 31
   <211> 20
   <212> DNA
   <213> Artificial Sequence
<220>
   <223> Description of Artificial Sequence: Synthetic oligonucleotide
<400> 31
   ggtggtgcag atgaacttca 20
<210> 32
   <211> 24
   <212> DNA
   <213> Artificial Sequence
<220>
   <223> Description of Artificial Sequence: Synthetic oligonucleotide
<400> 32
   ggtggtgcag atgaacttca gggt 24
<210> 33
   <211> 17
   <212> DNA
   <213> Artificial Sequence
<220>
   <223> Description of Artificial Sequence: Synthetic oligonucleotide
<400> 33
   ggtgcagatg aacttca 17
<210> 34
   <211> 21
   <212> DNA
   <213> Artificial Sequence
<220>
   <223> Description of Artificial Sequence: Synthetic oligonucleotide
<400> 34
   ggtgcagatg aacttcaggg t 21
<210> 35
   <211> 20
   <212> DNA
   <213> Artificial Sequence
<220>
   <223> Description of Artificial Sequence: Synthetic oligonucleotide
<400> 35
   gttggggtct ttgctcaggg 20
<210> 36
   <211> 24
   <212> DNA
   <213> Artificial Sequence
<220>
   <223> Description of Artificial Sequence: Synthetic oligonucleotide
<400> 36
   gttggggtct ttgctcaggg cgga 24
<210> 37
   <211> 20
   <212> DNA
   <213> Artificial Sequence
<220>
   <223> Description of Artificial Sequence: Synthetic oligonucleotide
<400> 37
   ggtggtcacg agggtgggcc 20
<210> 38
   <211> 24
   <212> DNA
   <213> Artificial Sequence
<220>
   <223> Description of Artificial Sequence: Synthetic oligonucleotide
<400> 38
   ggtggtcacg agggtgggcc aggg 24
<210> 39
   <211> 20
   <212> DNA
   <213> Artificial Sequence
<220>
   <223> Description of Artificial Sequence: Synthetic oligonucleotide
<400> 39
   gatgccgttc ttctgcttgt 20
<210> 40
   <211> 24
   <212> DNA
   <213> Artificial Sequence
<220>
   <223> Description of Artificial Sequence: Synthetic oligonucleotide
<400> 40
   gatgccgttc ttctgcttgt cggc 24
<210> 41
   <211> 17
   <212> DNA
   <213> Artificial Sequence
<220>
   <223> Description of Artificial Sequence: Synthetic oligonucleotide
<400> 41
   gccgttcttc tgcttgt 17
<210> 42
   <211> 21
   <212> DNA
   <213> Artificial Sequence
<220>
   <223> Description of Artificial Sequence: Synthetic oligonucleotide
<400> 42
   gccgttcttc tgcttgtcgg c 21
<210> 43
   <211> 20
   <212> DNA
   <213> Artificial Sequence
<220>
   <223> Description of Artificial Sequence: Synthetic oligonucleotide
<400> 43
   gtcgccacca tggtgagcaa 20
<210> 44
   <211> 24
   <212> DNA
   <213> Artificial Sequence
<220>
   <223> Description of Artificial Sequence: Synthetic oligonucleotide
<400> 44
   gtcgccacca tggtgagcaa gggc 24
<210> 45
   <211> 20
   <212> DNA
   <213> Artificial Sequence
<220>
   <223> Description of Artificial Sequence: Synthetic oligonucleotide
<400> 45
   gcactgcacg ccgtaggtca 20
<210> 46
   <211> 24
   <212> DNA
   <213> Artificial Sequence
<220>
   <223> Description of Artificial Sequence: Synthetic oligonucleotide
<400> 46
   gcactgcacg ccgtaggtca gggt 24
<210> 47
   <211> 20
   <212> DNA
   <213> Artificial Sequence
<220>
   <223> Description of Artificial Sequence: Synthetic oligonucleotide
<400> 47
   gtgaaccgca tcgagctgaa 20
<210> 48
   <211> 24
   <212> DNA
   <213> Artificial Sequence
<220>
   <223> Description of Artificial Sequence: Synthetic oligonucleotide
<400> 48
   gtgaaccgca tcgagctgaa gggc 24
<210> 49
   <211> 20
   <212> DNA
   <213> Artificial Sequence
<220>
   <223> Description of Artificial Sequence: Synthetic oligonucleotide
<400> 49
   gaagggcatc gacttcaagg 20
<210> 50
   <211> 24
   <212> DNA
   <213> Artificial Sequence
<220>
   <223> Description of Artificial Sequence: Synthetic oligonucleotide
<400> 50
   gaagggcatc gacttcaagg agga 24
<210> 51
   <211> 20
   <212> DNA
   <213> Artificial Sequence
<220>
   <223> Description of Artificial Sequence: Synthetic oligonucleotide
<400> 51
   gcttcatgtg gtcggggtag 20
<210> 52
   <211> 24
   <212> DNA
   <213> Artificial Sequence
<220>
   <223> Description of Artificial Sequence: Synthetic oligonucleotide
<400> 52
   gcttcatgtg gtcggggtag cggc 24
<210> 53
   <211> 20
   <212> DNA
   <213> Artificial Sequence
<220>
   <223> Description of Artificial Sequence: Synthetic oligonucleotide
<400> 53
   gctgaagcac tgcacgccgt 20
<210> 54
   <211> 24
   <212> DNA
   <213> Artificial Sequence
<220>
   <223> Description of Artificial Sequence: Synthetic oligonucleotide
<400> 54
   gctgaagcac tgcacgccgt aggt 24
<210> 55
   <211> 20
   <212> DNA
   <213> Artificial Sequence
<220>
   <223> Description of Artificial Sequence: Synthetic oligonucleotide
<400> 55
   gccgtcgtcc ttgaagaaga 20
<210> 56
   <211> 24
   <212> DNA
   <213> Artificial Sequence
<220>
   <223> Description of Artificial Sequence: Synthetic oligonucleotide
<400> 56
   gccgtcgtcc ttgaagaaga tggt 24
<210> 57
   <211> 20
   <212> DNA
   <213> Artificial Sequence
<220>
   <223> Description of Artificial Sequence: Synthetic oligonucleotide
<400> 57
   gaccaggatg ggcaccaccc 20
<210> 58
   <211> 24
   <212> DNA
   <213> Artificial Sequence
<220>
   <223> Description of Artificial Sequence: Synthetic oligonucleotide
<400> 58
   gaccaggatg ggcaccaccc cggt 24
<210> 59
   <211> 20
   <212> DNA
   <213> Artificial Sequence
<220>
   <223> Description of Artificial Sequence: Synthetic oligonucleotide
<400> 59
   gacgtagcct tcgggcatgg 20
<210> 60
   <211> 24
   <212> DNA
   <213> Artificial Sequence
<220>
   <223> Description of Artificial Sequence: Synthetic oligonucleotide
<400> 60
   gacgtagcct tcgggcatgg cgga 24
<210> 61
   <211> 20
   <212> DNA
   <213> Artificial Sequence
<220>
   <223> Description of Artificial Sequence: Synthetic oligonucleotide
<400> 61
   gaagttcgag ggcgacaccc 20
<210> 62
   <211> 24
   <212> DNA
   <213> Artificial Sequence
<220>
   <223> Description of Artificial Sequence: Synthetic oligonucleotide
<400> 62
   gaagttcgag ggcgacaccc tggt 24
<210> 63
   <211> 20
   <212> DNA
   <213> Artificial Sequence
<220>
   <223> Description of Artificial Sequence: Synthetic oligonucleotide
<400> 63
   gagctggacg gcgacgtaaa 20
<210> 64
   <211> 24
   <212> DNA
   <213> Artificial Sequence
<220>
   <223> Description of Artificial Sequence: Synthetic oligonucleotide
<400> 64
   gagctggacg gcgacgtaaa cggc 24
<210> 65
   <211> 20
   <212> DNA
   <213> Artificial Sequence
<220>
   <223> Description of Artificial Sequence: Synthetic oligonucleotide
<400> 65
   ggcatcgccc tcgccctcgc 20
<210> 66
   <211> 24
   <212> DNA
   <213> Artificial Sequence
<220>
   <223> Description of Artificial Sequence: Synthetic oligonucleotide
<400> 66
   ggcatcgccc tcgccctcgc cgga 24
<210> 67
   <211> 20
   <212> DNA
   <213> Artificial Sequence
<220>
   <223> Description of Artificial Sequence: Synthetic oligonucleotide
<400> 67
   ggccacaagt tcagcgtgtc 20
<210> 68
   <211> 24
   <212> DNA
   <213> Artificial Sequence
<220>
   <223> Description of Artificial Sequence: Synthetic oligonucleotide
<400> 68
   ggccacaagt tcagcgtgtc cggc 24
<210> 69
   <211> 20
   <212> DNA
   <213> Artificial Sequence
<220>
   <223> Description of Artificial Sequence: Synthetic oligonucleotide
<400> 69
   gggcgaggag ctgttcaccg 20
<210> 70
   <211> 24
   <212> DNA
   <213> Artificial Sequence
<220>
   <223> Description of Artificial Sequence: Synthetic oligonucleotide
<400> 70
   gggcgaggag ctgttcaccg gggt 24
<210> 71
   <211> 18
   <212> DNA
   <213> Artificial Sequence
<220>
   <223> Description of Artificial Sequence: Synthetic oligonucleotide
<400> 71
   gcgaggagct gttcaccg 18
<210> 72
   <211> 22
   <212> DNA
   <213> Artificial Sequence
<220>
   <223> Description of Artificial Sequence: Synthetic oligonucleotide
<400> 72
   gcgaggagct gttcaccggg gt 22
<210> 73
   <211> 20
   <212> DNA
   <213> Artificial Sequence
<220>
   <223> Description of Artificial Sequence: Synthetic oligonucleotide
<400> 73
   cctcgaactt cacctcggcg 20
<210> 74
   <211> 24
   <212> DNA
   <213> Artificial Sequence
<220>
   <223> Description of Artificial Sequence: Synthetic oligonucleotide
<400> 74
   cctcgaactt cacctcggcg cggg 24
<210> 75
   <211> 20
   <212> DNA
   <213> Artificial Sequence
<220>
   <223> Description of Artificial Sequence: Synthetic oligonucleotide
<400> 75
   gctcgaactt cacctcggcg 20
<210> 76
   <211> 24
   <212> DNA
   <213> Artificial Sequence
<220>
   <223> Description of Artificial Sequence: Synthetic oligonucleotide
<400> 76
   gctcgaactt cacctcggcg cggg 24
<210> 77
   <211> 20
   <212> DNA
   <213> Artificial Sequence
<220>
   <223> Description of Artificial Sequence: Synthetic oligonucleotide
<400> 77
   caactacaag acccgcgccg 20
<210> 78
   <211> 24
   <212> DNA
   <213> Artificial Sequence
<220>
   <223> Description of Artificial Sequence: Synthetic oligonucleotide
<400> 78
   caactacaag acccgcgccg aggt 24
<210> 79
   <211> 20
   <212> DNA
   <213> Artificial Sequence
<220>
   <223> Description of Artificial Sequence: Synthetic oligonucleotide
<400> 79
   gaactacaag acccgcgccg 20
<210> 80
   <211> 24
   <212> DNA
   <213> Artificial Sequence
<220>
   <223> Description of Artificial Sequence: Synthetic oligonucleotide
<400> 80
   gaactacaag acccgcgccg aggt 24
<210> 81
   <211> 20
   <212> DNA
   <213> Artificial Sequence
<220>
   <223> Description of Artificial Sequence: Synthetic oligonucleotide
<400> 81
   cgctcctgga cgtagccttc 20
<210> 82
   <211> 24
   <212> DNA
   <213> Artificial Sequence
<220>
   <223> Description of Artificial Sequence: Synthetic oligonucleotide
<400> 82
   cgctcctgga cgtagccttc gggc 24
<210> 83
   <211> 20
   <212> DNA
   <213> Artificial Sequence
<220>
   <223> Description of Artificial Sequence: Synthetic oligonucleotide
<400> 83
   ggctcctgga cgtagccttc 20
<210> 84
   <211> 24
   <212> DNA
   <213> Artificial Sequence
<220>
   <223> Description of Artificial Sequence: Synthetic oligonucleotide
<400> 84
   cgctcctgga cgtagccttc gggc 24
<210> 85
   <211> 20
   <212> DNA
   <213> Artificial Sequence
<220>
   <223> Description of Artificial Sequence: Synthetic oligonucleotide
<400> 85
   agggcgagga gctgttcacc 20
<210> 86
   <211> 24
   <212> DNA
   <213> Artificial Sequence
<220>
   <223> Description of Artificial Sequence: Synthetic oligonucleotide
<400> 86
   agggcgagga gctgttcacc gggg 24
<210> 87
   <211> 20
   <212> DNA
   <213> Artificial Sequence
<220>
   <223> Description of Artificial Sequence: Synthetic oligonucleotide
<400> 87
   ggggcgagga gctgttcacc 20
<210> 88
   <211> 24
   <212> DNA
   <213> Artificial Sequence
<220>
   <223> Description of Artificial Sequence: Synthetic oligonucleotide
<400> 88
   ggggcgagga gctgttcacc gggg 24
<210> 89
   <211> 20
   <212> DNA
   <213> Artificial Sequence
<220>
   <223> Description of Artificial Sequence: Synthetic oligonucleotide
<400> 89
   gttcgagggc gacaccctgg 20
<210> 90
   <211> 24
   <212> DNA
   <213> Artificial Sequence
<220>
   <223> Description of Artificial Sequence: Synthetic oligonucleotide
<400> 90
   gttcgagggc gacaccctgg tgaa 24
<210> 91
   <211> 20
   <212> DNA
   <213> Artificial Sequence
<220>
   <223> Description of Artificial Sequence: Synthetic oligonucleotide
<400> 91
   gttcaccagg gtgtcgccct 20
<210> 92
   <211> 24
   <212> DNA
   <213> Artificial Sequence
<220>
   <223> Description of Artificial Sequence: Synthetic oligonucleotide
<400> 92
   gttcaccagg gtgtcgccct cgaa 24
<210> 93
   <211> 20
   <212> DNA
   <213> Artificial Sequence
<220>
   <223> Description of Artificial Sequence: Synthetic oligonucleotide
<400> 93
   gcccaccctc gtgaccaccc 20
<210> 94
   <211> 24
   <212> DNA
   <213> Artificial Sequence
<220>
   <223> Description of Artificial Sequence: Synthetic oligonucleotide
<400> 94
   gcccaccctc gtgaccaccc tgac 24
<210> 95
   <211> 20
   <212> DNA
   <213> Artificial Sequence
<220>
   <223> Description of Artificial Sequence: Synthetic oligonucleotide
<400> 95
   gcccttgctc accatggtgg 20
<210> 96
   <211> 24
   <212> DNA
   <213> Artificial Sequence
<220>
   <223> Description of Artificial Sequence: Synthetic oligonucleotide
<400> 96
   gcccttgctc accatggtgg cgac 24
<210> 97
   <211> 20
   <212> DNA
   <213> Artificial Sequence
<220>
   <223> Description of Artificial Sequence: Synthetic oligonucleotide
<400> 97
   gtcgccgtcc agctcgacca 20
<210> 98
   <211> 24
   <212> DNA
   <213> Artificial Sequence
<220>
   <223> Description of Artificial Sequence: Synthetic oligonucleotide
<400> 98
   gtcgccgtcc agctcgacca ggat 24
<210> 99
   <211> 20
   <212> DNA
   <213> Artificial Sequence
<220>
   <223> Description of Artificial Sequence: Synthetic oligonucleotide
<400> 99
   gtgtccggcg agggcgaggg 20
<210> 100
   <211> 24
   <212> DNA
   <213> Artificial Sequence
<220>
   <223> Description of Artificial Sequence: Synthetic oligonucleotide
<400> 100
   gtgtccggcg agggcgaggg cgat 24
<210> 101
   <211> 20
   <212> DNA
   <213> Artificial Sequence
<220>
   <223> Description of Artificial Sequence: Synthetic oligonucleotide
<400> 101
   ggggtggtgc ccatcctggt 20
<210> 102
   <211> 24
   <212> DNA
   <213> Artificial Sequence
<220>
   <223> Description of Artificial Sequence: Synthetic oligonucleotide
<400> 102
   ggggtggtgc ccatcctggt cgag 24
<210> 103
   <211> 20
   <212> DNA
   <213> Artificial Sequence
<220>
   <223> Description of Artificial Sequence: Synthetic oligonucleotide
<400> 103
   gccaccatgg tgagcaaggg 20
<210> 104
   <211> 24
   <212> DNA
   <213> Artificial Sequence
<220>
   <223> Description of Artificial Sequence: Synthetic oligonucleotide
<400> 104
   gccaccatgg tgagcaaggg cgag 24
<210> 105
   <211> 20
   <212> DNA
   <213> Artificial Sequence
<220>
   <223> Description of Artificial Sequence: Synthetic oligonucleotide
<400> 105
   gagtccgagc agaagaagaa 20
<210> 106
   <211> 24
   <212> DNA
   <213> Artificial Sequence
<220>
   <223> Description of Artificial Sequence: Synthetic oligonucleotide
<400> 106
   gagtccgagc agaagaagaa gggc 24
<210> 107
   <211> 20
   <212> DNA
   <213> Artificial Sequence
<220>
   <223> Description of Artificial Sequence: Synthetic oligonucleotide
<400> 107
   gtcacctcca atgactaggg 20
<210> 108
   <211> 24
   <212> DNA
   <213> Artificial Sequence
<220>
   <223> Description of Artificial Sequence: Synthetic oligonucleotide
<400> 108
   gtcacctcca atgactaggg tggg 24
<210> 109
   <211> 20
   <212> DNA
   <213> Artificial Sequence
<220>
   <223> Description of Artificial Sequence: Synthetic oligonucleotide
<400> 109
   gggaagactg aggctacata 20
<210> 110
   <211> 24
   <212> DNA
   <213> Artificial Sequence
<220>
   <223> Description of Artificial Sequence: Synthetic oligonucleotide
<400> 110
   gggaagactg aggctacata gggt 24
<210> 111
   <211> 20
   <212> DNA
   <213> Artificial Sequence
<220>
   <223> Description of Artificial Sequence: Synthetic oligonucleotide
<400> 111
   gccacgaagc aggccaatgg 20
<210> 112
   <211> 24
   <212> DNA
   <213> Artificial Sequence
<220>
   <223> Description of Artificial Sequence: Synthetic oligonucleotide
<400> 112
   gccacgaagc aggccaatgg ggag 24
<210> 113
   <211> 20
   <212> DNA
   <213> Artificial Sequence
<220>
   <223> Description of Artificial Sequence: Synthetic oligonucleotide
<400> 113
   ggaatccctt ctgcagcacc 20
<210> 114
   <211> 24
   <212> DNA
   <213> Artificial Sequence
<220>
   <223> Description of Artificial Sequence: Synthetic oligonucleotide
<400> 114
   ggaatccctt ctgcagcacc tgga 24
<210> 115
   <211> 20
   <212> DNA
   <213> Artificial Sequence
<220>
   <223> Description of Artificial Sequence: Synthetic oligonucleotide
<400> 115
   gctgcagaag ggattccatg 20
<210> 116
   <211> 24
   <212> DNA
   <213> Artificial Sequence
<220>
   <223> Description of Artificial Sequence: Synthetic oligonucleotide
<400> 116
   gctgcagaag ggattccatg aggt 24
<210> 117
   <211> 20
   <212> DNA
   <213> Artificial Sequence
<220>
   <223> Description of Artificial Sequence: Synthetic oligonucleotide
<400> 117
   ggcggctgca caaccagtgg 20
<210> 118
   <211> 24
   <212> DNA
   <213> Artificial Sequence
<220>
   <223> Description of Artificial Sequence: Synthetic oligonucleotide
<400> 118
   ggcggctgca caaccagtgg aggc 24
<210> 119
   <211> 20
   <212> DNA
   <213> Artificial Sequence
<220>
   <223> Description of Artificial Sequence: Synthetic oligonucleotide
<400> 119
   gctccagagc cgtgcgaatg 20
<210> 120
   <211> 24
   <212> DNA
   <213> Artificial Sequence
<220>
   <223> Description of Artificial Sequence: Synthetic oligonucleotide
<400> 120
   gctccagagc cgtgcgaatg gggc 24
<210> 121
   <211> 20
   <212> DNA
   <213> Artificial Sequence
<220>
   <223> Description of Artificial Sequence: Synthetic oligonucleotide
<400> 121
   gaatcccttc tgcagcacct 20
<210> 122
   <211> 24
   <212> DNA
   <213> Artificial Sequence
<220>
   <223> Description of Artificial Sequence: Synthetic oligonucleotide
<400> 122
   gaatcccttc tgcagcacct ggat 24
<210> 123
   <211> 20
   <212> DNA
   <213> Artificial Sequence
<220>
   <223> Description of Artificial Sequence: Synthetic oligonucleotide
<400> 123
   gcggcggctg cacaaccagt 20
<210> 124
   <211> 24
   <212> DNA
   <213> Artificial Sequence
<220>
   <223> Description of Artificial Sequence: Synthetic oligonucleotide
<400> 124
   gcggcggctg cacaaccagt ggag 24
<210> 125
   <211> 20
   <212> DNA
   <213> Artificial Sequence
<220>
   <223> Description of Artificial Sequence: Synthetic oligonucleotide
<400> 125
   ggttgtgcag ccgccgctcc 20
<210> 126
   <211> 24
   <212> DNA
   <213> Artificial Sequence
<220>
   <223> Description of Artificial Sequence: Synthetic oligonucleotide
<400> 126
   ggttgtgcag ccgccgctcc agag 24
<210> 127
   <211> 20
   <212> DNA
   <213> Artificial Sequence
<220>
   <223> Description of Artificial Sequence: Synthetic oligonucleotide
<400> 127
   gcattttcag gaggaagcga 20
<210> 128
   <211> 24
   <212> DNA
   <213> Artificial Sequence
<220>
   <223> Description of Artificial Sequence: Synthetic oligonucleotide
<400> 128
   gcattttcag gaggaagcga tggc 24
<210> 129
   <211> 20
   <212> DNA
   <213> Artificial Sequence
<220>
   <223> Description of Artificial Sequence: Synthetic oligonucleotide
<400> 129
   gggagaagaa agagagatgt 20
<210> 130
   <211> 24
   <212> DNA
   <213> Artificial Sequence
<220>
   <223> Description of Artificial Sequence: Synthetic oligonucleotide
<400> 130
   gggagaagaa agagagatgt aggg 24
<210> 131
   <211> 20
   <212> DNA
   <213> Artificial Sequence
<220>
   <223> Description of Artificial Sequence: Synthetic oligonucleotide
<400> 131
   ggtgcatttt caggaggaag 20
<210> 132
   <211> 24
   <212> DNA
   <213> Artificial Sequence
<220>
   <223> Description of Artificial Sequence: Synthetic oligonucleotide
<400> 132
   ggtgcatttt caggaggaag cgat 24
<210> 133
   <211> 20
   <212> DNA
   <213> Artificial Sequence
<220>
   <223> Description of Artificial Sequence: Synthetic oligonucleotide
<400> 133
   gagatgtagg gctagagggg 20
<210> 134
   <211> 24
   <212> DNA
   <213> Artificial Sequence
<220>
   <223> Description of Artificial Sequence: Synthetic oligonucleotide
<400> 134
   gagatgtagg gctagagggg tgag 24
<210> 135
   <211> 20
   <212> DNA
   <213> Artificial Sequence
<220>
   <223> Description of Artificial Sequence: Synthetic oligonucleotide
<400> 135
   ggtatccagc agaggggaga 20
<210> 136
   <211> 24
   <212> DNA
   <213> Artificial Sequence
<220>
   <223> Description of Artificial Sequence: Synthetic oligonucleotide
<400> 136
   ggtatccagc agaggggaga agaa 24
<210> 137
   <211> 20
   <212> DNA
   <213> Artificial Sequence
<220>
   <223> Description of Artificial Sequence: Synthetic oligonucleotide
<400> 137
   gaggcatctc tgcaccgagg 20
<210> 138
   <211> 24
   <212> DNA
   <213> Artificial Sequence
<220>
   <223> Description of Artificial Sequence: Synthetic oligonucleotide
<400> 138
   gaggcatctc tgcaccgagg tgaa 24
<210> 139
   <211> 20
   <212> DNA
   <213> Artificial Sequence
<220>
   <223> Description of Artificial Sequence: Synthetic oligonucleotide
<400> 139
   gaggggtgag gctgaaacag 20
<210> 140
   <211> 24
   <212> DNA
   <213> Artificial Sequence
<220>
   <223> Description of Artificial Sequence: Synthetic oligonucleotide
<400> 140
   gaggggtgag gctgaaacag tgac 24
<210> 141
   <211> 20
   <212> DNA
   <213> Artificial Sequence
<220>
   <223> Description of Artificial Sequence: Synthetic oligonucleotide
<400> 141
   gagcaaaagt agatattaca 20
<210> 142
   <211> 24
   <212> DNA
   <213> Artificial Sequence
<220>
   <223> Description of Artificial Sequence: Synthetic oligonucleotide
<400> 142
   gagcaaaagt agatattaca agac 24
<210> 143
   <211> 20
   <212> DNA
   <213> Artificial Sequence
<220>
   <223> Description of Artificial Sequence: Synthetic oligonucleotide
<400> 143
   ggaattcaaa ctgaggcata 20
<210> 144
   <211> 24
   <212> DNA
   <213> Artificial Sequence
<220>
   <223> Description of Artificial Sequence: Synthetic oligonucleotide
<400> 144
   ggaattcaaa ctgaggcata tgat 24
<210> 145
   <211> 20
   <212> DNA
   <213> Artificial Sequence
<220>
   <223> Description of Artificial Sequence: Synthetic oligonucleotide
<400> 145
   gcagagggga gaagaaagag 20
<210> 146
   <211> 24
   <212> DNA
   <213> Artificial Sequence
<220>
   <223> Description of Artificial Sequence: Synthetic oligonucleotide
<400> 146
   gcagagggga gaagaaagag agat 24
<210> 147
   <211> 20
   <212> DNA
   <213> Artificial Sequence
<220>
   <223> Description of Artificial Sequence: Synthetic oligonucleotide
<400> 147
   gcaccgaggc atctctgcac 20
<210> 148
   <211> 24
   <212> DNA
   <213> Artificial Sequence
<220>
   <223> Description of Artificial Sequence: Synthetic oligonucleotide
<400> 148
   gcaccgaggc atctctgcac cgag 24
<210> 149
   <211> 20
   <212> DNA
   <213> Artificial Sequence
<220>
   <223> Description of Artificial Sequence: Synthetic oligonucleotide
<400> 149
   gagatgtagg gctagagggg 20
<210> 150
   <211> 24
   <212> DNA
   <213> Artificial Sequence
<220>
   <223> Description of Artificial Sequence: Synthetic oligonucleotide
<400> 150
   gagatgtagg gctagagggg tgag 24
<210> 151
   <211> 20
   <212> DNA
   <213> Artificial Sequence
<220>
   <223> Description of Artificial Sequence: Synthetic oligonucleotide
<400> 151
   gtgcggcaag agcttcagcc 20
<210> 152
   <211> 24
   <212> DNA
   <213> Artificial Sequence
<220>
   <223> Description of Artificial Sequence: Synthetic oligonucleotide
<400> 152
   gtgcggcaag agcttcagcc gggg 24
<210> 153
   <211> 20
   <212> DNA
   <213> Artificial Sequence
<220>
   <223> Description of Artificial Sequence: Synthetic oligonucleotide
<400> 153
   gggtgggggg agtttgctcc 20
<210> 154
   <211> 24
   <212> DNA
   <213> Artificial Sequence
<220>
   <223> Description of Artificial Sequence: Synthetic oligonucleotide
<400> 154
   gggtgggggg agtttgctcc tgga 24
<210> 155
   <211> 20
   <212> DNA
   <213> Artificial Sequence
<220>
   <223> Description of Artificial Sequence: Synthetic oligonucleotide
<400> 155
   gaccccctcc accccgcctc 20
<210> 156
   <211> 24
   <212> DNA
   <213> Artificial Sequence
<220>
   <223> Description of Artificial Sequence: Synthetic oligonucleotide
<400> 156
   gaccccctcc accccgcctc cggg 24
<210> 157
   <211> 20
   <212> DNA
   <213> Artificial Sequence
<220>
   <223> Description of Artificial Sequence: Synthetic oligonucleotide
<400> 157
   ggtgagtgag tgtgtgcgtg 20
<210> 158
   <211> 24
   <212> DNA
   <213> Artificial Sequence
<220>
   <223> Description of Artificial Sequence: Synthetic oligonucleotide
<400> 158
   ggtgagtgag tgtgtgcgtg tggg 24
<210> 159
   <211> 20
   <212> DNA
   <213> Artificial Sequence
<220>
   <223> Description of Artificial Sequence: Synthetic oligonucleotide
<400> 159
   gcgagcagcg tcttcgagag 20
<210> 160
   <211> 24
   <212> DNA
   <213> Artificial Sequence
<220>
   <223> Description of Artificial Sequence: Synthetic oligonucleotide
<400> 160
   gcgagcagcg tcttcgagag tgag 24
<210> 161
   <211> 20
   <212> DNA
   <213> Artificial Sequence
<220>
   <223> Description of Artificial Sequence: Synthetic oligonucleotide
<400> 161
   gtgcggcaag agcttcagcc 20
<210> 162
   <211> 24
   <212> DNA
   <213> Artificial Sequence
<220>
   <223> Description of Artificial Sequence: Synthetic oligonucleotide
<400> 162
   gtgcggcaag agcttcagcc agag 24
<210> 163
   <211> 23
   <212> DNA
   <213> Artificial Sequence
<220>
   <223> Description of Artificial Sequence: Synthetic oligonucleotide
<400> 163
   gagtccgagc agaagaagaa ggg 23
<210> 164
   <211> 23
   <212> DNA
   <213> Artificial Sequence
<220>
   <223> Description of Artificial Sequence: Synthetic oligonucleotide
<400> 164
   gtcacctcca atgactaggg tgg 23
<210> 165
   <211> 23
   <212> DNA
   <213> Artificial Sequence
<220>
   <223> Description of Artificial Sequence: Synthetic oligonucleotide
<400> 165
   ggaatccctt ctgcagcacc tgg 23
<210> 166
   <211> 23
   <212> DNA
   <213> Artificial Sequence
<220>
   <223> Description of Artificial Sequence: Synthetic oligonucleotide
<400> 166
   gctgcagaag ggattccatg agg 23
<210> 167
   <211> 23
   <212> DNA
   <213> Artificial Sequence
<220>
   <223> Description of Artificial Sequence: Synthetic oligonucleotide
<400> 167
   ggcggctgca caaccagtgg agg 23
<210> 168
   <211> 23
   <212> DNA
   <213> Artificial Sequence
<220>
   <223> Description of Artificial Sequence: Synthetic oligonucleotide
<400> 168
   gctccagagc cgtgcgaatg ggg 23
<210> 169
   <211> 23
   <212> DNA
   <213> Artificial Sequence
<220>
   <223> Description of Artificial Sequence: Synthetic oligonucleotide
<400> 169
   gcattttcag gaggaagcga tgg 23
<210> 170
   <211> 23
   <212> DNA
   <213> Artificial Sequence
<220>
   <223> Description of Artificial Sequence: Synthetic oligonucleotide
<400> 170
   gtgcggcaag agcttcagcc ggg 23
<210> 171
   <211> 23
   <212> DNA
   <213> Artificial Sequence
<220>
   <223> Description of Artificial Sequence: Synthetic oligonucleotide
<400> 171
   gaccccctcc accccgcctc cgg 23
<210> 172
   <211> 23
   <212> DNA
   <213> Artificial Sequence
<220>
   <223> Description of Artificial Sequence: Synthetic oligonucleotide
<400> 172
   ggtgagtgag tgtgtgcgtg tgg 23
<210> 173
   <211> 20
   <212> DNA
   <213> Artificial Sequence
<220>
   <223> Description of Artificial Sequence: Synthetic primer
<400> 173
   ggagcagctg gtcagagggg 20
<210> 174
   <211> 22
   <212> DNA
   <213> Artificial Sequence
<220>
   <223> Description of Artificial Sequence: Synthetic primer
<400> 174
   ccatagggaa gggggacact gg 22
<210> 175
   <211> 20
   <212> DNA
   <213> Artificial Sequence
<220>
   <223> Description of Artificial Sequence: Synthetic primer
<400> 175
   gggccgggaa agagttgctg 20
<210> 176
   <211> 22
   <212> DNA
   <213> Artificial Sequence
<220>
   <223> Description of Artificial Sequence: Synthetic primer
<400> 176
   gccctacatc tgctctccct cc 22
<210> 177
   <211> 25
   <212> DNA
   <213> Artificial Sequence
<220>
   <223> Description of Artificial Sequence: Synthetic primer
<400> 177
   ccagcacaac ttactcgcac ttgac 25
<210> 178
   <211> 22
   <212> DNA
   <213> Artificial Sequence
<220>
   <223> Description of Artificial Sequence: Synthetic primer
<400> 178
   catcaccaac ccacagccaa gg 22
<210> 179
   <211> 20
   <212> DNA
   <213> Artificial Sequence
<220>
   <223> Description of Artificial Sequence: Synthetic primer
<400> 179
   tccagatggc acattgtcag 20
<210> 180
   <211> 20
   <212> DNA
   <213> Artificial Sequence
<220>
   <223> Description of Artificial Sequence: Synthetic primer
<400> 180
   agggagcagg aaagtgaggt 20
<210> 181
   <211> 22
   <212> DNA
   <213> Artificial Sequence
<220>
   <223> Description of Artificial Sequence: Synthetic primer
<400> 181
   cgaggaagag agagacgggg tc 22
<210> 182
   <211> 23
   <212> DNA
   <213> Artificial Sequence
<220>
   <223> Description of Artificial Sequence: Synthetic primer
<400> 182
   ctccaatgca cccaagacag cag 23
<210> 183
   <211> 20
   <212> DNA
   <213> Artificial Sequence
<220>
   <223> Description of Artificial Sequence: Synthetic primer
<400> 183
   agtgtggggt gtgtgggaag 20
<210> 184
   <211> 20
   <212> DNA
   <213> Artificial Sequence
<220>
   <223> Description of Artificial Sequence: Synthetic primer
<400> 184
   gcaaggggaa gactctggca 20
<210> 185
   <211> 19
   <212> DNA
   <213> Artificial Sequence
<220>
   <223> Description of Artificial Sequence: Synthetic primer
<400> 185
   tacgagtgcc tagagtgcg 19
<210> 186
   <211> 22
   <212> DNA
   <213> Artificial Sequence
<220>
   <223> Description of Artificial Sequence: Synthetic primer
<400> 186
   gcagatgtag gtcttggagg ac 22
<210> 187
   <211> 20
   <212> DNA
   <213> Artificial Sequence
<220>
   <223> Description of Artificial Sequence: Synthetic primer
<400> 187
   ggagcagctg gtcagagggg 20
<210> 188
   <211> 22
   <212> DNA
   <213> Artificial Sequence
<220>
   <223> Description of Artificial Sequence: Synthetic primer
<400> 188
   cgatgtcctc cccattggcc tg 22
<210> 189
   <211> 25
   <212> DNA
   <213> Artificial Sequence
<220>
   <223> Description of Artificial Sequence: Synthetic primer
<400> 189
   gtggggagat ttgcatctgt ggagg 25
<210> 190
   <211> 26
   <212> DNA
   <213> Artificial Sequence
<220>
   <223> Description of Artificial Sequence: Synthetic primer
<400> 190
   gcttttatac catcttgggg ttacag 26
<210> 191
   <211> 24
   <212> DNA
   <213> Artificial Sequence
<220>
   <223> Description of Artificial Sequence: Synthetic primer
<400> 191
   caatgtgctt caacccatca cggc 24
<210> 192
   <211> 27
   <212> DNA
   <213> Artificial Sequence
<220>
   <223> Description of Artificial Sequence: Synthetic primer
<400> 192
   ccatgaattt gtgatggatg cagtctg 27
<210> 193
   <211> 24
   <212> DNA
   <213> Artificial Sequence
<220>
   <223> Description of Artificial Sequence: Synthetic primer
<400> 193
   gagaaggagg tgcaggagct agac 24
<210> 194
   <211> 24
   <212> DNA
   <213> Artificial Sequence
<220>
   <223> Description of Artificial Sequence: Synthetic primer
<400> 194
   catcccgacc ttcatccctc ctgg 24
<210> 195
   <211> 25
   <212> DNA
   <213> Artificial Sequence
<220>
   <223> Description of Artificial Sequence: Synthetic primer
<400> 195
   gtagttctga cattcctcct gaggg 25
<210> 196
   <211> 23
   <212> DNA
   <213> Artificial Sequence
<220>
   <223> Description of Artificial Sequence: Synthetic primer
<400> 196
   tcaaacaagg tgcagataca gca 23
<210> 197
   <211> 22
   <212> DNA
   <213> Artificial Sequence
<220>
   <223> Description of Artificial Sequence: Synthetic primer
<400> 197
   cagggtcgct cagtctgtgt gg 22
<210> 198
   <211> 24
   <212> DNA
   <213> Artificial Sequence
<220>
   <223> Description of Artificial Sequence: Synthetic primer
<400> 198
   ccagcgcacc attcactcca cctg 24
<210> 199
   <211> 25
   <212> DNA
   <213> Artificial Sequence
<220>
   <223> Description of Artificial Sequence: Synthetic primer
<400> 199
   ggctgaagag gaagaccaga ctcag 25
<210> 200
   <211> 25
   <212> DNA
   <213> Artificial Sequence
<220>
   <223> Description of Artificial Sequence: Synthetic primer
<400> 200
   ggcccctctg aattcaattc tctgc 25
<210> 201
   <211> 21
   <212> DNA
   <213> Artificial Sequence
<220>
   <223> Description of Artificial Sequence: Synthetic primer
<400> 201
   ccacagcgag gagtgacagc c 21
<210> 202
   <211> 26
   <212> DNA
   <213> Artificial Sequence
<220>
   <223> Description of Artificial Sequence: Synthetic primer
<400> 202
   ccaagtcttt cctaactcga ccttgg 26
<210> 203
   <211> 22
   <212> DNA
   <213> Artificial Sequence
<220>
   <223> Description of Artificial Sequence: Synthetic primer
<400> 203
   ccctaggccc acaccagcaa tg 22
<210> 204
   <211> 24
   <212> DNA
   <213> Artificial Sequence
<220>
   <223> Description of Artificial Sequence: Synthetic primer
<400> 204
   gggatgggaa tgggaatgtg aggc 24
<210> 205
   <211> 22
   <212> DNA
   <213> Artificial Sequence
<220>
   <223> Description of Artificial Sequence: Synthetic primer
<400> 205
   gcccaggtga aggtgtggtt cc 22
<210> 206
   <211> 21
   <212> DNA
   <213> Artificial Sequence
<220>
   <223> Description of Artificial Sequence: Synthetic primer
<400> 206
   ccaaagcctg gccagggagt g 21
<210> 207
   <211> 25
   <212> DNA
   <213> Artificial Sequence
<220>
   <223> Description of Artificial Sequence: Synthetic primer
<400> 207
   aggcaaagat ctaggacctg gatgg 25
<210> 208
   <211> 24
   <212> DNA
   <213> Artificial Sequence
<220>
   <223> Description of Artificial Sequence: Synthetic primer
<400> 208
   ccatctgagt cagccagcct tgtc 24
<210> 209
   <211> 21
   <212> DNA
   <213> Artificial Sequence
<220>
   <223> Description of Artificial Sequence: Synthetic primer
<400> 209
   ggttccctcc cttctgagcc c 21
<210> 210
   <211> 25
   <212> DNA
   <213> Artificial Sequence
<220>
   <223> Description of Artificial Sequence: Synthetic primer
<400> 210
   ggataggaat gaagaccccc tctcc 25
<210> 211
   <211> 25
   <212> DNA
   <213> Artificial Sequence
<220>
   <223> Description of Artificial Sequence: Synthetic primer
<400> 211
   ggactggctg gctgtgtgtt ttgag 25
<210> 212
   <211> 24
   <212> DNA
   <213> Artificial Sequence
<220>
   <223> Description of Artificial Sequence: Synthetic primer
<400> 212
   cttatccagg gctacctcat tgcc 24
<210> 213
   <211> 25
   <212> DNA
   <213> Artificial Sequence
<220>
   <223> Description of Artificial Sequence: Synthetic primer
<400> 213
   gctgctgctg ctttgatcac tcctg 25
<210> 214
   <211> 24
   <212> DNA
   <213> Artificial Sequence
<220>
   <223> Description of Artificial Sequence: Synthetic primer
<400> 214
   ctccttaaac cctcagaagc tggc 24
<210> 215
   <211> 23
   <212> DNA
   <213> Artificial Sequence
<220>
   <223> Description of Artificial Sequence: Synthetic primer
<400> 215
   gcactgtcag ctgatcctac agg 23
<210> 216
   <211> 24
   <212> DNA
   <213> Artificial Sequence
<220>
   <223> Description of Artificial Sequence: Synthetic primer
<400> 216
   acgttggaac agtcgagctg tagc 24
<210> 217
   <211> 25
   <212> DNA
   <213> Artificial Sequence
<220>
   <223> Description of Artificial Sequence: Synthetic primer
<400> 217
   tgtgcataac tcatgttggc aaact 25
<210> 218
   <211> 23
   <212> DNA
   <213> Artificial Sequence
<220>
   <223> Description of Artificial Sequence: Synthetic primer
<400> 218
   tccacaacta ccctcagctg gag 23
<210> 219
   <211> 25
   <212> DNA
   <213> Artificial Sequence
<220>
   <223> Description of Artificial Sequence: Synthetic primer
<400> 219
   ccactgacaa ttcactcaac cctgc 25
<210> 220
   <211> 24
   <212> DNA
   <213> Artificial Sequence
<220>
   <223> Description of Artificial Sequence: Synthetic primer
<400> 220
   aggcagacca gttatttggc agtc 24
<210> 221
   <211> 22
   <212> DNA
   <213> Artificial Sequence
<220>
   <223> Description of Artificial Sequence: Synthetic primer
<400> 221
   acaggcgcag ttcactgaga ag 22
<210> 222
   <211> 22
   <212> DNA
   <213> Artificial Sequence
<220>
   <223> Description of Artificial Sequence: Synthetic primer
<400> 222
   gggtaggctg actttgggct cc 22
<210> 223
   <211> 22
   <212> DNA
   <213> Artificial Sequence
<220>
   <223> Description of Artificial Sequence: Synthetic primer
<400> 223
   gccctcttgc ctccactggt tg 22
<210> 224
   <211> 23
   <212> DNA
   <213> Artificial Sequence
<220>
   <223> Description of Artificial Sequence: Synthetic primer
<400> 224
   cgcggatgtt ccaatcagta cgc 23
<210> 225
   <211> 22
   <212> DNA
   <213> Artificial Sequence
<220>
   <223> Description of Artificial Sequence: Synthetic primer
<400> 225
   gcgggcagtg gcgtcttagt cg 22
<210> 226
   <211> 22
   <212> DNA
   <213> Artificial Sequence
<220>
   <223> Description of Artificial Sequence: Synthetic primer
<400> 226
   ccctgggttt ggttggctgc tc 22
<210> 227
   <211> 21
   <212> DNA
   <213> Artificial Sequence
<220>
   <223> Description of Artificial Sequence: Synthetic primer
<400> 227
   ctccttgccg cccagccggt c 21
<210> 228
   <211> 23
   <212> DNA
   <213> Artificial Sequence
<220>
   <223> Description of Artificial Sequence: Synthetic primer
<400> 228
   cactggggaa gaggcgagga cac 23
<210> 229
   <211> 23
   <212> DNA
   <213> Artificial Sequence
<220>
   <223> Description of Artificial Sequence: Synthetic primer
<400> 229
   ccagtgtttc ccatccccaa cac 23
<210> 230
   <211> 23
   <212> DNA
   <213> Artificial Sequence
<220>
   <223> Description of Artificial Sequence: Synthetic primer
<400> 230
   gaatggatcc ccccctagag ctc 23
<210> 231
   <211> 22
   <212> DNA
   <213> Artificial Sequence
<220>
   <223> Description of Artificial Sequence: Synthetic primer
<400> 231
   caggcccaca ggtccttctg ga 22
<210> 232
   <211> 21
   <212> DNA
   <213> Artificial Sequence
<220>
   <223> Description of Artificial Sequence: Synthetic primer
<400> 232
   ccacacggaa ggctgaccac g 21
<210> 233
   <211> 21
   <212> DNA
   <213> Artificial Sequence
<220>
   <223> Description of Artificial Sequence: Synthetic primer
<400> 233
   gcgcagagag agcaggacgt c 21
<210> 234
   <211> 23
   <212> DNA
   <213> Artificial Sequence
<220>
   <223> Description of Artificial Sequence: Synthetic primer
<400> 234
   gcacctcatg gaatcccttc tgc 23
<210> 235
   <211> 23
   <212> DNA
   <213> Artificial Sequence
<220>
   <223> Description of Artificial Sequence: Synthetic primer
<400> 235
   caagtgatgc gacttccaac ctc 23
<210> 236
   <211> 26
   <212> DNA
   <213> Artificial Sequence
<220>
   <223> Description of Artificial Sequence: Synthetic primer
<400> 236
   ccctcagagt tcagcttaaa aagacc 26
<210> 237
   <211> 25
   <212> DNA
   <213> Artificial Sequence
<220>
   <223> Description of Artificial Sequence: Synthetic primer
<400> 237
   tgcttctcat ccactctaga ctgct 25
<210> 238
   <211> 22
   <212> DNA
   <213> Artificial Sequence
<220>
   <223> Description of Artificial Sequence: Synthetic primer
<400> 238
   caccaaccag ccatgtgcca tg 22
<210> 239
   <211> 22
   <212> DNA
   <213> Artificial Sequence
<220>
   <223> Description of Artificial Sequence: Synthetic primer
<400> 239
   ctgcctgtgc tcctcgatgg tg 22
<210> 240
   <211> 22
   <212> DNA
   <213> Artificial Sequence
<220>
   <223> Description of Artificial Sequence: Synthetic primer
<400> 240
   gggttcaaag ctcatctgcc cc 22
<210> 241
   <211> 23
   <212> DNA
   <213> Artificial Sequence
<220>
   <223> Description of Artificial Sequence: Synthetic primer
<400> 241
   gcatgtgcct tgagattgcc tgg 23
<210> 242
   <211> 25
   <212> DNA
   <213> Artificial Sequence
<220>
   <223> Description of Artificial Sequence: Synthetic primer
<400> 242
   gacattcaga gaagcgacca tgtgg 25
<210> 243
   <211> 23
   <212> DNA
   <213> Artificial Sequence
<220>
   <223> Description of Artificial Sequence: Synthetic primer
<400> 243
   ccatcttccc ctttggccca cag 23
<210> 244
   <211> 25
   <212> DNA
   <213> Artificial Sequence
<220>
   <223> Description of Artificial Sequence: Synthetic primer
<400> 244
   ccccaaaagt ggccaagagc ctgag 25
<210> 245
   <211> 26
   <212> DNA
   <213> Artificial Sequence
<220>
   <223> Description of Artificial Sequence: Synthetic primer
<400> 245
   gttctccaaa ggaagagagg ggaatg 26
<210> 246
   <211> 22
   <212> DNA
   <213> Artificial Sequence
<220>
   <223> Description of Artificial Sequence: Synthetic primer
<400> 246
   ggtgctgtgt cctcatgcat cc 22
<210> 247
   <211> 22
   <212> DNA
   <213> Artificial Sequence
<220>
   <223> Description of Artificial Sequence: Synthetic primer
<400> 247
   cggcttgcct agggtcgttg ag 22
<210> 248
   <211> 22
   <212> DNA
   <213> Artificial Sequence
<220>
   <223> Description of Artificial Sequence: Synthetic primer
<400> 248
   ccttcagggg ctcttccagg tc 22
<210> 249
   <211> 21
   <212> DNA
   <213> Artificial Sequence
<220>
   <223> Description of Artificial Sequence: Synthetic primer
<400> 249
   gggaactggc aggcaccgag g 21
<210> 250
   <211> 22
   <212> DNA
   <213> Artificial Sequence
<220>
   <223> Description of Artificial Sequence: Synthetic primer
<400> 250
   gggtgaggct gaaacagtga cc 22
<210> 251
   <211> 26
   <212> DNA
   <213> Artificial Sequence
<220>
   <223> Description of Artificial Sequence: Synthetic primer
<400> 251
   gggaggatgt tggttttagg gaactg 26
<210> 252
   <211> 27
   <212> DNA
   <213> Artificial Sequence
<220>
   <223> Description of Artificial Sequence: Synthetic primer
<400> 252
   tccaatcact acatgccatt ttgaaga 27
<210> 253
   <211> 24
   <212> DNA
   <213> Artificial Sequence
<220>
   <223> Description of Artificial Sequence: Synthetic primer
<400> 253
   ccaccctctt cctttgatcc tccc 24
<210> 254
   <211> 23
   <212> DNA
   <213> Artificial Sequence
<220>
   <223> Description of Artificial Sequence: Synthetic primer
<400> 254
   tcctccctac tccttcaccc agg 23
<210> 255
   <211> 23
   <212> DNA
   <213> Artificial Sequence
<220>
   <223> Description of Artificial Sequence: Synthetic primer
<400> 255
   gagtgcctga catgtgggga gag 23
<210> 256
   <211> 23
   <212> DNA
   <213> Artificial Sequence
<220>
   <223> Description of Artificial Sequence: Synthetic primer
<400> 256
   tccagctaaa gcctttccca cac 23
<210> 257
   <211> 26
   <212> DNA
   <213> Artificial Sequence
<220>
   <223> Description of Artificial Sequence: Synthetic primer
<400> 257
   gaactctctg atgcacctga aggctg 26
<210> 258
   <211> 25
   <212> DNA
   <213> Artificial Sequence
<220>
   <223> Description of Artificial Sequence: Synthetic primer
<400> 258
   accgtatcag tgtgatgcat gtggt 25
<210> 259
   <211> 28
   <212> DNA
   <213> Artificial Sequence
<220>
   <223> Description of Artificial Sequence: Synthetic primer
<400> 259
   tgggtttaat catgtgttct gcactatg 28
<210> 260
   <211> 24
   <212> DNA
   <213> Artificial Sequence
<220>
   <223> Description of Artificial Sequence: Synthetic primer
<400> 260
   cccatcttcc attctgccct ccac 24
<210> 261
   <211> 28
   <212> DNA
   <213> Artificial Sequence
<220>
   <223> Description of Artificial Sequence: Synthetic primer
<400> 261
   cagctagtcc atttgttctc agactgtg 28
<210> 262
   <211> 25
   <212> DNA
   <213> Artificial Sequence
<220>
   <223> Description of Artificial Sequence: Synthetic primer
<400> 262
   ggccaacatt gtgaaaccct gtctc 25
<210> 263
   <211> 22
   <212> DNA
   <213> Artificial Sequence
<220>
   <223> Description of Artificial Sequence: Synthetic primer
<400> 263
   ccagggacct gtgcttgggt tc 22
<210> 264
   <211> 23
   <212> DNA
   <213> Artificial Sequence
<220>
   <223> Description of Artificial Sequence: Synthetic primer
<400> 264
   caccccatga cctggcacaa gtg 23
<210> 265
   <211> 24
   <212> DNA
   <213> Artificial Sequence
<220>
   <223> Description of Artificial Sequence: Synthetic primer
<400> 265
   aagtgttcct cagaatgcca gccc 24
<210> 266
   <211> 23
   <212> DNA
   <213> Artificial Sequence
<220>
   <223> Description of Artificial Sequence: Synthetic primer
<400> 266
   caggagtgca gttgtgttgg gag 23
<210> 267
   <211> 25
   <212> DNA
   <213> Artificial Sequence
<220>
   <223> Description of Artificial Sequence: Synthetic primer
<400> 267
   ctgatgaagc accagagaac ccacc 25
<210> 268
   <211> 21
   <212> DNA
   <213> Artificial Sequence
<220>
   <223> Description of Artificial Sequence: Synthetic primer
<400> 268
   cacacctggc acccatatgg c 21
<210> 269
   <211> 25
   <212> DNA
   <213> Artificial Sequence
<220>
   <223> Description of Artificial Sequence: Synthetic primer
<400> 269
   gatccacact ggtgagaagc cttac 25
<210> 270
   <211> 26
   <212> DNA
   <213> Artificial Sequence
<220>
   <223> Description of Artificial Sequence: Synthetic primer
<400> 270
   cttcccacac tcacagcaga tgtagg 26
<210> 271
   <211> 4206
   <212> DNA
   <213> Artificial Sequence
<220>
   <223> Description of Artificial Sequence: Synthetic polynucleotide
<400> 271
<210> 272
   <211> 4206
   <212> DNA
   <213> Artificial Sequence
<220>
   <223> Description of Artificial Sequence: Synthetic polynucleotide
<400> 272
   atggataaaa agtattctat tggtttagac atcggcacta attccgttgg atgggctgtc      60
   ataaccgatg aatacaaagt accttcaaag aaatttaagg tgttggggaa cacagaccgt     120
   cattcgatta aaaagaatct tatcggtgcc ctcctattcg atagtggcga aacggcagag     180
   gcgactcgcc tgaaacgaac cgctcggaga aggtatacac gtcgcaagaa ccgaatatgt     240
   tacttacaag aaatttttag caatgagatg gccaaagttg acgattcttt ctttcaccgt     300
   ttggaagagt ccttccttgt cgaagaggac aagaaacatg aacggcaccc catctttgga     360
   aacatagtag atgaggtggc atatcatgaa aagtacccaa cgatttatca cctcagaaaa     420
   aagctagttg actcaactga taaagcggac ctgaggttaa tctacttggc tcttgcccat     480
   atgataaagt tccgtgggca ctttctcatt gagggtgatc taaatccgga caactcggat     540
   gtcgacaaac tgttcatcca gttagtacaa acctataatc agttgtttga agagaaccct     600
   ataaatgcaa gtggcgtgga tgcgaaggct attcttagcg cccgcctctc taaatcccga     660
   cggctagaaa acctgatcgc acaattaccc ggagagaaga aaaatgggtt gttcggtaac     720
   cttatagcgc tctcactagg cctgacacca aattttaagt cgaacttcga cttagctgaa     780
   gatgccaaat tgcagcttag taaggacacg tacgatgacg atctcgacaa tctactggca     840
   caaattggag atcagtatgc ggacttattt ttggctgcca aaaaccttag cgatgcaatc     900
   ctcctatctg acatactgag agttaatact gagattacca aggcgccgtt atccgcttca     960
   atgatcaaaa ggtacgatga acatcaccaa gacttgacac ttctcaaggc cctagtccgt    1020
   cagcaactgc ctgagaaata taaggaaata ttctttgatc agtcgaaaaa cgggtacgca    1080
   ggttatattg acggcggagc gagtcaagag gaattctaca agtttatcaa acccatatta    1140
   gagaagatgg atgggacgga agagttgctt gtaaaactca atcgcgaaga tctactgcga    1200
   aagcagcgga ctttcgacaa cggtagcatt ccacatcaaa tccacttagg cgaattgcat    1260
   gctatactta gaaggcagga ggatttttat ccgttcctca aagacaatcg tgaaaagatt    1320
   gagaaaatcc taacctttcg cataccttac tatgtgggac ccctggcccg agggaactct 1380
   cggttcgcat ggatgacaag aaagtccgaa gaaacgatta ctccctggaa ttttgaggaa 1440
   gttgtcgata aaggtgcgtc agctcaatcg ttcatcgaga ggatgaccgc ctttgacaag 1500
   aatttaccga acgaaaaagt attgcctaag cacagtttac tttacgagta tttcacagtg 1560
   tacaatgaac tcacgaaagt taagtatgtc actgagggca tgcgtaaacc cgcctttcta 1620
   agcggagaac agaagaaagc aatagtagat ctgttattca agaccaaccg caaagtgaca 1680
   gttaagcaat tgaaagagga ctactttaag aaaattgaat gcttcgattc tgtcgagatc 1740
   tccggggtag aagatcgatt taatgcgtca cttggtacgt atcatgacct cctaaagata 1800
   attaaagata aggacttcct ggataacgaa gagaatgaag atatcttaga agatatagtg 1860
   ttgactctta ccctctttga agatcgggaa atgattgagg aaagactaaa aacatacgct 1920
   cacctgttcg acgataaggt tatgaaacag ttaaagaggc gtcgctatac gggctgggga 1980
   gccttgtcgc ggaaacttat caacgggata agagacaagc aaagtggtaa aactattctc 2040
   gattttctaa agagcgacgg cttcgccaat aggaacttta tggccctgat ccatgatgac 2100
   tctttaacct tcaaagagga tatacaaaag gcacaggttt ccggacaagg ggactcattg 2160
   cacgaacata ttgcgaatct tgctggttcg ccagccatca aaaagggcat actccagaca 2220
   gtcaaagtag tggatgagct agttaaggtc atgggacgtc acaaaccgga aaacattgta 2280
   atcgagatgg cacgcgaaaa tcaaacgact cagaaggggc aaaaaaacag tcgagagcgg 2340
   atgaagagaa tagaagaggg tattaaagaa ctgggcagcc agatcttaaa ggagcatcct 2400
   gtggaaaata cccaattgca gaacgagaaa ctttacctct attacctaca aaatggaagg 2460
   gacatgtatg ttgatcagga actggacata aaccgtttat ctgattacga cgtcgatcac 2520
   attgtacccc aatccttttt gaaggacgat tcaatcgaca ataaagtgct tacacgctcg 2580
   gataagaacc gagggaaaag tgacaatgtt ccaagcgagg aagtcgtaaa gaaaatgaag 2640
   aactattggc ggcagctcct aaatgcgaaa ctgataacgc aaagaaagtt cgataactta 2700
   actaaagctg agaggggtgg cttgtctgaa cttgacaagg ccggatttat taaacgtcag 2760
   ctcgtggaaa cccgcgccat cacaaagcat gttgcgcaga tactagattc ccgaatgaat 2820
   acgaaatacg acgagaacga taagctgatt cgggaagtca aagtaatcac tttaaagtca 2880
   aaattggtgt cggacttcag aaaggatttt caattctata aagttaggga gataaataac 2940
   taccaccatg cgcacgacgc ttatcttaat gccgtcgtag ggaccgcact cattaagaaa 3000
   tacccgaagc tagaaagtga gtttgtgtat ggtgattaca aagtttatga cgtccgtaag 3060
   atgatcgcga aaagcgaaca ggagataggc aaggctacag ccaaatactt cttttattct 3120
   aacattatga atttctttaa gacggaaatc actctggcaa acggagagat acgcaaacga 3180
   cctttaattg aaaccaatgg ggagacaggt gaaatcgtat gggataaggg ccgggacttc 3240
   gcgacggtga gaaaagtttt gtccatgccc caagtcaaca tagtaaagaa aactgaggtg 3300
   cagaccggag ggttttcaaa ggaatcgatt cttccaaaaa ggaatagtga taagctcatc 3360
   gctcgtaaaa aggactggga cccgaaaaag tacggtggct tcgatagccc tacagttgcc 3420
   tattctgtcc tagtagtggc aaaagttgag aagggaaaat ccaagaaact gaagtcagtc 3480
   aaagaattat tggggataac gattatggag cgctcgtctt ttgaaaagaa ccccatcgac 3540
   ttccttgagg cgaaaggtta caaggaagta aaaaaggatc tcataattaa actaccaaag 3600
   tatagtctgt ttgagttaga aaatggccga aaacggatgt tggctagcgc cggagagctt 3660
   caaaagggga acgaactcgc actaccgtct aaatacgtga atttcctgta tttagcgtcc 3720
   cattacgaga agttgaaagg ttcacctgaa gataacgaac agaagcaact ttttgttgag 3780
   cagcacaaac attatctcga cgaaatcata gagcaaattt cggaattcag taagagagtc 3840
   atcctagctg atgccaatct ggacaaagta ttaagcgcat acaacaagca cagggataaa 3900
   cccatacgtg agcaggcgga aaatattatc catttgttta ctcttaccaa cctcggcgct 3960
   ccagccgcat tcaagtattt tgacacaacg atagatcgca aacgatacac ttctaccaag 4020
   gaggtgctag acgcgacact gattcaccaa tccatcacgg gattatatga aactcggata 4080
   gatttgtcac agcttggggg tgacggatcc cccaagaaga agaggaaagt ctcgagcgac 4140
   tacaaagacc atgacggtga ttataaagat catgacatcg attacaagga tgacgatgac 4200
   aagtga 4206
<210> 273
   <211> 4206
   <212> DNA
   <213> Artificial Sequence
<220>
   <223> Description of Artificial Sequence: Synthetic polynucleotide
<400> 273
   atggataaaa agtattctat tggtttagac atcggcacta attccgttgg atgggctgtc       60
   ataaccgatg aatacaaagt accttcaaag aaatttaagg tgttggggaa cacagaccgt      120
   cattcgatta aaaagaatct tatcggtgcc ctcctattcg atagtggcga aacggcagag      180
   gcgactcgcc tgaaacgaac cgctcggaga aggtatacac gtcgcaagaa ccgaatatgt      240
   tacttacaag aaatttttag caatgagatg gccaaagttg acgattcttt ctttcaccgt      300
   ttggaagagt ccttccttgt cgaagaggac aagaaacatg aacggcaccc catctttgga      360
   aacatagtag atgaggtggc atatcatgaa aagtacccaa cgatttatca cctcagaaaa      420
   aagctagttg actcaactga taaagcggac ctgaggttaa tctacttggc tcttgcccat      480
   atgataaagt tccgtgggca ctttctcatt gagggtgatc taaatccgga caactcggat 540
   gtcgacaaac tgttcatcca gttagtacaa acctataatc agttgtttga agagaaccct 600
   ataaatgcaa gtggcgtgga tgcgaaggct attcttagcg cccgcctctc taaatcccga 660
   cggctagaaa acctgatcgc acaattaccc ggagagaaga aaaatgggtt gttcggtaac 720
   cttatagcgc tctcactagg cctgacacca aattttaagt cgaacttcga cttagctgaa 780
   gatgccaaat tgcagcttag taaggacacg tacgatgacg atctcgacaa tctactggca 840
   caaattggag atcagtatgc ggacttattt ttggctgcca aaaaccttag cgatgcaatc 900
   ctcctatctg acatactgag agttaatact gagattacca aggcgccgtt atccgcttca 960
   atgatcaaaa ggtacgatga acatcaccaa gacttgacac ttctcaaggc cctagtccgt 1020
   cagcaactgc ctgagaaata taaggaaata ttctttgatc agtcgaaaaa cgggtacgca 1080
   ggttatattg acggcggagc gagtcaagag gaattctaca agtttatcaa acccatatta 1140
   gagaagatgg atgggacgga agagttgctt gtaaaactca atcgcgaaga tctactgcga 1200
   aagcagcgga ctttcgacaa cggtagcatt ccacatcaaa tccacttagg cgaattgcat 1260
   gctatactta gaaggcagga ggatttttat ccgttcctca aagacaatcg tgaaaagatt 1320
   gagaaaatcc taacctttcg cataccttac tatgtgggac ccctggcccg agggaactct 1380
   cggttcgcat ggatgacaag aaagtccgaa gaaacgatta ctccctggaa ttttgaggaa 1440
   gttgtcgata aaggtgcgtc agctcaatcg ttcatcgaga ggatgaccgc ctttgacaag 1500
   aatttaccga acgaaaaagt attgcctaag cacagtttac tttacgagta tttcacagtg 1560
   tacaatgaac tcacgaaagt taagtatgtc actgagggca tgcgtaaacc cgcctttcta 1620
   agcggagaac agaagaaagc aatagtagat ctgttattca agaccaaccg caaagtgaca 1680
   gttaagcaat tgaaagagga ctactttaag aaaattgaat gcttcgattc tgtcgagatc 1740
   tccggggtag aagatcgatt taatgcgtca cttggtacgt atcatgacct cctaaagata 1800
   attaaagata aggacttcct ggataacgaa gagaatgaag atatcttaga agatatagtg 1860
   ttgactctta ccctctttga agatcgggaa atgattgagg aaagactaaa aacatacgct 1920
   cacctgttcg acgataaggt tatgaaacag ttaaagaggc gtcgctatac gggctgggga 1980
   gccttgtcgc ggaaacttat caacgggata agagacaagc aaagtggtaa aactattctc 2040
   gattttctaa agagcgacgg cttcgccaat aggaacttta tggccctgat ccatgatgac 2100
   tctttaacct tcaaagagga tatacaaaag gcacaggttt ccggacaagg ggactcattg 2160
   cacgaacata ttgcgaatct tgctggttcg ccagccatca aaaagggcat actccagaca 2220
   gtcaaagtag tggatgagct agttaaggtc atgggacgtc acaaaccgga aaacattgta 2280
   atcgagatgg cacgcgaaaa tcaaacgact cagaaggggc aaaaaaacag tcgagagcgg 2340
   atgaagagaa tagaagaggg tattaaagaa ctgggcagcc agatcttaaa ggagcatcct 2400
   gtggaaaata cccaattgca gaacgagaaa ctttacctct attacctaca aaatggaagg 2460
   gacatgtatg ttgatcagga actggacata aaccgtttat ctgattacga cgtcgatcac 2520
   attgtacccc aatccttttt gaaggacgat tcaatcgaca ataaagtgct tacacgctcg 2580
   gataagaacc gagggaaaag tgacaatgtt ccaagcgagg aagtcgtaaa gaaaatgaag 2640
   aactattggc ggcagctcct aaatgcgaaa ctgataacgc aaagaaagtt cgataactta 2700
   actaaagctg agaggggtgg cttgtctgaa cttgacaagg ccggatttat taaacgtcag 2760
   ctcgtggaaa cccgcgccat cacaaagcat gttgcgcaga tactagattc ccgaatgaat 2820
   acgaaatacg acgagaacga taagctgatt cgggaagtca aagtaatcac tttaaagtca 2880
   aaattggtgt cggacttcag aaaggatttt caattctata aagttaggga gataaataac 2940
   taccaccatg cgcacgacgc ttatcttaat gccgtcgtag ggaccgcact cattaagaaa 3000
   tacccgaagc tagaaagtga gtttgtgtat ggtgattaca aagtttatga cgtccgtaag 3060
   atgatcgcga aaagcgaaca ggagataggc aaggctacag ccaaatactt cttttattct 3120
   aacattatga atttctttaa gacggaaatc actctggcaa acggagagat acgcaaacga 3180
   cctttaattg aaaccaatgg ggagacaggt gaaatcgtat gggataaggg ccgggacttc 3240
   gcgacggtga gaaaagtttt gtccatgccc caagtcaaca tagtaaagaa aactgaggtg 3300
   cagaccggag ggttttcaaa ggaatcgatt cttccaaaaa ggaatagtga taagctcatc 3360
   gctcgtaaaa aggactggga cccgaaaaag tacggtggct tcgagagccc tacagttgcc 3420
   tattctgtcc tagtagtggc aaaagttgag aagggaaaat ccaagaaact gaagtcagtc 3480
   aaagaattat tggggataac gattatggag cgctcgtctt ttgaaaagaa ccccatcgac 3540
   ttccttgagg cgaaaggtta caaggaagta aaaaaggatc tcataattaa actaccaaag 3600
   tatagtctgt ttgagttaga aaatggccga aaacggatgt tggctagcgc cggagagctt 3660
   caaaagggga acgaactcgc actaccgtct aaatacgtga atttcctgta tttagcgtcc 3720
   cattacgaga agttgaaagg ttcacctgaa gataacgaac agaagcaact tttgttggag 3780
   cagcacaaac attatctcga cgaaatcata gagcaaattt cggaattcag taagagagtc 3840
   atcctagctg atgccaatct ggacaaagta ttaagcgcat acaacaagca cagggataaa 3900
   cccatacgtg agcaggcgga aaatattatc catttgttta ctcttaccaa cctcggcgct 3960
   ccagccgcat tcaagtattt tgacacaacg atagatcgca aacgatacac ttctaccaag 4020
   gaggtgctag acgcgacact gattcaccaa tccatcacgg gattatatga aactcggata 4080
   gatttgtcac agcttggggg tgacggatcc cccaagaaga agaggaaagt ctcgagcgac 4140
   tacaaagacc atgacggtga ttataaagat catgacatcg attacaagga tgacgatgac 4200
   aagtga 4206
<210> 274
   <211> 4206
   <212> DNA
   <213> Artificial Sequence
<220>
   <223> Description of Artificial Sequence: Synthetic polynucleotide
<400> 274
   atggataaaa agtattctat tggtttagac atcggcacta attccgttgg atgggctgtc      60
   ataaccgatg aatacaaagt accttcaaag aaatttaagg tgttggggaa cacagaccgt     120
   cattcgatta aaaagaatct tatcggtgcc ctcctattcg atagtggcga aacggcagag     180
   gcgactcgcc tgaaacgaac cgctcggaga aggtatacac gtcgcaagaa ccgaatatgt     240
   tacttacaag aaatttttag caatgagatg gccaaagttg acgattcttt ctttcaccgt     300
   ttggaagagt ccttccttgt cgaagaggac aagaaacatg aacggcaccc catctttgga     360
   aacatagtag atgaggtggc atatcatgaa aagtacccaa cgatttatca cctcagaaaa     420
   aagctagttg actcaactga taaagcggac ctgaggttaa tctacttggc tcttgcccat     480
   atgataaagt tccgtgggca ctttctcatt gagggtgatc taaatccgga caactcggat     540
   gtcgacaaac tgttcatcca gttagtacaa acctataatc agttgtttga agagaaccct     600
   ataaatgcaa gtggcgtgga tgcgaaggct attcttagcg cccgcctctc taaatcccga     660
   cggctagaaa acctgatcgc acaattaccc ggagagaaga aaaatgggtt gttcggtaac     720
   cttatagcgc tctcactagg cctgacacca aattttaagt cgaacttcga cttagctgaa     780
   gatgccaaat tgcagcttag taaggacacg tacgatgacg atctcgacaa tctactggca     840
   caaattggag atcagtatgc ggacttattt ttggctgcca aaaaccttag cgatgcaatc     900
   ctcctatctg acatactgag agttaatact gagattacca aggcgccgtt atccgcttca     960
   atgatcaaaa ggtacgatga acatcaccaa gacttgacac ttctcaaggc cctagtccgt    1020
   cagcaactgc ctgagaaata taaggaaata ttctttgatc agtcgaaaaa cgggtacgca    1080
   ggttatattg acggcggagc gagtcaagag gaattctaca agtttatcaa acccatatta    1140
   gagaagatgg atgggacgga agagttgctt gtaaaactca atcgcgaaga tctactgcga    1200
   aagcagcgga ctttcgacaa cggtagcatt ccacatcaaa tccacttagg cgaattgcat    1260
   gctatactta gaaggcagga ggatttttat ccgttcctca aagacaatcg tgaaaagatt    1320
   gagaaaatcc taacctttcg catacctgcc tatgtgggac ccctggcccg agggaactct    1380
   cggttcgcat ggatgacaag aaagtccgaa gaaacgatta ctccctggaa ttttgaggaa    1440
   gttgtcgata aaggtgcgtc agctcaatcg ttcatcgaga ggatgaccgc ctttgacaag    1500
   aatttaccga acgaaaaagt attgcctaag cacagtttac tttacgagta tttcacagtg    1560
   tacaatgaac tcacgaaagt taagtatgtc actgagggca tgcgtaaacc cgcctttcta 1620
   agcggagaac agaagaaagc aatagtagat ctgttattca agaccaaccg caaagtgaca 1680
   gttaagcaat tgaaagagga ctactttaag aaaattgaat gcttcgattc tgtcgagatc 1740
   tccggggtag aagatcgatt taatgcgtca cttggtacgt atcatgacct cctaaagata 1800
   attaaagata aggacttcct ggataacgaa gagaatgaag atatcttaga agatatagtg 1860
   ttgactctta ccctctttga agatcgggaa atgattgagg aaagactaaa aacatacgct 1920
   cacctgttcg acgataaggt tatgaaacag ttaaagaggc gtcgctatac gggctgggga 1980
   gccttgtcgc ggaaacttat caacgggata agagacaagc aaagtggtaa aactattctc 2040
   gattttctaa agagcgacgg cttcgccaat aggaacttta tggccctgat ccatgatgac 2100
   tctttaacct tcaaagagga tatacaaaag gcacaggttt ccggacaagg ggactcattg 2160
   cacgaacata ttgcgaatct tgctggttcg ccagccatca aaaagggcat actccagaca 2220
   gtcaaagtag tggatgagct agttaaggtc atgggacgtc acaaaccgga aaacattgta 2280
   atcgagatgg cacgcgaaaa tcaaacgact cagaaggggc aaaaaaacag tcgagagcgg 2340
   atgaagagaa tagaagaggg tattaaagaa ctgggcagcc agatcttaaa ggagcatcct 2400
   gtggaaaata cccaattgca gaacgagaaa ctttacctct attacctaca aaatggaagg 2460
   gacatgtatg ttgatcagga actggacata aaccgtttat ctgattacga cgtcgatcac 2520
   attgtacccc aatccttttt gaaggacgat tcaatcgaca ataaagtgct tacacgctcg 2580
   gataagaacc gagggaaaag tgacaatgtt ccaagcgagg aagtcgtaaa gaaaatgaag 2640
   aactattggc ggcagctcct aaatgcgaaa ctgataacgc aaagaaagtt cgataactta 2700
   actaaagctg agaggggtgg cttgtctgaa cttgacaagg ccggatttat taaacgtcag 2760
   ctcgtggaaa cccgcgccat cacaaagcat gttgcgcaga tactagattc ccgaatgaat 2820
   acgaaatacg acgagaacga taagctgatt cgggaagtca aagtaatcac tttaaagtca 2880
   aaattggtgt cggacttcag aaaggatttt caattctata aagttaggga gataaataac 2940
   taccaccatg cgcacgacgc ttatcttaat gccgtcgtag ggaccgcact cattaagaaa 3000
   tacccgaagc tagaaagtga gtttgtgtat ggtgattaca aagtttatga cgtccgtaag 3060
   atgatcgcga aaagcgaaca ggagataggc aaggctacag ccaaatactt cttttattct 3120
   aacattatga atttctttaa gacggaaatc actctggcaa acggagagat acgcaaacga 3180
   cctttaattg aaaccaatgg ggagacaggt gaaatcgtat gggataaggg ccgggacttc 3240
   gcgacggtga gaaaagtttt gtccatgccc caagtcaaca tagtaaagaa aactgaggtg 3300
   cagaccggag ggttttcaaa ggaatcgatt cttccaaaaa ggaatagtga taagctcatc 3360
   gctcgtaaaa aggactggga cccgaaaaag tacggtggct tcgatagccc tacagttgcc 3420
   tattctgtcc tagtagtggc aaaagttgag aagggaaaat ccaagaaact gaagtcagtc 3480
   aaagaattat tggggataac gattatggag cgctcgtctt ttgaaaagaa ccccatcgac 3540
   ttccttgagg cgaaaggtta caaggaagta aaaaaggatc tcataattaa actaccaaag 3600
   tatagtctgt ttgagttaga aaatggccga aaacggatgt tggctagcgc cggagagctt 3660
   caaaagggga acgaactcgc actaccgtct aaatacgtga atttcctgta tttagcgtcc 3720
   cattacgaga agttgaaagg ttcacctgaa gataacgaac agaagcaact ttttgttgag 3780
   cagcacaaac attatctcga cgaaatcata gagcaaattt cggaattcag taagagagtc 3840
   atcctagctg atgccaatct ggacaaagta ttaagcgcat acaacaagca cagggataaa 3900
   cccatacgtg agcaggcgga aaatattatc catttgttta ctcttaccaa cctcggcgct 3960
   ccagccgcat tcaagtattt tgacacaacg atagatcgca aacgatacac ttctaccaag 4020
   gaggtgctag acgcgacact gattcaccaa tccatcacgg gattatatga aactcggata 4080
   gatttgtcac agcttggggg tgacggatcc cccaagaaga agaggaaagt ctcgagcgac 4140
   tacaaagacc atgacggtga ttataaagat catgacatcg attacaagga tgacgatgac 4200
   aagtga 4206
<210> 275
   <211> 4206
   <212> DNA
   <213> Artificial Sequence
<220>
   <223> Description of Artificial Sequence: Synthetic polynucleotide
<400> 275
   atggataaaa agtattctat tggtttagac atcggcacta attccgttgg atgggctgtc      60
   ataaccgatg aatacaaagt accttcaaag aaatttaagg tgttggggaa cacagaccgt     120
   cattcgatta aaaagaatct tatcggtgcc ctcctattcg atagtggcga aacggcagag     180
   gcgactcgcc tgaaacgaac cgctcggaga aggtatacac gtcgcaagaa ccgaatatgt     240
   tacttacaag aaatttttag caatgagatg gccaaagttg acgattcttt ctttcaccgt     300
   ttggaagagt ccttccttgt cgaagaggac aagaaacatg aacggcaccc catctttgga     360
   aacatagtag atgaggtggc atatcatgaa aagtacccaa cgatttatca cctcagaaaa     420
   aagctagttg actcaactga taaagcggac ctgaggttaa tctacttggc tcttgcccat     480
   atgataaagt tccgtgggca ctttctcatt gagggtgatc taaatccgga caactcggat     540
   gtcgacaaac tgttcatcca gttagtacaa acctataatc agttgtttga agagaaccct     600
   ataaatgcaa gtggcgtgga tgcgaaggct attcttagcg cccgcctctc taaatcccga     660
   cggctagaaa acctgatcgc acaattaccc ggagagaaga aaaatgggtt gttcggtaac     720
   cttatagcgc tctcactagg cctgacacca aattttaagt cgaacttcga cttagctgaa     780
   gatgccaaat tgcagcttag taaggacacg tacgatgacg atctcgacaa tctactggca 840
   caaattggag atcagtatgc ggacttattt ttggctgcca aaaaccttag cgatgcaatc 900
   ctcctatctg acatactgag agttaatact gagattacca aggcgccgtt atccgcttca 960
   atgatcaaaa ggtacgatga acatcaccaa gacttgacac ttctcaaggc cctagtccgt 1020
   cagcaactgc ctgagaaata taaggaaata ttctttgatc agtcgaaaaa cgggtacgca 1080
   ggttatattg acggcggagc gagtcaagag gaattctaca agtttatcaa acccatatta 1140
   gagaagatgg atgggacgga agagttgctt gtaaaactca atcgcgaaga tctactgcga 1200
   aagcagcgga ctttcgacaa cggtagcatt ccacatcaaa tccacttagg cgaattgcat 1260
   gctatactta gaaggcagga ggatttttat ccgttcctca aagacaatcg tgaaaagatt 1320
   gagaaaatcc taacctttcg cataccttac tatgtgggac ccctggcccg agggaactct 1380
   cggttcgcat ggatgacaag aaagtccgaa gaaacgatta ctccatggaa ttttgaggaa 1440
   gttgtcgata aaggtgcgtc agctcaatcg ttcatcgaga ggatgaccaa ctttgacaag 1500
   aatttaccga acgaaaaagt attgcctaag cacagtttac tttacgagta tttcacagtg 1560
   tacaatgaac tcacgaaagt taagtatgtc actgagggca tgcgtaaacc cgcctttcta 1620
   agcggagaac agaagaaagc aatagtagat ctgttattca agaccaaccg caaagtgaca 1680
   gttaagcaat tgaaagagga ctactttaag aaaattgaat gcttcgattc tgtcgagatc 1740
   tccggggtag aagatcgatt taatgcgtca cttggtacgt atcatgacct cctaaagata 1800
   attaaagata aggacttcct ggataacgaa gagaatgaag atatcttaga agatatagtg 1860
   ttgactctta ccctctttga agatcgggaa atgattgagg aaagactaaa aacatacgct 1920
   cacctgttcg acgataaggt tatgaaacag ttaaagaggc gtcgctatac gggctgggga 1980
   cgattgtcgc ggaaacttat caacgggata agagacaagc aaagtggtaa aactattctc 2040
   gattttctaa agagcgacgg cttcgccaat aggaacttta tgcagctgat ccatgatgac 2100
   tctttaacct tcaaagagga tatacaaaag gcacaggttt ccggacaagg ggactcattg 2160
   cacgaacata ttgcgaatct tgctggttcg ccagccatca aaaagggcat actccagaca 2220
   gtcaaagtag tggatgagct agttaaggtc atgggacgtc acaaaccgga aaacattgta 2280
   atcgagatgg cacgcgaaaa tcaaacgact cagaaggggc aaaaaaacag tcgagagcgg 2340
   atgaagagaa tagaagaggg tattaaagaa ctgggcagcc agatcttaaa ggagcatcct 2400
   gtggaaaata cccaattgca gaacgagaaa ctttacctct attacctaca aaatggaagg 2460
   gacatgtatg ttgatcagga actggacata aaccgtttat ctgattacga cgtcgatcac 2520
   attgtacccc aatccttttt gaaggacgat tcaatcgaca ataaagtgct tacacgctcg 2580
   gataagaacc gagggaaaag tgacaatgtt ccaagcgagg aagtcgtaaa gaaaatgaag 2640
   aactattggc ggcagctcct aaatgcgaaa ctgataacgc aaagaaagtt cgataactta 2700
   actaaagctg agaggggtgg cttgtctgaa cttgacaagg ccggatttat taaacgtcag 2760
   ctcgtggaaa cccgccaaat cacaaagcat gttgcacaga tactagattc ccgaatgaat 2820
   acgaaatacg acgagaacga taagctgatt cgggaagtca aagtaatcac tttaaagtca 2880
   aaattggtgt cggacttcag aaaggatttt caattctata aagttaggga gataaataac 2940
   taccaccatg cgcacgacgc ttatcttaat gccgtcgtag ggaccgcact cattaagaaa 3000
   tacccgaagc tagaaagtga gtttgtgtat ggtgattaca aagtttatga cgtccgtaag 3060
   atgatcgcga aaagcgaaca ggagataggc aaggctacag ccaaatactt cttttattct 3120
   aacattatga atttctttaa gacggaaatc actctggcaa acggagagat acgcaaacga 3180
   cctttaattg aaaccaatgg ggagacaggt gaaatcgtat gggataaggg ccgggacttc 3240
   gcgacggtga gaaaagtttt gtccatgccc caagtcaaca tagtaaagaa aactgaggtg 3300
   cagaccggag ggttttcaaa ggaatcgatt cttccaaaaa ggaatagtga taagctcatc 3360
   gctcgtaaaa aggactggga cccgaaaaag tacggtggct tcgtgagccc tacagttgcc 3420
   tattctgtcc tagtagtggc aaaagttgag aagggaaaat ccaagaaact gaagtcagtc 3480
   aaagaattat tggggataac gattatggag cgctcgtctt ttgaaaagaa ccccatcgac 3540
   ttccttgagg cgaaaggtta caaggaagta aaaaaggatc tcataattaa actaccaaag 3600
   tatagtctgt ttgagttaga aaatggccga aaacggatgt tggctagcgc cggagagctt 3660
   caaaagggga acgaactcgc actaccgtct aaatacgtga atttcctgta tttagcgtcc 3720
   cattacgaga agttgaaagg ttcacctgaa gataacgaac agaagcaact ttttgttgag 3780
   cagcacaaac attatctcga cgaaatcata gagcaaattt cggaattcag taagagagtc 3840
   atcctagctg atgccaatct ggacaaagta ttaagcgcat acaacaagca cagggataaa 3900
   cccatacgtg agcaggcgga aaatattatc catttgttta ctcttaccaa cctcggcgct 3960
   ccagccgcat tcaagtattt tgacacaacg atagatcgca aacagtacag atctaccaag 4020
   gaggtgctag acgcgacact gattcaccaa tccatcacgg gattatatga aactcggata 4080
   gatttgtcac agcttggggg tgacggatcc cccaagaaga agaggaaagt ctcgagcgac 4140
   tacaaagacc atgacggtga ttataaagat catgacatcg attacaagga tgacgatgac 4200
   aagtga 4206
<210> 276
   <211> 4206
   <212> DNA
   <213> Artificial Sequence
<220>
   <223> Description of Artificial Sequence: Synthetic polynucleotide
<400> 276
   atggataaaa agtattctat tggtttagac atcggcacta attccgttgg atgggctgtc 60
   ataaccgatg aatacaaagt accttcaaag aaatttaagg tgttggggaa cacagaccgt 120
   cattcgatta aaaagaatct tatcggtgcc ctcctattcg atagtggcga aacggcagag 180
   gcgactcgcc tgaaacgaac cgctcggaga aggtatacac gtcgcaagaa ccgaatatgt 240
   tacttacaag aaatttttag caatgagatg gccaaagttg acgattcttt ctttcaccgt 300
   ttggaagagt ccttccttgt cgaagaggac aagaaacatg aacggcaccc catctttgga 360
   aacatagtag atgaggtggc atatcatgaa aagtacccaa cgatttatca cctcagaaaa 420
   aagctagttg actcaactga taaagcggac ctgaggttaa tctacttggc tcttgcccat 480
   atgataaagt tccgtgggca ctttctcatt gagggtgatc taaatccgga caactcggat 540
   gtcgacaaac tgttcatcca gttagtacaa acctataatc agttgtttga agagaaccct 600
   ataaatgcaa gtggcgtgga tgcgaaggct attcttagcg cccgcctctc taaatcccga 660
   cggctagaaa acctgatcgc acaattaccc ggagagaaga aaaatgggtt gttcggtaac 720
   cttatagcgc tctcactagg cctgacacca aattttaagt cgaacttcga cttagctgaa 780
   gatgccaaat tgcagcttag taaggacacg tacgatgacg atctcgacaa tctactggca 840
   caaattggag atcagtatgc ggacttattt ttggctgcca aaaaccttag cgatgcaatc 900
   ctcctatctg acatactgag agttaatact gagattacca aggcgccgtt atccgcttca 960
   atgatcaaaa ggtacgatga acatcaccaa gacttgacac ttctcaaggc cctagtccgt 1020
   cagcaactgc ctgagaaata taaggaaata ttctttgatc agtcgaaaaa cgggtacgca 1080
   ggttatattg acggcggagc gagtcaagag gaattctaca agtttatcaa acccatatta 1140
   gagaagatgg atgggacgga agagttgctt gtaaaactca atcgcgaaga tctactgcga 1200
   aagcagcgga ctttcgacaa cggtagcatt ccacatcaaa tccacttagg cgaattgcat 1260
   gctatactta gaaggcagga ggatttttat ccgttcctca aagacaatcg tgaaaagatt 1320
   gagaaaatcc taacctttcg cataccttac tatgtgggac ccctggcccg agggaactct 1380
   cggttcgcat ggatgacaag aaagtccgaa gaaacgatta ctccctggaa ttttgaggaa 1440
   gttgtcgata aaggtgcgtc agctcaatcg ttcatcgaga ggatgaccgc ctttgacaag 1500
   aatttaccga acgaaaaagt attgcctaag cacagtttac tttacgagta tttcacagtg 1560
   tacaatgaac tcacgaaagt taagtatgtc actgagggca tgcgtaaacc cgcctttcta 1620
   agcggagaac agaagaaagc aatagtagat ctgttattca agaccaaccg caaagtgaca 1680
   gttaagcaat tgaaagagga ctactttaag aaaattgaat gcttcgattc tgtcgagatc 1740
   tccggggtag aagatcgatt taatgcgtca cttggtacgt atcatgacct cctaaagata 1800
   attaaagata aggacttcct ggataacgaa gagaatgaag atatcttaga agatatagtg 1860
   ttgactctta ccctctttga agatcgggaa atgattgagg aaagactaaa aacatacgct 1920
   cacctgttcg acgataaggt tatgaaacag ttaaagaggc gtcgctatac gggctgggga 1980
   gccttgtcgc ggaaacttat caacgggata agagacaagc aaagtggtaa aactattctc 2040
   gattttctaa agagcgacgg cttcgccaat aggaacttta tggccctgat ccatgatgac 2100
   tctttaacct tcaaagagga tatacaaaag gcacaggttt ccggacaagg ggactcattg 2160
   cacgaacata ttgcgaatct tgctggttcg ccagccatca aaaagggcat actccagaca 2220
   gtcaaagtag tggatgagct agttaaggtc atgggacgtc acaaaccgga aaacattgta 2280
   atcgagatgg cacgcgaaaa tcaaacgact cagaaggggc aaaaaaacag tcgagagcgg 2340
   atgaagagaa tagaagaggg tattaaagaa ctgggcagcc agatcttaaa ggagcatcct 2400
   gtggaaaata cccaattgca gaacgagaaa ctttacctct attacctaca aaatggaagg 2460
   gacatgtatg ttgatcagga actggacata aaccgtttat ctgattacga cgtcgatcac 2520
   attgtacccc aatccttttt gaaggacgat tcaatcgaca ataaagtgct tacacgctcg 2580
   gataagaacc gagggaaaag tgacaatgtt ccaagcgagg aagtcgtaaa gaaaatgaag 2640
   aactattggc ggcagctcct aaatgcgaaa ctgataacgc aaagaaagtt cgataactta 2700
   actaaagctg agaggggtgg cttgtctgaa cttgacaagg ccggatttat taaacgtcag 2760
   ctcgtggaaa cccgcgccat cacaaagcat gttgcgcaga tactagattc ccgaatgaat 2820
   acgaaatacg acgagaacga taagctgatt cgggaagtca aagtaatcac tttaaagtca 2880
   aaattggtgt cggacttcag aaaggatttt caattctata aagttaggga gataaataac 2940
   taccaccatg cgcacgacgc ttatcttaat gccgtcgtag ggaccgcact cattaagaaa 3000
   tacccgaagc tagaaagtga gtttgtgtat ggtgattaca aagtttatga cgtccgtaag 3060
   atgatcgcga aaagcgaaca ggagataggc aaggctacag ccaaatactt cttttattct 3120
   aacattatga atttctttaa gacggaaatc actctggcaa acggagagat acgcaaacga 3180
   cctttaattg aaaccaatgg ggagacaggt gaaatcgtat gggataaggg ccgggacttc 3240
   gcgacggtga gaaaagtttt gtccatgccc caagtcaaca tagtaaagaa aactgaggtg 3300
   cagaccggag ggttttcaaa ggaatcgatt cttccaaaaa ggaatagtga taagctcatc 3360
   gctcgtaaaa aggactggga cccgaaaaag tacggtggct tcgtgagccc tacagttgcc 3420
   tattctgtcc tagtagtggc aaaagttgag aagggaaaat ccaagaaact gaagtcagtc 3480
   aaagaattat tggggataac gattatggag cgctcgtctt ttgaaaagaa ccccatcgac 3540
   ttccttgagg cgaaaggtta caaggaagta aaaaaggatc tcataattaa actaccaaag 3600
   tatagtctgt ttgagttaga aaatggccga aaacggatgt tggctagcgc cggagagctt 3660
   caaaagggga acgaactcgc actaccgtct aaatacgtga atttcctgta tttagcgtcc 3720
   cattacgaga agttgaaagg ttcacctgaa gataacgaac agaagcaact ttttgttgag 3780
   cagcacaaac attatctcga cgaaatcata gagcaaattt cggaattcag taagagagtc 3840
   atcctagctg atgccaatct ggacaaagta ttaagcgcat acaacaagca cagggataaa 3900
   cccatacgtg agcaggcgga aaatattatc catttgttta ctcttaccaa cctcggcgct 3960
   ccagccgcat tcaagtattt tgacacaacg atagatcgca aacagtacag atctaccaag 4020
   gaggtgctag acgcgacact gattcaccaa tccatcacgg gattatatga aactcggata 4080
   gatttgtcac agcttggggg tgacggatcc cccaagaaga agaggaaagt ctcgagcgac 4140
   tacaaagacc atgacggtga ttataaagat catgacatcg attacaagga tgacgatgac 4200
   aagtga 4206
<210> 277
   <211> 4206
   <212> DNA
   <213> Artificial Sequence
<220>
   <223> Description of Artificial Sequence: Synthetic polynucleotide
<400> 277
   atggataaaa agtattctat tggtttagac atcggcacta attccgttgg atgggctgtc      60
   ataaccgatg aatacaaagt accttcaaag aaatttaagg tgttggggaa cacagaccgt     120
   cattcgatta aaaagaatct tatcggtgcc ctcctattcg atagtggcga aacggcagag     180
   gcgactcgcc tgaaacgaac cgctcggaga aggtatacac gtcgcaagaa ccgaatatgt     240
   tacttacaag aaatttttag caatgagatg gccaaagttg acgattcttt ctttcaccgt     300
   ttggaagagt ccttccttgt cgaagaggac aagaaacatg aacggcaccc catctttgga     360
   aacatagtag atgaggtggc atatcatgaa aagtacccaa cgatttatca cctcagaaaa     420
   aagctagttg actcaactga taaagcggac ctgaggttaa tctacttggc tcttgcccat     480
   atgataaagt tccgtgggca ctttctcatt gagggtgatc taaatccgga caactcggat     540
   gtcgacaaac tgttcatcca gttagtacaa acctataatc agttgtttga agagaaccct     600
   ataaatgcaa gtggcgtgga tgcgaaggct attcttagcg cccgcctctc taaatcccga     660
   cggctagaaa acctgatcgc acaattaccc ggagagaaga aaaatgggtt gttcggtaac     720
   cttatagcgc tctcactagg cctgacacca aattttaagt cgaacttcga cttagctgaa     780
   gatgccaaat tgcagcttag taaggacacg tacgatgacg atctcgacaa tctactggca     840
   caaattggag atcagtatgc ggacttattt ttggctgcca aaaaccttag cgatgcaatc     900
   ctcctatctg acatactgag agttaatact gagattacca aggcgccgtt atccgcttca     960
   atgatcaaaa ggtacgatga acatcaccaa gacttgacac ttctcaaggc cctagtccgt    1020
   cagcaactgc ctgagaaata taaggaaata ttctttgatc agtcgaaaaa cgggtacgca 1080
   ggttatattg acggcggagc gagtcaagag gaattctaca agtttatcaa acccatatta 1140
   gagaagatgg atgggacgga agagttgctt gtaaaactca atcgcgaaga tctactgcga 1200
   aagcagcgga ctttcgacaa cggtagcatt ccacatcaaa tccacttagg cgaattgcat 1260
   gctatactta gaaggcagga ggatttttat ccgttcctca aagacaatcg tgaaaagatt 1320
   gagaaaatcc taacctttcg cataccttac tatgtgggac ccctggcccg agggaactct 1380
   cggttcgcat ggatgacaag aaagtccgaa gaaacgatta ctccatggaa ttttgaggaa 1440
   gttgtcgata aaggtgcgtc agctcaatcg ttcatcgaga ggatgaccaa ctttgacaag 1500
   aatttaccga acgaaaaagt attgcctaag cacagtttac tttacgagta tttcacagtg 1560
   tacaatgaac tcacgaaagt taagtatgtc actgagggca tgcgtaaacc cgcctttcta 1620
   agcggagaac agaagaaagc aatagtagat ctgttattca agaccaaccg caaagtgaca 1680
   gttaagcaat tgaaagagga ctactttaag aaaattgaat gcttcgattc tgtcgagatc 1740
   tccggggtag aagatcgatt taatgcgtca cttggtacgt atcatgacct cctaaagata 1800
   attaaagata aggacttcct ggataacgaa gagaatgaag atatcttaga agatatagtg 1860
   ttgactctta ccctctttga agatcgggaa atgattgagg aaagactaaa aacatacgct 1920
   cacctgttcg acgataaggt tatgaaacag ttaaagaggc gtcgctatac gggctgggga 1980
   cgattgtcgc ggaaacttat caacgggata agagacaagc aaagtggtaa aactattctc 2040
   gattttctaa agagcgacgg cttcgccaat aggaacttta tgcagctgat ccatgatgac 2100
   tctttaacct tcaaagagga tatacaaaag gcacaggttt ccggacaagg ggactcattg 2160
   cacgaacata ttgcgaatct tgctggttcg ccagccatca aaaagggcat actccagaca 2220
   gtcaaagtag tggatgagct agttaaggtc atgggacgtc acaaaccgga aaacattgta 2280
   atcgagatgg cacgcgaaaa tcaaacgact cagaaggggc aaaaaaacag tcgagagcgg 2340
   atgaagagaa tagaagaggg tattaaagaa ctgggcagcc agatcttaaa ggagcatcct 2400
   gtggaaaata cccaattgca gaacgagaaa ctttacctct attacctaca aaatggaagg 2460
   gacatgtatg ttgatcagga actggacata aaccgtttat ctgattacga cgtcgatcac 2520
   attgtacccc aatccttttt gaaggacgat tcaatcgaca ataaagtgct tacacgctcg 2580
   gataagaacc gagggaaaag tgacaatgtt ccaagcgagg aagtcgtaaa gaaaatgaag 2640
   aactattggc ggcagctcct aaatgcgaaa ctgataacgc aaagaaagtt cgataactta 2700
   actaaagctg agaggggtgg cttgtctgaa cttgacaagg ccggatttat taaacgtcag 2760
   ctcgtggaaa cccgccaaat cacaaagcat gttgcacaga tactagattc ccgaatgaat 2820
   acgaaatacg acgagaacga taagctgatt cgggaagtca aagtaatcac tttaaagtca 2880
   aaattggtgt cggacttcag aaaggatttt caattctata aagttaggga gataaataac 2940
   taccaccatg cgcacgacgc ttatcttaat gccgtcgtag ggaccgcact cattaagaaa 3000
   tacccgaagc tagaaagtga gtttgtgtat ggtgattaca aagtttatga cgtccgtaag 3060
   atgatcgcga aaagcgaaca ggagataggc aaggctacag ccaaatactt cttttattct 3120
   aacattatga atttctttaa gacggaaatc actctggcaa acggagagat acgcaaacga 3180
   cctttaattg aaaccaatgg ggagacaggt gaaatcgtat gggataaggg ccgggacttc 3240
   gcgacggtga gaaaagtttt gtccatgccc caagtcaaca tagtaaagaa aactgaggtg 3300
   cagaccggag ggttttcaaa ggaatcgatt cttccaaaaa ggaatagtga taagctcatc 3360
   gctcgtaaaa aggactggga cccgaaaaag tacggtggct tcgtgagccc tacagttgcc 3420
   tattctgtcc tagtagtggc aaaagttgag aagggaaaat ccaagaaact gaagtcagtc 3480
   aaagaattat tggggataac gattatggag cgctcgtctt ttgaaaagaa ccccatcgac 3540
   ttccttgagg cgaaaggtta caaggaagta aaaaaggatc tcataattaa actaccaaag 3600
   tatagtctgt ttgagttaga aaatggccga aaacggatgt tggctagcgc cagagagctt 3660
   caaaagggga acgaactcgc actaccgtct aaatacgtga atttcctgta tttagcgtcc 3720
   cattacgaga agttgaaagg ttcacctgaa gataacgaac agaagcaact ttttgttgag 3780
   cagcacaaac attatctcga cgaaatcata gagcaaattt cggaattcag taagagagtc 3840
   atcctagctg atgccaatct ggacaaagta ttaagcgcat acaacaagca cagggataaa 3900
   cccatacgtg agcaggcgga aaatattatc catttgttta ctcttaccaa cctcggcgct 3960
   ccagccgcat tcaagtattt tgacacaacg atagatcgca aacagtacag atctaccaag 4020
   gaggtgctag acgcgacact gattcaccaa tccatcacgg gattatatga aactcggata 4080
   gatttgtcac agcttggggg tgacggatcc cccaagaaga agaggaaagt ctcgagcgac 4140
   tacaaagacc atgacggtga ttataaagat catgacatcg attacaagga tgacgatgac 4200
   aagtga 4206
<210> 278
   <211> 4206
   <212> DNA
   <213> Artificial Sequence
<220>
   <223> Description of Artificial Sequence: Synthetic polynucleotide
<400> 278
   atggataaaa agtattctat tggtttagac atcggcacta attccgttgg atgggctgtc 60
   ataaccgatg aatacaaagt accttcaaag aaatttaagg tgttggggaa cacagaccgt 120
   cattcgatta aaaagaatct tatcggtgcc ctcctattcg atagtggcga aacggcagag 180
   gcgactcgcc tgaaacgaac cgctcggaga aggtatacac gtcgcaagaa ccgaatatgt 240
   tacttacaag aaatttttag caatgagatg gccaaagttg acgattcttt ctttcaccgt      300
   ttggaagagt ccttccttgt cgaagaggac aagaaacatg aacggcaccc catctttgga      360
   aacatagtag atgaggtggc atatcatgaa aagtacccaa cgatttatca cctcagaaaa      420
   aagctagttg actcaactga taaagcggac ctgaggttaa tctacttggc tcttgcccat      480
   atgataaagt tccgtgggca ctttctcatt gagggtgatc taaatccgga caactcggat      540
   gtcgacaaac tgttcatcca gttagtacaa acctataatc agttgtttga agagaaccct      600
   ataaatgcaa gtggcgtgga tgcgaaggct attcttagcg cccgcctctc taaatcccga      660
   cggctagaaa acctgatcgc acaattaccc ggagagaaga aaaatgggtt gttcggtaac      720
   cttatagcgc tctcactagg cctgacacca aattttaagt cgaacttcga cttagctgaa      780
   gatgccaaat tgcagcttag taaggacacg tacgatgacg atctcgacaa tctactggca      840
   caaattggag atcagtatgc ggacttattt ttggctgcca aaaaccttag cgatgcaatc      900
   ctcctatctg acatactgag agttaatact gagattacca aggcgccgtt atccgcttca      960
   atgatcaaaa ggtacgatga acatcaccaa gacttgacac ttctcaaggc cctagtccgt     1020
   cagcaactgc ctgagaaata taaggaaata ttctttgatc agtcgaaaaa cgggtacgca     1080
   ggttatattg acggcggagc gagtcaagag gaattctaca agtttatcaa acccatatta     1140
   gagaagatgg atgggacgga agagttgctt gtaaaactca atcgcgaaga tctactgcga     1200
   aagcagcgga ctttcgacaa cggtagcatt ccacatcaaa tccacttagg cgaattgcat     1260
   gctatactta gaaggcagga ggatttttat ccgttcctca aagacaatcg tgaaaagatt     1320
   gagaaaatcc taacctttcg cataccttac tatgtgggac ccctggcccg agggaactct     1380
   cggttcgcat ggatgacaag aaagtccgaa gaaacgatta ctccctggaa ttttgaggaa     1440
   gttgtcgata aaggtgcgtc agctcaatcg ttcatcgaga ggatgaccgc ctttgacaag     1500
   aatttaccga acgaaaaagt attgcctaag cacagtttac tttacgagta tttcacagtg     1560
   tacaatgaac tcacgaaagt taagtatgtc actgagggca tgcgtaaacc cgcctttcta     1620
   agcggagaac agaagaaagc aatagtagat ctgttattca agaccaaccg caaagtgaca     1680
   gttaagcaat tgaaagagga ctactttaag aaaattgaat gcttcgattc tgtcgagatc     1740
   tccggggtag aagatcgatt taatgcgtca cttggtacgt atcatgacct cctaaagata     1800
   attaaagata aggacttcct ggataacgaa gagaatgaag atatcttaga agatatagtg     1860
   ttgactctta ccctctttga agatcgggaa atgattgagg aaagactaaa aacatacgct     1920
   cacctgttcg acgataaggt tatgaaacag ttaaagaggc gtcgctatac gggctgggga     1980
   gccttgtcgc ggaaacttat caacgggata agagacaagc aaagtggtaa aactattctc     2040
   gattttctaa agagcgacgg cttcgccaat aggaacttta tggccctgat ccatgatgac     2100
   tctttaacct tcaaagagga tatacaaaag gcacaggttt ccggacaagg ggactcattg 2160
   cacgaacata ttgcgaatct tgctggttcg ccagccatca aaaagggcat actccagaca 2220
   gtcaaagtag tggatgagct agttaaggtc atgggacgtc acaaaccgga aaacattgta 2280
   atcgagatgg cacgcgaaaa tcaaacgact cagaaggggc aaaaaaacag tcgagagcgg 2340
   atgaagagaa tagaagaggg tattaaagaa ctgggcagcc agatcttaaa ggagcatcct 2400
   gtggaaaata cccaattgca gaacgagaaa ctttacctct attacctaca aaatggaagg 2460
   gacatgtatg ttgatcagga actggacata aaccgtttat ctgattacga cgtcgatcac 2520
   attgtacccc aatccttttt gaaggacgat tcaatcgaca ataaagtgct tacacgctcg 2580
   gataagaacc gagggaaaag tgacaatgtt ccaagcgagg aagtcgtaaa gaaaatgaag 2640
   aactattggc ggcagctcct aaatgcgaaa ctgataacgc aaagaaagtt cgataactta 2700
   actaaagctg agaggggtgg cttgtctgaa cttgacaagg ccggatttat taaacgtcag 2760
   ctcgtggaaa cccgcgccat cacaaagcat gttgcgcaga tactagattc ccgaatgaat 2820
   acgaaatacg acgagaacga taagctgatt cgggaagtca aagtaatcac tttaaagtca 2880
   aaattggtgt cggacttcag aaaggatttt caattctata aagttaggga gataaataac 2940
   taccaccatg cgcacgacgc ttatcttaat gccgtcgtag ggaccgcact cattaagaaa 3000
   tacccgaagc tagaaagtga gtttgtgtat ggtgattaca aagtttatga cgtccgtaag 3060
   atgatcgcga aaagcgaaca ggagataggc aaggctacag ccaaatactt cttttattct 3120
   aacattatga atttctttaa gacggaaatc actctggcaa acggagagat acgcaaacga 3180
   cctttaattg aaaccaatgg ggagacaggt gaaatcgtat gggataaggg ccgggacttc 3240
   gcgacggtga gaaaagtttt gtccatgccc caagtcaaca tagtaaagaa aactgaggtg 3300
   cagaccggag ggttttcaaa ggaatcgatt cttccaaaaa ggaatagtga taagctcatc 3360
   gctcgtaaaa aggactggga cccgaaaaag tacggtggct tcgtgagccc tacagttgcc 3420
   tattctgtcc tagtagtggc aaaagttgag aagggaaaat ccaagaaact gaagtcagtc 3480
   aaagaattat tggggataac gattatggag cgctcgtctt ttgaaaagaa ccccatcgac 3540
   ttccttgagg cgaaaggtta caaggaagta aaaaaggatc tcataattaa actaccaaag 3600
   tatagtctgt ttgagttaga aaatggccga aaacggatgt tggctagcgc cagagagctt 3660
   caaaagggga acgaactcgc actaccgtct aaatacgtga atttcctgta tttagcgtcc 3720
   cattacgaga agttgaaagg ttcacctgaa gataacgaac agaagcaact ttttgttgag 3780
   cagcacaaac attatctcga cgaaatcata gagcaaattt cggaattcag taagagagtc 3840
   atcctagctg atgccaatct ggacaaagta ttaagcgcat acaacaagca cagggataaa 3900
   cccatacgtg agcaggcgga aaatattatc catttgttta ctcttaccaa cctcggcgct 3960
   ccagccgcat tcaagtattt tgacacaacg atagatcgca aacagtacag atctaccaag 4020
   gaggtgctag acgcgacact gattcaccaa tccatcacgg gattatatga aactcggata     4080
   gatttgtcac agcttggggg tgacggatcc cccaagaaga agaggaaagt ctcgagcgac     4140
   tacaaagacc atgacggtga ttataaagat catgacatcg attacaagga tgacgatgac     4200
   aagtga                                                                4206
<210> 279
   <211> 422
   <212> DNA
   <213> Artificial Sequence
<220>
   <223> Description of Artificial Sequence: Synthetic polynucleotide
<400> 279
   tgtacaaaaa agcaggcttt aaaggaacca attcagtcga ctggatccgg taccaaggtc       60
   gggcaggaag agggcctatt tcccatgatt ccttcatatt tgcatatacg atacaaggct      120
   gttagagaga taattagaat taatttgact gtaaacacaa agatattagt acaaaatacg      180
   tgacgtagaa agtaataatt tcttgggtag tttgcagttt taaaattatg ttttaaaatg      240
   gactatcata tgcttaccgt aacttgaaag tatttcgatt tcttggcttt atatatcttg      300
   tggaaaggac gaaacaccgg agacgattaa tgcgtctccg ttttagagct agaaatagca      360
   agttaaaata aggctagtcc gttatcaact tgaaaaagtg gcaccgagtc ggtgcttttt      420
   tt                                                                     422

## Claims

1. An isolated *Streptococcus pyogenes* Cas9 (SpCas9) protein with mutations Q695A and Q926A in SEQ ID NO:1, wherein the SpCas9 protein is at least 95% identical to the amino acid sequence of SEQ ID NO: 1; and optionally one or more of a nuclear localization sequence, cell penetrating peptide sequence, and/or affinity tag.

2. The isolated protein of claim 1, comprising all four of the following mutations:
N497A, R661A, Q695A, and Q926A.

3. The isolated protein of claim 2, further comprising mutations at one, two, three, four, or all five of L169, Y450, N497, R661, and D1135.

4. The isolated protein of claim 1, further comprising mutations at one, two, three, four, or all five of L169, Y450, N497, R661, and D1135.

5. The isolated protein of claim 1, further comprising one or more of the following mutations: D1135E; D1135V; D1135V/R1335Q/T1337R (VQR variant):
D1135E/R1335Q/T1337R (EQR variant); D1135V/G1218R/R1335Q/T1337R (VRQR variant); or D1135V/G1218R/R1335E/T1337R (VRER variant).

6. The isolated protein of claims 1-5, further comprising one or more mutations that decrease nuclease activity selected from the group consisting of mutations at D10, E762, D839, H983, or D986; and at H840 or N863.

7. The isolated protein of claim 6, wherein the mutations that decrease nuclease activity are: (i) D10A or DION, and (ii) H840A, H840N, or H840Y

8. A fusion protein comprising the isolated protein of claims 1-7, fused to a heterologous functional domain, with an optional intervening linker, wherein the linker does not interfere with activity of the fusion protein.

9. The fusion protein of claim 8, wherein the heterologous functional domain is a transcriptional activation domain, preferably, wherein the transcriptional activation domain is from VP64 or NF-κB p65.

10. The fusion protein of claim 8, wherein the heterologous functional domain is a transcriptional silencer or transcriptional repression domain, preferably wherein the transcriptional repression domain is a Krueppel-associated box (KRAB) domain, ERF repressor domain (ERD), or mSin3A interaction domain (SID), or, wherein the transcriptional silencer is Heterochromatin Protein 1 (HP1), preferably HP1α or HP1β.

11. The fusion protein of claim 8, wherein the heterologous functional domain is an enzyme that modifies the methylation state of DNA, preferably wherein the enzyme that modifies the methylation state of DNA is a DNA methyltransferase (DNMT) or a TET protein, preferably, wherein the TET protein is TET1.

12. The fusion protein of claim 8, wherein the heterologous functional domain is an enzyme that modifies a histone subunit, preferably, wherein the enzyme that modifies a histone subunit is a histone acetyltransferase (HAT), histone deacetylase (HDAC), histone methyltransferase (HMT), or histone demethylase.

13. The fusion protein of claim 8, wherein the heterologous functional domain is a biological tether, preferably, wherein the biological tether is MS2, Csy4 or lambda N protein.

14. The fusion protein of claim 8, wherein the heterologous functional domain is FokI.

15. An isolated nucleic acid encoding the protein of claims 1-7.

16. A vector comprising the isolated nucleic acid of claim 15, optionally operably linked to one or more regulatory domains for expressing the protein of claims 1-7.

17. A host cell, preferably a mammalian host cell, comprising the nucleic acid of claim 15, and optionally expressing the protein of claims 1-7.

18. An *ex vivo* or *in vitro* method of altering the genome or epigenome of a cell, the method comprising expressing in the cell or contacting the cell with the isolated protein of claims 1-7, or the isolated fusion protein of claims 8-14, and a guide RNA having a region complementary to a selected portion of the genome of the cell.

19. The method of claim 18, wherein the isolated protein or fusion protein comprises one or more of a nuclear localization sequence, cell penetrating peptide sequence, and/or affinity tag, or, wherein the cell is a stem cell, preferably an embryonic stem cell, mesenchymal stem cell, or induced pluripotent stem cell.

20. A *ex vivo* or *in vitro* method of altering a double stranded DNA (dsDNA) molecule, the method comprising contacting the dsDNA molecule with the isolated protein of claims 1-7 or the fusion protein of claims 8-14, and a guide RNA having a region complementary to a selected portion of the dsDNA molecule.

## Patentansprüche

1. Isoliertes Streptococcus-pyogenes-Cas9-Protein (SpCas9-Protein) mit den Mutationen Q695A und Q926A in SEQ ID Nr. 1, wobei das SpCas9-Protein zu mindestens 95 % mit der Aminosäuresequenz von SEQ ID Nr. 1 identisch ist; und optional einer bzw. einem oder mehreren von einer Kernlokalisierungssequenz, einer zellpenetrierenden Peptidsequenz und/oder einem Affinitäts-Tag.

2. Isoliertes Protein nach Anspruch 1, umfassend alle vier der folgenden Mutationen: N497A, R661A, Q695A und Q926A.

3. Isoliertes Protein nach Anspruch 2, weiterhin umfassend Mutationen an einer, zwei, drei, vier oder allen fünf von L169, Y450, N497, R661 und D1135.

4. Isoliertes Protein nach Anspruch 1, weiterhin umfassend Mutationen an einer, zwei, drei, vier oder allen fünf von L169, Y450, N497, R661 und D1135.

5. Isoliertes Protein nach Anspruch 1, weiterhin umfassend eine oder mehrere der folgenden Mutationen: D1135E; D1135V; D1135V/R1335Q/T1337R (VQR-Variante):
D1135E/R1335Q/T1337R (EQR-Variante); D1135V/G1218R/R1335Q/T1337R (VRQR-Variante); oder D1135V/G1218R/R1335E/T1337R (VRER-Variante).

6. Isoliertes Protein nach den Ansprüchen 1-5, weiterhin umfassend eine oder mehrere Mutationen, die die Nuklease-Aktivität verringern, ausgewählt aus der Gruppe bestehend aus Mutationen an D10, E762, D839, H983 oder D986; und an H840 oder N863.

7. Isoliertes Protein nach Anspruch 6, wobei die Mutationen, die die Nuklease-Aktivität verringern, sind: (i) D10A oder D10N, und (ii) H840A, H840N oder H840Y.

8. Fusionsprotein, umfassend das isolierte Protein nach den Ansprüchen 1-7, das an eine heterologe funktionelle Domäne fusioniert ist, mit einem optionalen intervenierenden Linker, wobei der Linker die Aktivität des Fusionsproteins nicht stört.

9. Fusionsprotein nach Anspruch 8, wobei die heterologe funktionelle Domäne eine Transkriptionsaktivierungsdomäne ist, vorzugsweise wobei die Transkriptionsaktivierungsdomäne von VP64 oder NF-κB p65 ist.

10. Fusionsprotein nach Anspruch 8, wobei die heterologe funktionelle Domäne eine Transkriptionssilencer- oder Transkriptionsrepressionsdomäne ist, vorzugsweise wobei die Transkriptionsrepressionsdomäne eine Krüppel-assoziierte Box-Domäne (KRAB-Domäne, ERF-Repressordomäne (ERD) oder mSin3A-Interaktionsdomäne (SID) ist oder wobei die Transkriptionssilencer-Domäne Heterochromatin-Protein 1 (HP1), vorzugsweise HP1α oder HP1β ist.

11. Fusionsprotein nach Anspruch 8, wobei die heterologe funktionelle Domäne ein Enzym ist, das den Methylierungszustand von DNA modifiziert, vorzugsweise wobei das Enzym, das den Methylierungszustand von DNA modifiziert, eine DNA-Methyltransferase (DNMT) oder ein TET-Protein ist, vorzugsweise wobei das TET-Protein TET1 ist.

12. Fusionsprotein nach Anspruch 8, wobei die heterologe funktionelle Domäne ein Enzym ist, das eine Histon-Untereinheit modifiziert, vorzugsweise wobei das Enzym, das eine Histon-Untereinheit modifiziert, eine Histonacetyltransferase (HAT), Histondeacetylase (HDAC), Histonmethyltransferase (HMT) oder Histondemethylase ist.

13. Fusionsprotein nach Anspruch 8, wobei die heterologe funktionelle Domäne eine biologische Anbindung ist, vorzugsweise wobei die biologische Anbindung MS2, Csy4 oder Lambda-N-Protein ist.

14. Fusionsprotein nach Anspruch 8, wobei die heterologe funktionelle Domäne Fokl ist.

15. Isolierte Nukleinsäure, die das Protein nach den Ansprüchen 1-7 codiert.

16. Vektor, umfassend die isolierte Nukleinsäure nach Anspruch 15, optional operativ mit einer oder mehreren Regulationsdomänen verknüpft, zur Expression des Proteins nach den Ansprüchen 1-7.

17. Wirtszelle, vorzugsweise eine Säugetier-Wirtszelle, umfassend die Nukleinsäure nach Anspruch 15 und optional das Protein nach den Ansprüchen 1-7 exprimierend.

18. *Ex-vivo-* oder *In-vitro*-Verfahren zur Veränderung des Genoms oder Epigenoms einer Zelle, wobei das Verfahren ein Exprimieren in der Zelle oder ein Inkontaktbringen der Zelle mit dem isolierten Protein nach den Ansprüchen 1-7 oder dem isolierten Fusionsprotein nach den Ansprüchen 8-14 und einer Guide-RNA mit einer Region, die zu einem ausgewählten Abschnitt des Genoms der Zelle komplementär ist, umfasst.

19. Verfahren nach Anspruch 18, wobei das isolierte Protein oder Fusionsprotein eine bzw.
eines oder mehrere von einer Kernlokalisierungssequenz, einer zellpenetrierenden Peptidsequenz und/oder einem Affinitäts-Tag umfasst oder wobei die Zelle eine Stammzelle, vorzugsweise eine embryonale Stammzelle, mesenchymale Stammzelle oder induzierte pluripotente Stammzelle ist.

20. *Ex-vivo-* oder *In-vitro*-Verfahren zur Veränderung eines doppelsträngigen DNA-Moleküls (dsDNA-Moleküls), wobei das Verfahren ein Inkontaktbringen des dsDNA-Moleküls mit dem isolierten Protein nach den Ansprüchen 1-7 oder dem Fusionsprotein nach den Ansprüchen 8-14 und einer Guide-RNA mit einer Region, die zu einem ausgewählten Abschnitt des dsDNA-Moleküls komplementär ist, umfasst.

## Revendications

1. Une protéine *Streptococcus pyogenes* Cas9 (SpCas9) isolée avec des mutations Q695A et Q926A dans SEQ ID NO: 1, dans laquelle la protéine SpCas9 est au moins 95 % identique à la séquence d'acides aminés de SEQ ID NO: 1 ; et en option une ou plusieurs séquences de localisation nucléaire, une séquence de peptides pénétrant dans les cellules et/ou une étiquette d'affinité.

2. La protéine isolée selon la revendication 1, comprenant les quatre mutations suivantes :
N497A, R661A, Q695A et Q926A.

3. La protéine isolée selon la revendication 2, comprenant en outre des mutations à une, deux, trois, quatre ou les cinq parmi L169, Y450, N497, R661 et D1135.

4. La protéine isolée selon la revendication 1, comprenant en outre des mutations à une, deux, trois, quatre ou les cinq parmi L169, Y450, N497, R661 et D1135.

5. La protéine isolée selon la revendication 1, comprenant en outre une ou plusieurs des mutations suivantes: D1135E ; D1135V ; D1135V/R1335Q/T1337R (variant VQR) ; D1135E/R1335Q/T1337R (variant EQR) ; D1135V/G1218R/R1335Q/T1337R (variant VRQR) ; ou D1135V/G1218R/R1335E/T1337R (variant VRER).

6. La protéine isolée selon les revendications 1-5, comprenant en outre une ou plusieurs mutations qui diminuent l'activité des nucléases sélectionnées dans le groupe constitué des mutations à D10, E762, D839, H983 ou D986 ; et à H840 ou N863.

7. La protéine isolée selon la revendication 6, dans laquelle les mutations qui diminuent l'activité des nucléases sont : (i) D10A ou D10N et (ii) H840A, H840N ou H840Y.

8. Une protéine de fusion comprenant la protéine isolée selon les revendications 1-7, fusionnée à un domaine fonctionnel hétérologue, avec un lieur intervenant en option, dans laquelle le lieur n'interfère pas avec l'activité de la protéine de fusion.

9. La protéine de fusion selon la revendication 8, dans laquelle le domaine fonctionnel hétérologue est un domaine d'activation transcriptionnelle, de préférence, dans laquelle le domaine d'activation transcriptionnelle provient de VP64 ou NF-kB p65.

10. La protéine de fusion selon la revendication 8, dans laquelle le domaine fonctionnel hétérologue est un atténuateur transcriptionnel ou domaine de répression transcriptionnelle, de préférence dans laquelle le domaine de répression transcriptionnelle est un domaine de la boîte associée à Krueppel (KRAB), un domaine répresseur ERF (ERD) ou un domaine d'interaction mSin3A (SID) ou, dans laquelle l'atténuateur transcriptionnel est la protéine hétérochromatine 1 (HP1), de préférence HP1α ou HP1β.

11. La protéine de fusion selon la revendication 8, dans laquelle le domaine fonctionnel hétérologue est une enzyme qui modifie l'état de méthylation de l'ADN, de préférence dans laquelle l'enzyme qui modifie l'état de méthylation de l'ADN est un ADN méthyltransférase (DNMT) ou une protéine TET, de préférence, dans laquelle la protéine TET est TET1.

12. La protéine de fusion selon la revendication 8, dans laquelle le domaine fonctionnel hétérologue est une enzyme qui modifie une sous-unité histone, de préférence, dans laquelle l'enzyme qui modifie une sous-unité histone est un histone acétyltransférase (HAT), histone déacétylase (HDAC), histone méthyltransférase (HMT) ou histone déméthylase.

13. La protéine de fusion selon la revendication 8, dans laquelle le domaine fonctionnel hétérologue est un lien biologique, de préférence, dans laquelle le lien biologique est une protéine MS2, Csy4 ou lambda N.

14. La protéine de fusion selon la revendication 8, dans laquelle le domaine fonctionnel hétérologue est Fokl.

15. Un acide nucléique isolé codant la protéine selon les revendications 1-7.

16. Un vecteur comprenant l'acide nucléique isolé selon la revendication 15, en option, lié de manière fonctionnelle à un ou plusieurs domaines réglementaires pour exprimer la protéine selon les revendications 1-7.

17. Une cellule hôte, de préférence une cellule hôte de mammifère, comprenant l'acide nucléique selon la revendication 15, et en option, exprimant la protéine selon les revendications 1-7.

18. Un procédé *ex vivo* ou *in vitro* de modification du génome ou l'épigénome d'une cellule, le procédé consistant à exprimer dans la cellule ou à mettre la cellule en contact avec la protéine isolée selon les revendications 1-7 ou la protéine de fusion isolée selon les revendications 8-14, et un ARN guide ayant une région complémentaire d'une partie sélectionnée du génome de la cellule.

19. Le procédé selon la revendication 18, dans lequel la protéine isolée ou la protéine de fusion comprend une ou plusieurs séquences de localisation nucléaire, une séquence de peptides pénétrant dans les cellules et/ou une étiquette d'affinité, ou, dans lequel la cellule est une cellule souche, de préférence une cellule souche embryonique, une cellule souche mésenchymateuse ou une cellule souche pluripotente induite.

20. Un procédé *ex vivo* ou *in vitro* de modification d'une molécule d'ADN double brin (ADNds), le procédé consistant à mettre en contact la molécule ADNds avec la protéine isolée selon les revendications 1-7 ou la protéine de fusion selon les revendications 8-14 et un ARN guide ayant une région complémentaire d'une partie sélectionnée de la molécule ADNds.
